# EUROPEAN PATENT SPECIFICATION

(11) **EP 2 054 060 B1**
(45) Date of publication and mention of the grant of the patent: **02.04.2014**
(21) Application number: 07784851.3
(22) Date of filing: 24.08.2007
(51) Int. Cl.: C07B 59/00, C07D 471/04, C07D 487/04, A61K 31/437, A61K 31/498, A61K 31/4985, A61K 31/5025, A61K 31/519, A61K 31/53, A61P 25/00, A61P 25/28, A61P 35/00

(54) **FLUORINATED LIGANDS FOR TARGETING PERIPHERAL BENZODIAZEPINE RECEPTORS**
FLUORIERTE LIGANDEN FÜR TARGETING VON PERIPHEREN BENZODIAZEPIN-REZEPTOREN
LIGANDS FLUORÉS POUR LE CIBLAGE DES RÉCEPTEURS PÉRIPHERIQUES DES BENZODIAZÉPINES

(30) Priority: 24.08.2006 AU 2006904617 P; 22.12.2006 AU 2006907288 P
(43) Date of publication of application: 06.05.2009
(60) Divisional application: 14155943.5
(73) Proprietor: AUSTRALIAN NUCLEAR SCIENCE & TECHNOLOGY ORGANISATION, Lucas Heights New South Wales 2234 (AU)
(72) Inventor: KATSIFIS, Andrew, Lugarno, NSW 2210 (AU); FOOKES, Christopher, John, Reginald, Grays Point, NSW 2232 (AU); PHAM, Tien, Quoc, Barden Ridge, NSW 2234 (AU); GREGURIC, Ivan, Damir, Oatley, NSW 2223 (AU); MATTNER, Maria, Filomena, Pereira Soares, Barden Ridge, NSW 2234 (AU)
(74) Representative: Elend, Almut Susanne
(86) International application number: PCT/AU2007/001216
(87) International publication number: WO 2008/022396

(56) References cited:
- EP-B1- 0 050 563
- WO-A1-2006/051063
- WO-A1-2007/134362
- SELLERI ET AL.: "2-Arylpyrazolo[1,5-a]pyrimidin-3-yl Acetamides. New Potent and Selective Peripheral Benzodiazepine Receptor Ligands" BIOORG. MED. CHEM., vol. 9, no. 10, 1 January 2001 (2001-01-01), pages 2661-2671, XP008090470 ISSN: 0968-0896 DOI: 10.1016/S0968-0896(01)00192-4
- KOZIKOWSKI ET AL.: J. MED. CHEM., vol. 36, no. 20, 1 January 1993 (1993-01-01), pages 2908-2920, XP002118394 ISSN: 0022-2623 DOI: 10.1021/JM00072A010
- PIKE ET AL.: "Radioligands for PET studies of central benzodiazepine receptors and PK (peripheral benzodiazepine) binding sites-current status" NUCL. MED. BIOL., vol. 20, no. 4, 1 May 1993 (1993-05-01), pages 503-525, XP026310326 ISSN: 0969-8051 DOI: 10.1016/0969-8051(93)90082-6 [retrieved on 1993-05-01]
- TRAPANI ET AL.: "Synthesis and Binding Affinity of 2-Phenylimidazo[1,2-alpha]pyridine Derivatives for both Central and Peripheral Benzodiazepine Receptors. A New Series of High-Affinity and Selective Ligands for the Peripheral Type" J. MED. CHEM., vol. 40, 1 January 1997 (1997-01-01), pages 3109-3118, XP002900938 ISSN: 0022-2623 DOI: 10.1021/JM970112+
- BARLIN ET AL.: 'Imadazo[1,2-]pyridazines. XX. Syntheses of some 3-acylaminomethyl-6-(chloro, fluoro, methoxy, methylthio, phenoxy and phenylthio)-2-(phenyl, 4-t-butylphenyl,4-cyclohexylphenyl. beta-napthyl and styryl)imidazo[1,2-b]pyridazines and their interaction with c..' AUST. J. CHEM. vol. 49, 1996, pages 451 - 462, XP008103376
- SELLERI ET AL.: '2-Arylpyrazolo[1,5-a]pyrimidin-3-yl acetamides. New potent and selective peripheral benzodiazepine receptor ligands' BIOORGANIC & MEDICINAL CHEMISTRY vol. 9, 2001, pages 2661 - 2671, XP008090470
- BARLIN ET AL.: 'Imidazo[1,2-b]pyridazines. X. Syntheses and central nervous system activities of some 3-(acetamido, benzamido, substituted or dimethylamino)methyl-2-(phenyl or substituted phenyl)-6-(halogeno, alkylthio, alkoxy, phenylthio, penoxy, benzylthio or benzyloxy)imidazo[1,2-]pyridazines' AUST. J. CHEM. vol. 45, 1992, pages 731 - 749, XP009066843
- HARRISON ET AL.: 'Syntheses, pharmacological evaluation and molecular modelling of sunstituted 6-alkoxyimidazo[1,2-b]pyridazines as new ligands for the benzodiazepine receptor' EUR. J. MED. CHEM. vol. 31, 1996, pages 651 - 662, XP004040242
- BLACKBURN: 'A three-component solid-phase synthesis of 3-aminoimidazo[1,2-a]azines' TETRAHEDRON LETTERS vol. 39, 1998, pages 5469 - 5472, XP004124091
- BARLIN ET AL.: 'Imidazo[1,2-b]pyridazines. XV. Synthesis of anxiolytic activity of some 3-(benzamidomethyl and fluorobenzamidomethyl)-6-(fluoro, chloro and methylthio-)-2-(4-totyl and 3,4-methylenedioxyphenyl)imidazo[1,2-b]pyri dazines' AUST. J. CHEM. vol. 47, 1994, pages 609 - 621, XP008103377
- DATABASE CAPLUS [Online] 2004 BELYUGA A.G. ET AL: 'Synthesis of 3-acylamino-2-arylimidazo[1,2-a]pyridines and their pyrimidine analogs on the basis of aminophenacylating reagents', XP008103527 Database accession no. (143:326321) & ZHURNAL ORGANICHNOI TA FARMATSEVTICHNOI KHIMII vol. 2, no. 4, pages 25 - 31
- DATABASE CAPLUS [Online] 08 July 1997 XP008103562 Database accession no. (127:108931) & JP 09 176165 A
- BARLIN ET AL.: 'Imidazo[1,2-b]pyridazines. XIII. Syntheses and central nervous system activities of some 2-benzyl(phenethyl, biphenyl-4'-yl, 6'-methyl-naphtalen-2'-yl, t-butyl and cyclohexyl)-3-methoxy-(acylyminomethyl and dimethylaminomethyl)-variously substituted)imidazo[1,2-b]pyridazines' AUST. J. CHEM. vol. 45, 1992, pages 1281 - 1300, XP009080477
- DE PAULIS ET AL.: 'Substituent effects of N-(1,3-diphenyl-1H-pyrazol-5-yl)benzamides on positive allosteric modulation on the metabotropic glutamate-5 receptor in rat cortical astrocytes' J. MED. CHEM. vol. 49, 2006, pages 3332 - 3344, XP008103364
- ASCALONE ET AL.: 'Determination of zolpidem, a new sleep-inducing agent, and its metabolites in biological fluids: pharmacokinetics, drug metabolism and overdosing investigations in humans' JOURNAL OF CHROMATOGRAPHY vol. 581, 1992, pages 237 - 250, XP008103366
- ASCALONE ET AL.: 'Determination of alpidem and its metabolites in human plasma by high-performance liquid chromatography and fluorimetric detection' JOURNAL OF CHROMATOGRAPHY vol. 414, 1987, pages 101 - 108, XP008103367

## Description

### Technical Field

The present invention relates to radiolabelled and non-radiolabelled heterocyclic compounds which bind to peripheral benzodiazepine receptors and are useful for imaging such receptors and providing therapeutic treatment of conditions associated with PBR receptors.

### Background of the Invention

The peripheral benzodiazepine receptors, which are also commonly referred to as the peripheral benzodiazepine binding sites, mitochondrial benzodiazepine receptors or ω-3-receptors, are distinct from the central benzodiazepine receptors in their pharmacology, subcellular location and structural requirements. Peripheral benzodiazepine receptors are predominantly found in the peripheral organs such as kidney, heart, adren8al cortex, testis, ovaries, plasma (platelets) as well as in the glial cells and olfactory bulbs in the brain. It has also been reported that peripheral benzodiazepine receptor density is higher in tumours, such as breast, prostate, glioma, ovarian and colon carcinoma, than in corresponding normal tissue. Recently high concentrations of peripheral benzodiazepine receptors has been reported in Dunning rat prostate tumours compared to the normal ventral or dorsolateral prostate.

The peripheral benzodiazepine receptors appear to be associated (but not exclusively) with the outer mitochondrial membrane in many tissues where they are modulated by hormones and drugs and reflect the effects of emotional stress, and hypertension. Peripheral benzodiazepine receptors in the brain have been investigated for use as markers of neurodegeneration and inflammation including Huntington's disease, Alzheimer's disease, multiple sclerosis, anxiety, stress, emotional disturbances, cognitive impairment, stroke, and cerebral ischemia. The Peripheral Benzodiazepine Receptor has also been implicated in apoptotic processes.

PBR ligands themselves modulate various cellular processes including heme transport, cholesterol and steroid synthesis, apoptosis and calcium transport among others. They are also involved in cellular proliferation and differentiation.

Several classes of ligands have been shown to exhibit high affinity binding to the peripheral benzodiazepine receptor, the most widely investigated being the benzodiazepine Ro 5-4864 (7-chloro-5-(4-chlorophenyl)-1,3-dihydro-1-methyl-2*H*-1,4-benzodiazepin-2-one) and the isoquinoline PK-11195 (1-(2-chlorophenyl)-N-methyl-N-(1-methylpropyl)-3-isoquinolinecarboxamide). Labelled with ¹¹C, ¹⁴C and ³H, these ligands have been used to map peripheral benzodiazepine receptors in the human brain and heart by *in vivo* and *in vitro* means. Enhanced uptake of [³H]PK-11195 has been reported in a variety of tumour cells including breast, ovarian, prostate, adrenal, brain and colon. Radiolabelling with suitable levels of radioactive isotopes such as carbon-11, fluorine-18 and iodine-123/124 may be used firstly to diagnose these tumours and subsequently to treat them with therapeutic doses (for instance using ¹²³I, ¹²⁵I or ¹³¹I). Furthermore, recent work has also revealed the existence of several binding domains which differ in affinity for isoquinoline and benzodiazepine ligands in different organs and species.

Various 2-aryl substituted imidazo[1,2-a]pyridines imidazopyridazines and aryl-pyrazolo pyrimidines having anxiolytic, hypnotic, anticonvulsant, analgesic and other properties nave been reported (Almirante L. et al., J. Med. Chem. 12 122-126 (1969); Langer S.Z. et al., Pharmacol. Biochem, Behav. 29 763-766 (1988); Langer S.Z. et al., Pharmacopsychiatry 23 103-107 (1990); Bourguignon, J-J., "Endogenous and Synthetic Ligands of Mitochondrial Benzodiazepine Receptors: Structure Affinity Relationships" in Giesen-Grouse, E. ed. Peripheral Benzodiazapine Receptors, Academic Press, London (1993)). For example ¹²³I labelled 6-methyl-2-(4'-iodophenyl)imidazo[1,2-a]pyridine-3-(N,N-dimethyl)-acetamide has been reported as a potential tracer for the study of the peripheral benzodiazepine receptor using SPECT (Katsitis A. et al., J. Lab. Comp. Radiopharm. 40 620-622 (1997)).

In published international application WO 99/51594 it was discovered that certain 2-(iodophenyl)-imidazo[1,2-a]pyridines having an electronegative substituent, especially halogen, in the pyridine nucleus exhibit strong binding to peripheral benzodiazepine receptors and mucn weaker binding to central benzodiazepine receptors.

Aryl-pyrazolo pyrimidines as selective peripheral benzodiazepine receptor ligands are known from Selleri S. et al., Bioorg. Med. Chem. 9 2661-2671 (2001).

The present invention relates to a class of fluorinated compounds for use as PBR pharmacological agents in the treatment of various disorders such as neuroinflammation, neurodegeneration, stroke, apoptosis and other disorders. The invention also relates to compounds labelled with the PET radionuclide ¹⁸F that are useful for PET imaging using positron emission tomography. Appropriately radiolabelled compounds of the present invention are clinically useful in PET scanning, for example to detect those cancers which express a high density of the peripheral benzodiazepine receptors and/or to detect, or non-invasively diagnose, neurodegenerative disorders. The compounds of the present invention are also useful for the treatment of disorders characterised by an abnormal density of peripheral benzodiazepine receptors, such as neurodegenerative disorders and tumours. The present disclosure also relates to intermediates of the fluorinated compounds.

### Summary of the Invention

The present invention is limited to the subject-matter of the appendant claims. The following description is subject to this limitation. In a first aspect the present invention provides a fluorinated compound of formula (I): 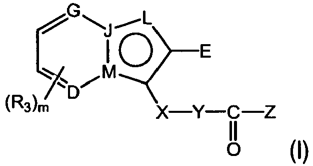 wherein,
D, G, and L are independently selected from the group consisting of: CH, C and N, and J and M are independently selected from the group consisting of C and N provided that at least one of J and M is C, wherein at least two of D, G, M, J and L are N;
X is selected from the group consisting of: O, NH, (CH₂)ₙ and S;
Y is absent, or is selected from the group consisting of: O, NH, (CH₂)ₙ, and S;
Z is selected from the group consisting of: NR₁R₂ and aryl;
R₁ and R₂ are independently selected from the group consisting of: hydrogen, C₁-C₁₀ alkyl, C₂-C₁₀ alkenyl, C₂-C₁₀ alkynyl, aryl and heteroaryl, each of which may optionally be substituted with one or more of the following substituents: halogen and C₁-C₆ alkyl;
or R₁ and R₂, together with the nitrogen to which they are attached, form a heterocyclic ring having between 3 and 7 ring members, which may optionally be substituted with one or more of the following substituents: halogen and C₁-C₆ alkyl;
R₃ is selected from the group consisting of: halogen, C₁-C₁₀ alkyl and O-(C₁-C₁₀ alkyl), wherein the C₁-C₁₀ alkyl group is optionally substituted;
E is an aryl group or a heteroaryl group, wherein each group is optionally substituted with one or more fluoro substituents, or with one or more of the following substituents: C₁-C₆ alkyl, C₂-C₁₀ alkenyl, C₂-C₁₀ alkynyl, QC₁-C₁₀ alkyl, QC₂-C₁₀ alkenyl, QC₂-C₁₀ alkynyl, Q(CH₂)ₚ-Q-(CH₂)_{q}CH₃ or Q(CH₂)ₚ-Q-(CH₂)_{q}-Q-(CH₂)ᵣCH₃, each of which may be optionally substituted with one or more fluoro substituents, and wherein p, q and r are, independently, integers between 1 and 3, and wherein Q is selected from the group consisting of: NH, O and S;
m is a number between 0 and 3 (e.g. 0, 1, 2 or 3) wherein if m is 2 or 3, the R₃ groups may be the same or may be different;
n is a number between 1 and 4, e.g. 1, 2, 3 or 4 (wherein n in X may be the same as or different to n in Y);
with the proviso that R₃ is a fluoro substituent, or
the group E comprises a fluoro substituent, or
the group Z comprises a fluoro substituent, with the further proviso that E is not 4-fluorophenyl.
The further proviso may be that E is not -PhF.

The invention provides a fluorinated compound of formula (I) wherein:
D, G, and L are independently selected from the group consisting of: CH, C and N, and J and M are independently selected from the group consisting of C and N provided that at least one of J and M is C, wherein at least two of D, G, M, J and L are N;
X is independently selected from the group consisting of: O, NH, (CH₂)ₙ and S;
Y is absent, or is independently selected from the group consisting of: O, NH and (CH₂)ₙ, and S;
Z is selected from the group consisting of: NR₁R₂ and aryl;
R₁ and R₂ are independently selected from the group consisting of: hydrogen, C₁-C₁₀ alkyl, C₂-C₁₀ alkenyl, C₂-C₁₀ alkynyl, aryl and heteroaryl, each of which may optionally be substituted with one or more of the following substituents: halogen and C₁-C₆ alkyl;
or R₁ and R₂, together with the nitrogen to which they are attached, form a heterocyclic ring having between 3 and 7 ring members, which may optionally be substituted with one or more of the following substituents: halogen and C₁-C₆ alkyl;
R₃ is selected from the group consisting of: halogen and C₁-C₁₀ alkyl;
E is an aryl group or a heteroaryl group, which may be optionally substituted with one or more fluoro substituents, or one or more of the following substituents: C₁-C₆ alkyl, C₂-C₁₀ alkenyl, C₂-C₁₀ alkynyl, QC₁-C₁₀ alkyl, QC₂-C₁₀ alkenyl, QC₂-C₁₀ alkynyl, Q(CH₂)ₚ-Q-(CH₂)_{q}CH₃ or Q(CH₂)ₚ-Q-(CH₂)_{q}-Q-(CH₂)ᵣCH₃, each of which may be optionally substituted with one or more fluoro substituents, and wherein p, q and r are integers between 1 and 3, and wherein Q is independently selected from the group consisting of: NH, O and S;
m is a number between 0 and 3;
n is a number between 1 and 4;
with the proviso that R₃ is a fluoro substituent, or
the group E comprises a fluoro substituent, or
the group Z comprises a fluoro substituent, with the further proviso that E may not be -PhF.

The term fluoro substituent(s) may refer to ¹⁹F (also referred to as F) or may refer to ¹⁸F, or may refer to a mixture of the two.

E may be an aryl group or a heteroaryl group, which may be optionally substituted with one or more fluoro substituents selected from ¹⁹F, ¹⁸F and a combination thereof, and/or one or more of the following substituents: C₁-C₆ alkyl, C₂-C₁₀ alkenyl, C₂-C₁₀ alkynyl, QC₁-C₁₀ alkyl, QC₂-C₁₀ alkenyl, QC₂-C₁₀ alkynyl, Q(CH₂)ₚ-Q-(CH₂)_{q}CH₃ or Q(CH₂)ₚ-Q-(CH₂)_{q}-Q-(CH₂)ᵣCH₃, each of which may be optionally substituted with one or more fluoro substituents selected from ¹⁹F, ¹⁸F and a combination thereof, and wherein p, q and r are, independently, integers between 1 and 3, and wherein Q is selected from the group consisting of: NH, O and S.

E may be an aryl group or a heteroaryl group, which may be optionally substituted with ¹⁸F, or one or more of the following substituents: C₁-C₆ alkyl, C₂-C₁₀ alkenyl, C₂-C₁₀ alkynyl, QC₁-C₁₀ alkyl, QC₂-C₁₀ alkenyl, QC₂-C₁₀ alkynyl, Q(CH₂)ₚ-Q-(CH₂)_{q}CH₃ or Q(CH₂)ₚ-Q-(CH₂)_{q}-Q-(CH₂)ᵣCH₃, each of which may be optionally substituted with ¹⁸F, and wherein p, q and r are, independently, integers between 1 and 3, and wherein Q is selected from the group consisting of: NH, O and S.

E may be an aryl group or a heteroaryl group, which is substituted with ¹⁸F.

E may be an aryl group or a heteroaryl group, which is substituted with ¹⁹F.

The first aspect may be with the proviso that R₃ is a fluoro substituent which is ¹⁹F or ¹⁸F, or the group E comprises a fluoro substituent which is ¹⁹F or ¹⁸F or a combination thereof, or the group Z comprises a fluoro substituent which is ¹⁹F or ¹⁸F or a combination thereof, with the further proviso that E may not be -PhF (or may not be 4-fluorophenyl).

R₃ may be selected from the group consisting of: ¹⁹F and ¹⁸F. R₃ may be ¹⁸F. R₃ may be ¹⁹F.

¹⁸F is a radioactive fluoro substituent. ¹⁹F, which is alternatively referred to as F throughout the specification, is a non-radioactive fluoro substituent.

In an embodiment of the invention Q may be oxygen.

In another embodiment of the invention, m is 1 or 2, and n is 1 or 2, optionally 1.

In a further embodiment of the invention, p may be 1 or 2, q and r may be 1, and Q may be oxygen.

In some embodiments, L is N. In some embodiments one of M and J is N.

In one embodiment, M and L are N, D and G are CH and J is C. In another embodiment, D, M and L are N, J is C and G is CH. In an alternative embodiment, D, J and L are N, M is C and G is CH.

R₃ may be attached to a carbon atom.

R₃ may be optionally substituted with one or more halogens, such as fluorine, chlorine, bromine and/or iodine.

R₃ may be selected from the group consisting of: halogen and C₁-C₆ alkyl, optionally substituted (e.g. with one or more halogens such as fluorine, chlorine, bromine and/or iodine). R₃ may be methyl. It may be methoxy. It may be trifluoromethyl. It may be trifluoromethoxy.

In another embodiment, R₃ may be selected from the group consisting of chloro and C₁-C₃ alkyl. In a further embodiment, R₃ may be chloro or methyl and m may be 1 or 2. Alternatively, R₃ may be chloro and m may be 1 or 2, or R₃ may be fluoro and m may be 1 or 2. R₃ may be chloro and m may be 1 or 2, or R₃ may be ¹⁸fluoro and m may be 1 or 2. If m>1, the R₃ groups may be the same or they may be different. For example, if m=2, the R₃ groups may be both methyl, or one may be methyl and the other trifluoromethyl, or one may be F and the other trifluoromethyl. R₃ may be chloro and m may be 1 or 2, or R₃ may be ¹⁹fluoro and m may be 1 or 2.

In one embodiment, Y is absent and X is (CH₂)ₙ, wherein n is 1 or 2.

In another embodiment, X and Y may be selected from the group consisting of: NH and (CH₂)ₙ, wherein n is 1.

Y may be (CH₂)ₙ and X may be NH, wherein n is 1 or 2.

In one embodiment, -X-Y-C(O)-Z may have a structure selected from the group consisting of: -(CH₂)ₙ-C(O)-Z, wherein n is 1 or 2, or -(CH₂)ₙ-NH-C(O)-Z, wherein n is 1 or 2.

Z may be NR₁R₂ or phenyl.

In one embodiment Z is NR₁R₂.

R₁ and R₂ may be independently selected from the group consisting of: hydrogen C₁-C₆ alkyl, phenyl, pyridyl, pyrimidinyl, pyridazinyl and where R₁ and R₂, together with the nitrogen to which they are attached, form a heterocyclic ring having between 4 and 6 ring members, wherein the C₁-C₆ alkyl group, the phenyl group, the pyridyl group, the pyrimidinyl group, the pyridazinyl group or the heterocyclic ring may optionally be substituted with between one and three fluoro substituents. The heterocyclic ring may be a pyrrolidine ring.

R₁ and R₂ may be independently selected from the group consisting of: hydrogen C₁-C₆ alkyl, phenyl, pyridyl, pyrimidinyl, pyridazinyl and where R₁ and R₂, together with the nitrogen to which they are attached, form a heterocyclic ring having between 4 and 6 ring members, wherein the C₁-C₆ alkyl group, the phenyl group, the pyridyl group, the pyrimidinyl group, the pyridazinyl group or the heterocyclic ring may optionally be substituted with between one or more fluoro substituents selected from the group consisting of 1, 2, or 3 ¹⁹F substituents or an ¹⁸F substituent or a combination of a ¹⁹F substituent and an ¹⁸F substituent or a combination of two ¹⁹F substituents and an ¹⁸F substituent. The heterocyclic ring may be a pyrrolidine ring.

R₁ and R₂ may be independently selected from the group consisting of: hydrogen C₁-C₆ alkyl, phenyl, pyridyl, and where R₁ and R₂, together with the nitrogen to which they are attached, form a heterocyclic ring having between 4 and 6 ring members, wherein the heterocyclic ring, the C₁-C₆ alkyl group, the phenyl group or the pyridyl group may optionally be substituted with between 1 and 3 fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

In another embodiment, R₁ and R₂ may be independently selected from the group consisting of: hydrogen, phenyl, pyridyl, methyl, ethyl, propyl, isopropyl, butyl, t-butyl, and pentyl, wherein each of these substituents may optionally be substituted with between 1 and 3 fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

In an alternative embodiment, R₁ and R₂ may be selected from the group consisting of: hydrogen, phenyl, pyridyl, methyl, ethyl, isopropyl and wherein R₁ and R₂ together with the nitrogen to which they are attached, form a pyrrolidine ring, and wherein the phenyl, pyridyl, methyl, ethyl, isopropyl or pyrrolidine ring may optionally be substituted with between 1 and 3 fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

When E is phenyl, E may optionally not have a halogen attached directly to it in the 4 position. In particular E may not have a chloro, bromo, fluoro or iodo substituent attached directly to it in the 4 position. E may be phenyl or substituted phenyl which is not substituted by a fluoro or iodo substituent in the 4 position. If E is phenyl, it may in some embodiments have no halogen attached directly to it.

E may be a pyridyl group, a pyrimidinyl group or a pyridazinyl group, each of which may optionally be substituted with one or more fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent), and/or substituted with one or more of the following substituents: C₁-C₆ alkyl or C₁-C₁₀ alkoxy, each of which may optionally be substituted with one or more fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

E may be a pyridyl group, a pyrimidinyl group or a pyridazinyl group, each of which may optionally be substituted with between one and three fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent), or substituted with one of the following substituents: C₁-C₆ alkyl or C₁-C₆ alkoxy, either of which may optionally be substituted with between one and three fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

E may be a pyridyl group, a pyrimidinyl group or a pyridazinyl group, each of which may optionally be substituted with between one and three fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

E may be a phenyl group, a pyridyl group, a pyrimidinyl group or a pyridazinyl group, each of which may optionally be substituted with one or more of the following substituents: C₁-C₆ alkyl or C₁-C₁₀ alkoxy, each of which may optionally be substituted with one or more fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

E may be a phenyl group, a pyridyl group, a pyrimidinyl group or a pyridazinyl group, each of which may optionally be substituted with one of the following substituents: C₁-C₆ alkyl or C₁-C₁₀ alkoxy, either of which may optionally be substituted with between one and three fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

E may be a phenyl group, a pyridyl group, a pyrimidinyl group or a pyridazinyl group, each of which may optionally be substituted with one of the following substituents: C₁-C₆ alkyl or C₁-C₁₀ alkoxy, either of which may optionally be substituted with between one and three fluoro substituents(e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent), or E may be a pyridyl group, a pyrimidinyl group or a pyridazinyl group, each of which may optionally be substituted with between one and three fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

E may be a phenyl group which is substituted with one or more C₁-C₆ alkyl or C₁-C₁₀ alkoxy groups, each of which may optionally be substituted with one or more fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

E may be a phenyl group which is substituted with one or more C₁-C₆ alkyl or C₁-C₁₀ alkoxy groups, each of which may optionally be substituted with between one to three fluoro substituents (e.g. 1, 2, or 3 fluoro substituents or 1, 2 or 3 ¹⁸F substituents or any combination of 2 or 3 F and 18F substituents).

E may be a phenyl group which is substituted with one or more C₁-C₆ alkyl or C₁-C₆ alkoxy groups, each of which may optionally be substituted with one or more fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

E may be a phenyl group which is substituted with one or more C₁-C₆ alkyl or C₁-C₆ alkoxy groups, each of which may optionally be substituted with between one and three fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

E may be a phenyl group which is substituted with a C₁-C₆ alkyl group or a C₁-C₁₀ alkoxy group, each of which may optionally be substituted with a fluoro substituent (e.g. an ¹⁹F substituent or an ¹⁸F substituent).

E may be a phenyl group which is substituted with a C₁-C₆ alkyl group or a C₁-C₆ alkoxy group, each of which may optionally be substituted with a fluoro substituent (e.g. an ¹⁹F substituent or an ¹⁸F substituent).

E may be a phenyl group which is substituted with a C₁-C₆ alkoxy group, which may optionally be substituted with a fluoro substituent (e.g. an ¹⁹F substituent or an ¹⁸F substituent).

In an embodiment, R₃ is chloro, m is 1 or 2, E is fluoropyridyl, fluoropyrimidinyl or fluoropyridazinyl, Z is NR₁R₂, wherein R₁ and R₂ are selected from the group consisting of: C₁-C₆ alkyl and phenyl, Y is absent, and X is (CH₂)ₙ, wherein n is 1 or 2.

In another embodiment, R₃ is chloro, m is 1 or 2, E is ¹⁸fluoropyridyl, ¹⁸fluoropyrimidinyl or ¹⁸fluoropyridazinyl, Z is NR₁R₂, wherein R₁ and R₂ are selected from the group consisting of: C₁-C₆ alkyl and phenyl, Y is absent, and X is (CH₂)ₙ, wherein n is 1 or 2.

In another embodiment, R₃ is chloro, m is 1 or 2, E is ¹⁹fluoropyridyl, ¹⁹fluoropyrimidinyl or ¹⁹fluoropyridazinyl, Z is NR₁R₂, wherein R₁ and R₂ are selected from the group consisting of: C₁-C₆ alkyl and phenyl, Y is absent, and X is (CH₂)ₙ, wherein n is 1 or 2.

In a further embodiment, R₃ is chloro, m is 1 or 2, E is phenyl substituted with a C₁-C₆ alkoxy group, Z is NR₁R₂, wherein R₁ is C₁-C₆ alkyl and R₂ is fluoropyridyl or fluorophenyl.

In a further embodiment, R₃ is chloro, m is 1 or 2, E is phenyl substituted with a C₁-C₆ alkoxy group, Z is NR₁R₂, wherein R₁ is C₁-C₆ alkyl and R₂ is ¹⁸fluoropyridyl or ¹⁸fluorophenyl.

In a further embodiment, R₃ is chloro, m is 1 or 2, E is phenyl substituted with a C₁-C₆ alkoxy group, Z is NR₁R₂, wherein R₁ is C₁-C₆alkyl and R₂ is ¹⁹fluoropyridyl or ¹⁹fluorophenyl.

In one embodiment, R₃ is F, m is 1 and E is phenyl substituted with a C₁-C₆ alkyl group or a C₁-C₆ alkoxy group.

In one embodiment, R₃ is ¹⁸F, m is 1 and E is phenyl substituted with a C₁-C₆ alkyl group or a C₁-C₆ alkoxy group.

In another embodiment, R₃ is F, m is 1 and E is phenyl substituted with a C₁-C₃ alkoxy group or a C₁-C₆ alkyl group.

In another embodiment, R₃ is ¹⁸F, m is 1 and E is phenyl substituted with a C₁-C₃ alkoxy group or a C₁-C₆ alkyl group.

In a further embodiment, R₃ is C₁-C₆ alkyl or chloro, m is 1 or 2, Y is absent, X is (CH₂)ₙ, wherein n is 1 or 2, and E is a phenyl group to which is attached one or more C₁-C₆ alkoxy groups, said C₁-C₆ alkoxy groups comprising one or more fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

In a further embodiment, R₃ is C₁-C₆ alkyl or chloro, m is 1 or 2 and E is a phenyl group to which is attached one or more C₁-C₆ alkoxy groups, said C₁-C₆ alkoxy groups comprising between one and three fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

In another embodiment, R₃ is methyl or chloro, m is 1 or 2 and E is a phenyl group to which is attached one or more C₁-C₆ alkoxy groups, said C₁-C₆ alkoxy groups comprising one or more fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

In another embodiment, R₃ is methyl or chloro, m is 1 or 2 and E is a phenyl group to which is attached one or more C₁-C₆ alkoxy groups, said C₁-C₆ alkoxy groups comprising between one and three fluoro substituents (e.g. 1, 2, or 3 ¹⁹F substituents or an ¹⁸F substituent or a combination of a ¹⁹F substituent and an ¹⁸F substituent or a combination of two ¹⁹F substituents and an ¹⁸F substituent).

In another embodiment, R₃ is methyl or chloro, m is 1 or 2 and E is a phenyl group to which is attached a C₁-C₆ alkoxy group, said C₁-C₆ alkoxy group comprising a single fluoro substituent (¹⁹F or ¹⁸F).

In another embodiment, R₃ is methyl or chloro, m is 1 or 2 and E is a phenyl group to which is attached an alkoxy group of the following formula: -O(CH₂)ₙF, wherein n is a number between 1 and 4.

In another embodiment, R₃ is methyl or chloro, m is 1 or 2 and E is a phenyl group to which is attached an alkoxy group of the following formula: -O(CH₂)ₙ¹⁸F, wherein n is a number between 1 and 4.

In one embodiment, the alkoxy group attached to the phenyl group is in the para position.

In a particular embodiment of the invention, R₃ may be chloro or C₁-C₆ alkyl, m may be 1 or 2, G may be CH, D may be CH, M may be N, J may be C, L may be N, Y may be absent, X may be CH₂ or CH₂CH₂, Z may be NR₁R₂, wherein R₁ and R₂ are independently selected from hydrogen, pyridyl, phenyl, C₁-C₄ alkyl, or R₁ and R₂ together with the nitrogen to which they are attached form a pyrrolidine ring, and E may be phenyl group substituted with a single substituent of the formula - O(CH₂)ₙF, wherein n is a number between 1 and 4.

In a particular embodiment of the invention, R₃ may be chloro or C₁-C₆ alkyl, m may be 1 or 2, G may be CH, D may be CH, M may be N, J may be C, L may be N, Y may be absent, X may be CH₂ or CH₂CH₂, Z may be NR₁R₂, wherein R₁ and R₂ are independently selected from hydrogen, pyridyl, phenyl, C₁-C₄ alkyl, or R₁ and R₂ together with the nitrogen to which they are attached form a pyrrolidine ring, and E may be phenyl group substituted with a single substituent of the formula - O(CH₂)ₙ¹⁸F, wherein n is a number between 1 and 4.

In another embodiment of the invention, R₃ may be methyl, m may be 1 or 2, G may be CH, D may be N, M may be C, J may be N, L may be N, Y may be absent, X may be CH₂ or CH₂CH₂, Z may be NR₁R₂, wherein R₁ and R₂ are independently C₁-C₆ alkyl, and E may be a phenyl group substituted with a single substituent of the formula -O(CH₂)ₙF, wherein n is a number between 1 and 4.

In a further particular embodiment of the invention, R₃ may be fluoro, m may be 1, G may be CH, D may be N, M may be N, J may be C, L may be N, Y may be absent, X may be CH₂, Z may be NR₁R₂, wherein R₁ and R₂ are independently C₁-C₄ alkyl, and E may be a phenyl group substituted with a C₁-C₃ alkoxy group or a C₁-C₆ alkyl group.

In a further particular embodiment of the invention, R₃ may be fluoro, m may be 1, G may be CH, D may be N, M may be N, J may be C, L may be N, Y may be NH, X may be CH₂ or CH₂CH₂, Z may be phenyl, and E may be a phenyl group substituted with one or two C₁-C₆ alkyl groups.

In another particular embodiment of the invention, R₃ may be chloro, m may be 1 or 2, G may be CH, D may be CH, M may be N, J may be C, L may be N, Y may be absent, X may be CH₂ or CH₂CH₂, Z may be NR₁R₂, wherein R₁ and R₂ are independently selected from hydrogen, phenyl and C₁-C₄ alkyl, and E may be fluoropyridine, fluoropyridazine or fluoropyrimidine.

In another particular embodiment of the invention, R₃ may be chloro, m may be 1 or 2, G may be CH, D may be CH, M may be N, J may be C, L may be N, Y may be absent, X may be CH₂ or CH₂CH₂, Z may be NR₁R₂, wherein R₁ and R₂ are independently selected from hydrogen, phenyl and C₁-C₄ alkyl, and E may be ¹⁹fluoropyridine, ¹⁹fluoropyridazine or ¹⁹fluoropyrimidine.

In another particular embodiment of the invention, R₃ may be chloro, m may be 1 or 2, G may be CH, D may be CH, M may be N, J may be C, L may be N, Y may be absent, X may be CH₂ or CH₂CH₂, Z may be NR₁R₂, wherein R₁ and R₂ are independently selected from hydrogen, phenyl and C₁-C₄ alkyl, and E may be ¹⁸fluoropyridine, ¹⁸fluoropyridazine or ¹⁸fluoropyrimidine.

In another particular embodiment of the invention, R₃ may be chloro, m may be 1 or 2, G may be CH, D may be CH, M may be N, J may be C, L may be N, Y may be absent, X may be CH₂ or CH₂CH₂, Z may be NR₁R₂, wherein R₁ and R₂ are independently selected from hydrogen, C₁-C₄ alkyl and fluoropyridyl, and E may be a phenyl group substituted with a C₁-C₆ alkoxy group.

In another particular embodiment of the invention, R₃ may be chloro, m may be 1 or 2, G may be CH, D may be CH, M may be N, J may be C, L may be N, Y may be absent, X may be CH₂ or CH₂CH₂, Z may be NR₁R₂, wherein R₁ and R₂ are independently selected from hydrogen, C₁-C₄ alkyl and ¹⁸fluoropyridyl, and E may be a phenyl group substituted with a C₁-C₆ alkoxy group.

In another particular embodiment of the invention, R₃ may be chloro, m may be 1 or 2, G may be CH, D may be CH, M may be N, J may be C, L may be N, Y may be absent, X may be CH₂ or CH₂CH₂, Z may be NR₁R₂, wherein R₁ and R₂ are independently selected from hydrogen, C₁-C₄ alkyl and ¹⁹fluoropyridyl, and E may be a phenyl group substituted with a C₁-C₆ alkoxy group.

In an embodiment of the invention:
G is C,
D, M, J and L are, respectively, N, C, N and N; C, N, C and N; or N, N, C and N,
R₃ is Cl, F or Me
m is 1 or 2
E is 4-(2-fluoroethoxy)phenyl, 4-(3-fluoropropoxy)phenyl, 4-t-butylphenyl, 4-methoxyphenyl, 2-fluoropyridin-5-yl or 2-fluoropyridazin-5-yl
X is CH₂ (i.e. n is 1)
Y is absent or is NH, and
Z is NMe₂, NEt₂, NPr₂, NMeH, NHiPr, NMeEt, N-pyrrolidinyl, Ph, NMePh or NMe(5-(2-fluoro)pyridinyl).

In a second aspect the present invention provides a compound of formula (Ia): 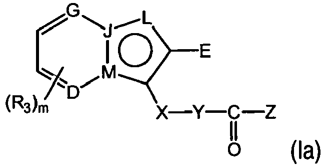 wherein,
D, G, and L are independently selected from the group consisting of: CH, C and N, and J and M are independently selected from the group consisting of C and N provided that at least one of J and M is C, wherein at least two of D, G, M, J and L are N;
X is selected from the group consisting of: O, NH, (CH₂)ₙ and S;
Y is absent, or is selected from the group consisting of: O, NH, (CH₂)ₙ, and S;
Z is selected from the group consisting of: NR₁R₂ and aryl;
R₁ and R₂ are independently selected from the group consisting of: hydrogen, C₁-C₁₀ alkyl, C₂-C₁₀ alkenyl, C₂-C₁₀ alkynyl, (CH₂)ₙaryl, aryl and heteroaryl, each of which may optionally be substituted with one or more of the following substituents: chloro, bromo, iodo, C₁-C₆ alkyl and hydroxy;
or R₁ and R₂, together with the nitrogen to which they are attached, form a heterocyclic ring having between 3 and 7 ring members, which may optionally be substituted with one or more of the following substituents: chloro, bromo, iodo and C₁-C₆ alkyl;
R₃ is selected from the group consisting of: chloro, bromo, iodo, C₁-C₁₀ alkyl and O-(C₁-C₁₀ alkyl), wherein the C₁-C₁₀ alkyl group is optionally substituted;
E is an aryl group or a heteroaryl group, wherein each group is optionally substituted with a chloro, bromo or iodo substituent, and/or with one or more of the following substituents: C₁-C₆ alkyl, C₂-C₁₀ alkenyl, C₂-C₁₀ alkynyl, QC₁-C₁₀ alkyl, QC₁-C₁₀ alkenyl, QC₂-C₁₀ alkynyl, Q(CH₂)ₚ-Q-(CH₂)_{q}CH₃ or Q(CH₂)ₚ-Q-(CH₂)_{q}-Q-(CH₂)ᵣCH₃, each of which may be substituted with one or more chloro, bromo, iodo or hydroxy substituents, and wherein p, q and r are, independently, integers between 1 and 3, and wherein Q is selected from the group consisting of: NH, O and S;
m is a number between 0 and 3 (e.g. 0, 1, 2 or 3) wherein if m is 2 or 3, the R₃ groups may be the same or may be different;
n is a number between 1 and 4, e.g. 1, 2, 3 or 4 (wherein n in X may be the same as or different to n in Y);
with the proviso that E is not 4-iodophenyl.

The invention provides a compound of formula (Ia) wherein:
D, G, and L are independently selected from the group consisting of: CH, C and N, and J and M are independently selected from the group consisting of C and N provided that at least one of J and M is C, wherein at least two of D, G, M, J and L are N;
X is independently selected from the group consisting of: O, NH, (CH₂)ₙ and S;
Y is absent, or is independently selected from the group consisting of: O, NH and (CH₂)ₙ, and S;
Z is selected from the group consisting of: NR₁R₂ and aryl;
R₁ and R₂ are independently selected from the group consisting of: hydrogen, C₁-C₁₀ alkyl, C₂-C₁₀ alkenyl, C₂-C₁₀ alkynyl, (CH₂)ₙaryl, aryl and heteroaryl, each of which may optionally be substituted with one or more of the following substituents: chloro, bromo, iodo, C₁-C₆ alkyl and hydroxy;
or R₁ and R₂, together with the nitrogen to which they are attached, form a heterocyclic ring having between 3 and 7 ring members, which may optionally be substituted with one or more of the following substituents: chloro, bromo, iodo and C₁-C₆ alkyl;
R₃ is selected from the group consisting of: chloro, bromo, iodo and C₁-C₁₀ alkyl;
E may be an aryl group or a heteroaryl group, which is substituted with a chloro, bromo or iodo substituent, and/or one or more of the following substituents: C₁-C₆ alkyl, C₂-C₁₀ alkenyl, C₂-C₁₀ alkynyl, QC₁-C₁₀ alkyl, QC₁-C₁₀ alkenyl, QC₂-C₁₀ alkynyl, Q(CH₂)ₚ-Q-(CH₂)_{q}CH₃ or Q(CH₂)ₚ-Q-(CH₂)_{q}-Q-(CH₂)ᵣCH₃, each of which may be substituted with one or more chloro, bromo, iodo or hydroxy substituents, and wherein p, q and r are integers between 1 and 3, and wherein Q is independently selected from the group consisting of: NH, O and S;
m is a number between 0 and 3;
n is a number between 1 and 4;
with the proviso that E is not -Phl.

In some embodiments E is not -Phl

In an embodiment of the invention Q may be oxygen.

In another embodiment of the invention, m is 1 or 2, and n is 1.

In a further embodiment of the invention, p may be 1 or 2, q and r may be 1, and Q may be oxygen.

In some embodiments, L is N. In some embodiments one of M and J is N.

In one embodiment, M and L are N, D and G are CH and J is C. In another embodiment, D, M and L are N, J is C and G is CH. In an alternative embodiment, D, J and L are N, M is C and G is CH.

R₃ may be attached to a carbon atom.

R₃ may be selected from the group consisting of: chloro, bromo and iodo and C₁-C₆ alkyl. In another embodiment, R₃ may be selected from the group consisting of chloro and C₁-C₃ alkyl. In a further embodiment, R₃ may be chloro or methyl and m may be 1 or 2. Alternatively, R₃ may be chloro and m may be 1 or 2, or R₃ may be fluoro (¹⁹F or ¹⁸F) and m may be 1 or 2.

In one embodiment, Y is absent and X is (CH₂)ₙ, wherein n is 1 or 2.

In another embodiment, X and Y may be independently selected from the group consisting of: NH and (CH₂)ₙ, wherein n is 1.

Y may be (CH₂)ₙ and X may be NH, wherein n is a number between 1 and 3.

In one embodiment, -X-Y-C(O)-Z may have a structure selected from the group consisting of: -(CH₂)ₙ-C(O)-Z, wherein n is 1 or 2, or -(CH₂)ₙ-NH-C(O)-Z, wherein n is 1 or 2, and -NH-(CH₂)ₙ-C(O)-Z, wherein n is 1 or 2.

Z may be NR₁R₂ or phenyl.

R₁ and R₂ may be independently selected from the group consisting of: hydrogen C₁-C₆ alkyl, benzyl, phenyl, pyridyl, pyrimidinyl or pyridazinyl, and where R₁ and R₂, together with the nitrogen to which they are attached, form a heterocyclic ring having between 4 and 6 ring members. The heterocyclic ring may be a pyrrolidine ring. The C₁-C₆ alkyl group, the heterocyclic group, the phenyl group, the pyridyl group, the pyrmidinyl group, the pyridazinyl group or the benzyl group may optionally be substituted with between 1 and 3 chloro, bromo, iodo or hydroxy substituents.

R₁ and R₂ may be independently selected from the group consisting of: hydrogen C₁-C₆ alkyl, phenyl, pyridyl, pyrimidinyl or pyridazinyl, and where R₁ and R₂, together with the nitrogen to which they are attached, form a heterocyclic ring having between 4 and 6 ring members. The heterocyclic ring may be a pyrrolidine ring. The C₁-C₆ alkyl group, the heterocyclic group, the phenyl group, the pyridyl group, the pyrmidinyl group, or the pyridazinyl group may optionally be substituted with between 1 and 3 chloro, bromo, iodo or hydroxy substituents.

In another embodiment, R₁ and R₂ may be independently selected from the group consisting of: hydrogen, phenyl, pyridyl, pyrimidinyl or pyridazinyl, methyl, ethyl, propyl, isopropyl, butyl, *t*-butyl and pentyl, each of which may optionally be substituted with between 1 and 3 chloro, bromo, iodo or hydroxy substituents.

In an alternative embodiment, R₁ and R₂ may be selected from the group consisting of: hydrogen, phenyl, pyridyl, pyrimidinyl, pyridazinyl, methyl, ethyl, isopropyl, propyl, butyl and *t*-butyl, and wherein R₁ and R₂ together with the nitrogen to which they are attached, form a pyrrolidine ring, each of which may optionally be substituted with between 1 and 3 chloro, bromo, iodo or hydroxy substituents.

R₃ may be selected from the group consisting of: halogen and C₁-C₆ alkyl, optionally substituted (e.g. with one or more halogens such as fluorine, chlorine, bromine and/or iodine). R₃ may be methyl. It may be methoxy. It may be trifluoromethyl. It may be trifluoromethoxy.

When E is phenyl, E may not have an iodo substituent attached directly to it in the 4 position. E may be substituted phenyl which does not have an iodo substitutent attached directly to the 4 position. It may be substituted phenyl which does not have an iodo substituent attached directly to the phenyl ring.

E may be a phenyl group, a pyridyl group, a pyrimidinyl group or a pyridazinyl group, each of which may optionally be substituted with one or more chloro, bromo, iodo or hydroxy substituents, and/or substituted with one or more of the following substituents: C₁-C₆ alkyl, C₂-C₆ alkenyl or C₁-C₁₀ alkoxy, each of which may optionally be substituted with one or more of the following substituents: chloro, bromo, iodo and hydroxy.

E may be a phenyl group, a pyridyl group, a pyrimidinyl group or a pyridazinyl group, each of which may optionally be substituted with between one and three chloro, bromo, iodo or hydroxy substituents, or substituted with one of the following substituents: C₁-C₆ alkyl, C₂-C₆ alkenyl or C₁-C₆ alkoxy, either of which may optionally be substituted with between one and three chloro, bromo, iodo or hydroxy substituents.

E may be a phenyl group, a pyridyl group, a pyrimidinyl group or a pyridazinyl group, each of which may optionally be substituted with between one and three chloro, bromo, iodo or hydroxy substituents.

In a third aspect, the present invention provides a compound of the first aspect or the second aspect that is radiolabelled with ¹⁸F. The invention provides a compound of the first aspect that is radiolabelled with ¹⁸F.

In a fourth aspect, the present invention provides a pharmaceutical composition comprising a compound of the first or second aspects, said compound being not radiolabelled with ¹⁸F, together with at least one pharmaceutically acceptable carrier, diluent, excipient or adjuvant.

In a fifth aspect, the present invention provides a pharmaceutical composition comprising a compound of the third aspect, together with at least one pharmaceutically acceptable carrier, diluent, excipient or adjuvant.

In a sixth aspect, the present invention provides a method for diagnosis of a disorder in a mammal characterised by an abnormal density of peripheral benzodiazepine receptors, the method including the steps of:
(i) administering to the mammal a compound of the third aspect in an amount sufficient to allow a detectable image of the location of the radiolabel in the body of the mammal to be recorded.
(ii) diagnosing the presence or absence of the disorder from the image.

The method of the sixth aspect may comprise recording an image of the distribution of the radiolabel in at least part of the body of the mammal.

The method for diagnosis of the sixth aspect may be PET. Thus step (ii) may comprise conducting positron emission tomography of the body or part thereof of the mammal and determining from a resulting image the presence or absence of the disorder.

In a seventh aspect, the present invention provides a method for the treatment of a disorder characterised by an abnormal density of peripheral benzodiazepine receptors in a mammal in need of said treatment, the method comprising administering to the mammal a therapeutically effective amount of a compound of the first aspect or the second aspect, said compound being not radiolabelled with ¹⁸F, or of a pharmaceutical composition of the fourth aspect.

In an eighth aspect, the invention provides the use of a compound of the third aspect in the manufacture of a diagnostic composition in the diagnosis of a disorder in a mammal characterised by an abnormal density of peripheral benzodiazepine receptors.

In a ninth aspect, the invention provides the use of a compound of the first aspect or the second aspect in the manufacture of a medicament for the treatment of a disorder characterised by an abnormal density of peripheral benzodiazepine receptors in a mammal in need of said treatment, said compound being not radiolabelled with ¹⁸F.

The compound of, or used in, the third, fifth, sixth or eighth aspects may be selected from the group consisting of: PBRs 099*, 102*, 103*, 104*, 105*, 106*, 107*, 110*, 111*, 130*, 120*, 132*, 133*, 136*, 139*, 143*, 146*, 147*, 149*, 080*, 081*, 082*, 083* (as defined herein), or any combination thereof. These compounds are all radiolabelled with ¹⁸F.

The compound of the fourth, seventh or ninth aspects may be selected from the group consisting of: PBRs 099, 102, 103, 104, 105, 106, 107, 110, 111, 130, 120, 132, 133, 136, 139, 143,146, 147,149, 080, 081, 082, 083,115,138, 145, 155, 142, 153, 154, 156, 131, 134, 135, 141, 125, 126, 137, 98, 139, 132, 133, 117, 118, 121, 123, 124, 128, 129, 116, 119, 140, 144, 101, 113, and 114, or any combination thereof. None of these compounds are radiolabelled with ¹⁸F.

In a tenth aspect, the invention provides the use of a compound according the first or the second aspect for the treatment of a condition selected from the group consisting of apoptosis, neurodegenerative disorders, including Huntington's disease, Alzheimer's disease, anxiety, stress, emotional disturbances, cognitive impairment, stroke, and cerebral ischemia; tumours, such as glioma, and carcinoma of the ovary, colon, breast, prostate, brain and adrenals; neurotoxic injury, including that associated with anoxia or ischemia which results from stroke or cardiac arrest; and cognitive disorders, or for cognitive enhancement, said compound being not radiolabelled with ¹⁸F.

In an eleventh aspect, the invention provides a process for making a compound according to the first, second or third aspect, said process being substantially as described herein with reference to Schemes 1 to 8 (*vide infra*).

### Brief Description of the Drawings

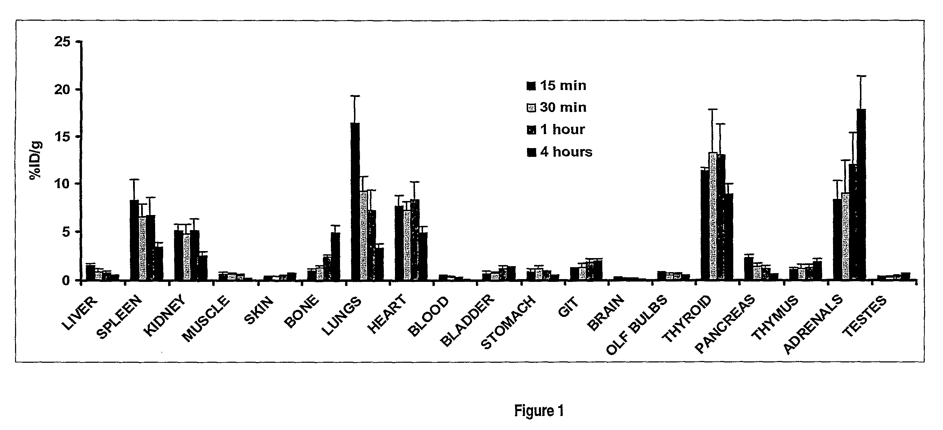

Figure 1 shows a plot of the biodistribution of compound PBR111 in rats (n = 4) over 4 hours. The plot depicts percent of injected does per gram of tissue v. time.

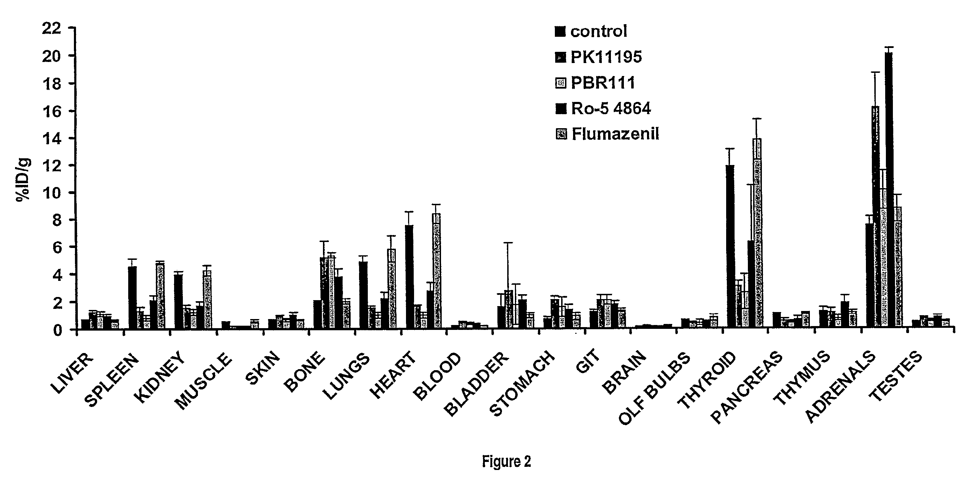

Figure 2 indicates reduced uptake of [¹⁸F]PBR111 radiotracer distribution in organs expressing the PBR receptor when pre-treated with the selective PBR ligands PK 11195, Ro 5-4864 and the non-radioactive analogue of PBR111 all at 1 mg/kg. The central benzodiazepine ligand flumazenil does not compete and hence not reduce the [¹⁸F]PBR 111 activity in these tissues.

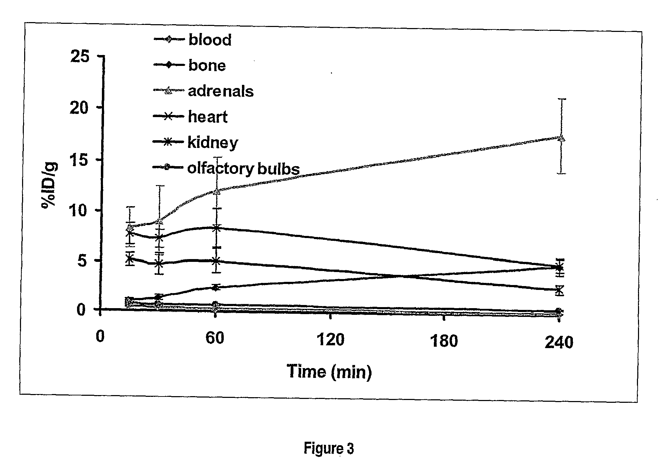

Figure 3 indicates the percentage ID of [¹⁸F]PBR111/gram of tissue v. time. The plot indicates that the tracers follow normal distribution of PBR expression over time.

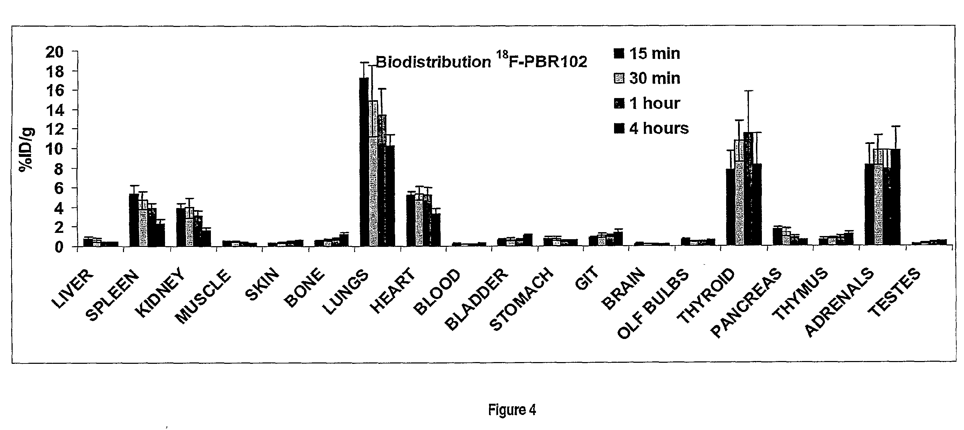

Figure 4 shows biodistribution of [¹⁸F]PBR102 in rodents over a 4 hour time period. The plot shows percentage uptake of [¹⁸F]PBR102.

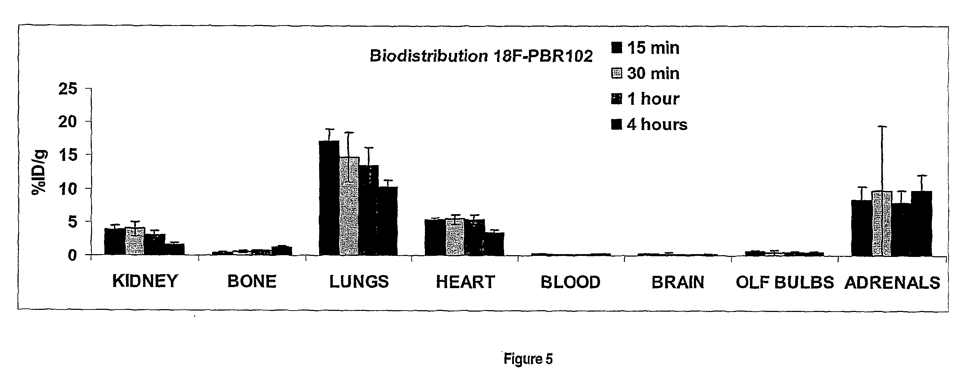

Figure 5 shows the biodistribution data of a selection of tissues from Figure 4 that are relevant to PBR expression.

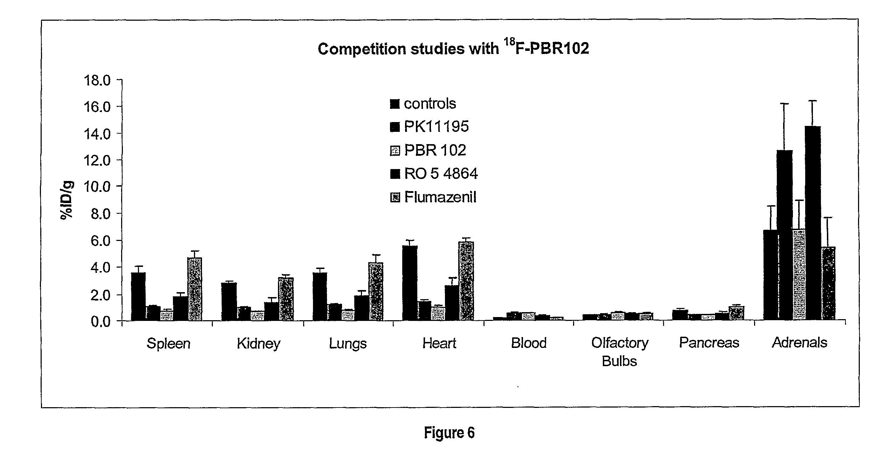

Figure 6 shows the blocking effect on the uptake of [¹⁸F]PBR102 by the selective PBR ligands PK 11195, Ro 5-4864, and the non-radioactive analogue PBR102 all at 1mg/kg. The central benzodiazepine ligand flumazenil does not compete and hence does not reduce the [¹⁸F]PBR102 uptake in these tissues.

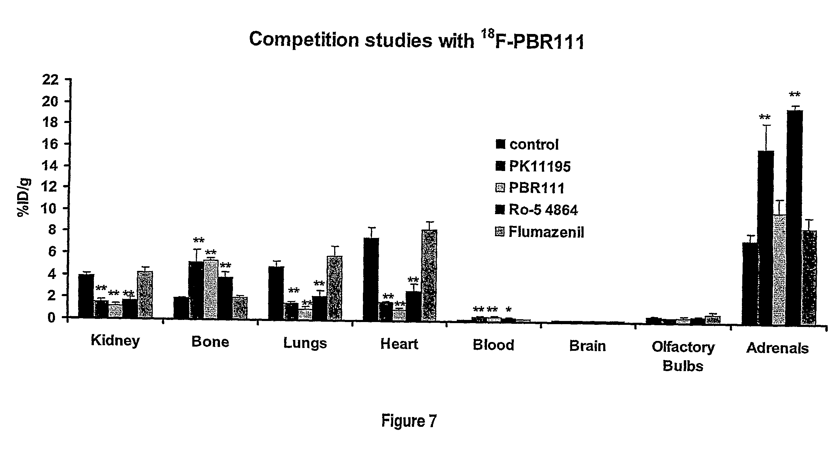

Figure 7* shows the blocking effect on the uptake of [¹⁸F]PBR111 by the selective PBR ligands PK 11195, Ro 5-4864, and the non-radioactive analogue PBR111 all at 1mg/kg. The central benzodiazepine ligand flumazenil does not compete and hence does not reduce the [¹⁸F]PBR111 uptake in these tissues.

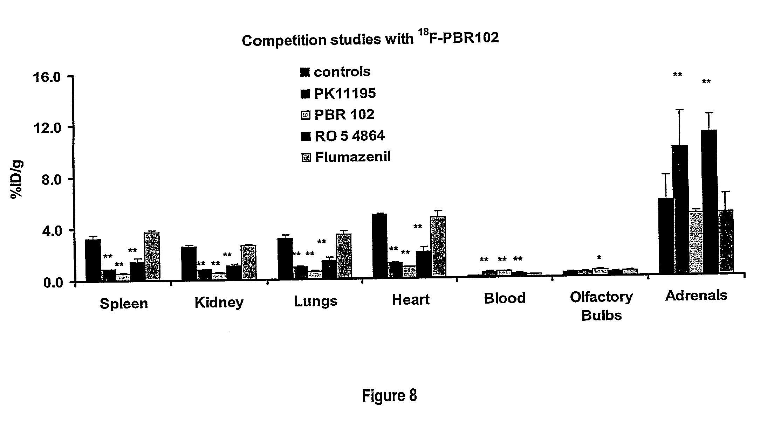

Figure 8* shows the blocking effect on the uptake of [¹⁸F]PBR102 by the selective PBR ligands PK 11195, Ro 5-4864 and the non-radioactive analogue PBR102 all at 1mg/kg. The central benzodiazepine ligand flumazenil does not compete and hence does not reduce the [¹⁸F]PBR102 uptake in these tissues.

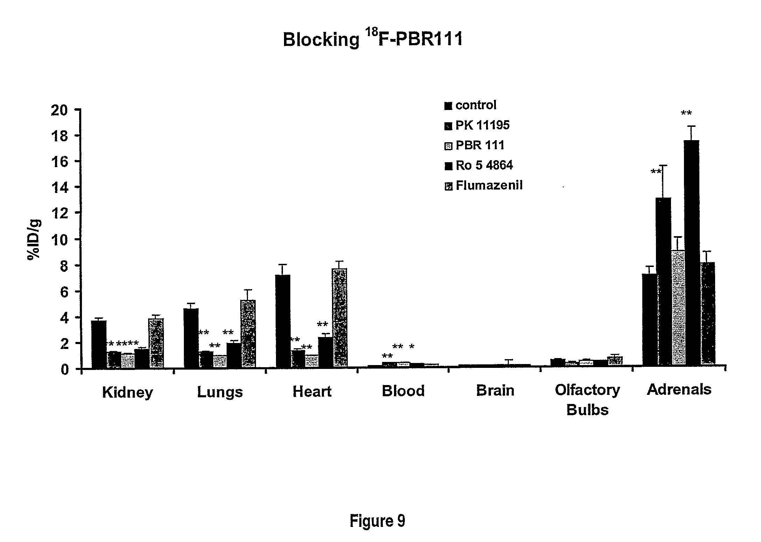

Figure 9* shows the blocking effect on the uptake of [¹⁸F]PBR111 by the selective PBR ligands PK 11195, Ro 5-4864 and the non-radioactive analogue PBR111 all at 1mg/kg. The central benzodiazepine ligand flumazenil does not compete and hence does not reduce the [¹⁸F]PBR111 uptake in these tissues.

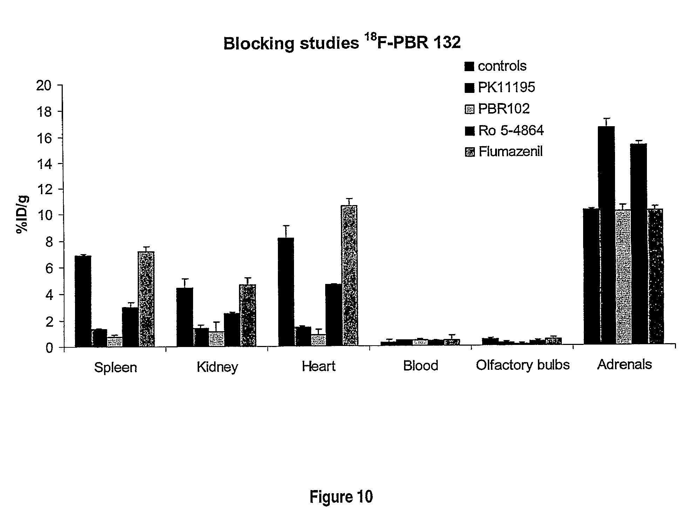

Figure 10 shows the blocking effect on the uptake of [¹⁸F]PBR132 by the selective PBR ligands PK 11195, Ro 5-4864 and the non-radioactive analogue PBR132 all at 1mg/kg. The central benzodiazepine ligand flumazenil does not compete and hence does not reduce the [¹⁸F]PBR132 uptake in these tissues.

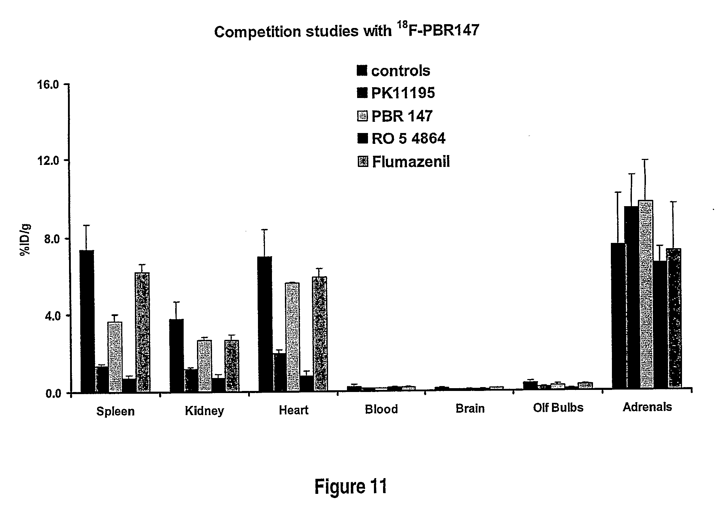

Figure 11 shows the blocking effect on the uptake of [¹⁸F]PBR147 by the selective PBR ligands PK 11195, Ro 5-4864 and the non-radioactive analogue PBR147 all at 1 mg/kg. The central benzodiazepine ligand flumazenil does not compete and hence does not reduce the [¹⁸F]PBR147 uptake in these tissues.

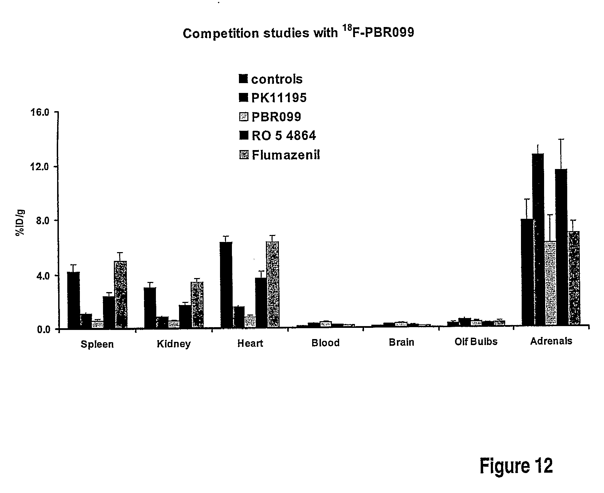

Figure 12 shows the blocking effect on the uptake of [¹⁸F]PBR099 by the selective PBR ligands PK 11195, Ro 5-4864 and the non-radioactive analogue PBR099 all at 1 mg/kg. The central benzodiazepine ligand flumazenil does not compete and hence does not reduce the [¹⁸F]PBR099 uptake in these tissues.

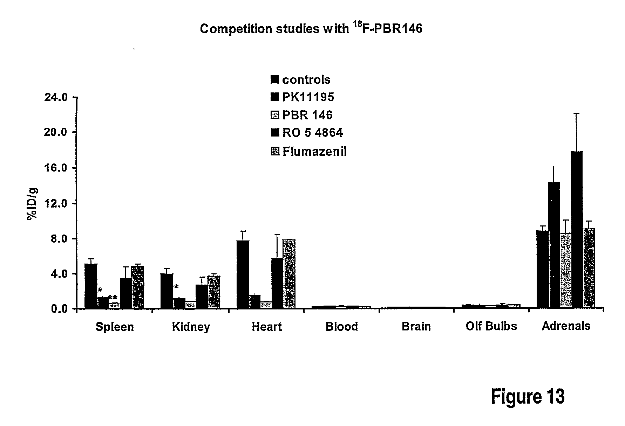

Figure 13 shows the blocking effect on the uptake of [¹⁸F]PBR146 by the selective PBR ligands PK 11195, Ro 5-4864 and the non-radioactive analogue PBR146 all at 1mg/kg. The central benzodiazepine ligand flumazenil does not compete and hence does not reduce the [¹⁸F]PBR146 uptake in these tissues.

* The results in the graphs showing "stars" relate to statistical analysis of the results, i.e. Results from the competition studies were analysed by one-way analysis of variance (ANOVA) for comparing the tissue radioactivity concentrations in the treated animals (n = 3-4) and in the controls (n = 4). The criteria for significance were **p < 0.01 (highly significant) and *p < 0.05 (significant). No stars implies not statistical difference from normals or controls.

### Detailed Description of the Invention

### Definitions

The following are some definitions that may be helpful in understanding the description of the present invention. These are intended as general definitions and should in no way limit the scope of the present invention to those terms alone, but are put forth for a better understanding of the following description.

Unless the context requires otherwise or it is specifically stated to the contrary, integers, steps, or elements of the invention recited herein as singular integers, steps or elements clearly encompass both singular and plural forms of the recited integers, steps or elements.

Throughout this specification, unless the context requires otherwise, the word "comprise", or variations such as "comprises" or" comprising", will be understood to imply the inclusion of a stated step or element or integer or group of steps or elements or integers, but not the exclusion of any other step or element or integer or group of elements or integers. Thus, in the context of this specification, the term "comprising" means "including principally, but not necessarily solely".

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations or any two or more of said steps or features.

As used herein, the term "therapeutically effective amount(s)" includes within its meaning a sufficient but non-toxic amount of a compound or composition of the invention to provide the desired therapeutic effect. The exact amount required will vary from subject to subject depending on factors such as the species being treated, the age and general condition of the subject, the severity of the condition being treated, the particular compound being administered, the mode of administration and so forth. Thus, it is not possible to specify an exact "therapeutically effective amount", however for any given case an appropriate "therapeutically effective amount" may be determined by one of ordinary skill in the art using only routine trial and experimentation.

As used herein, the term "treatment" refers to any and all uses which remedy a disease state or symptoms, prevent the establishment of a disease, or otherwise prevent, hinder, retard or reverse the progression of disease or other undesirable symptoms in any way whatsoever.

As used herein, the term "alkyl" includes within its meaning monovalent straight chain or branched chain saturated aliphatic groups having from 1 to 10 carbon atoms, e.g. 1, 2, 3, 4, 5, 6, 7, 8, 9 or 10 carbon atoms. The alkyl group may have from 1 to 4, 1 to 6 or 1 to 8 carbon atoms. Examples of such alkyl groups include but are not limited to: methyl, ethyl, propyl, isopropyl, butyl, isobutyl, sec-butyl, tert-butyl, amyl, isoamyl, sec-amyl, 1,2-dimethylpropyl, 1,1-dimethyl-propyl, hexyl, 4-methylpentyl, 1-methylpentyl, 2-methylpentyl, 3-methylpentyl, 1,1-dimethylbutyl, 2,2-dimethylbutyl, 3,3-dimethylbutyl, 1,2-dimethylbutyl, 1,3-dimethylbutyl, 1,2,2-trimethylpropyl and 1,1,2-trimethylpropyl, octyl, nonyl, decyl and the like.

As used herein, the term "alkoxy" refers to a group of the formula alkyl-O-, wherein the alkyl group is as defined above. Examples include methoxy, ethoxy, n-propoxy, isopropoxy, tert-butoxy, and the like.

As used herein, the term "alkenyl" includes within its meaning monovalent straight chain or branched chain unsaturated aliphatic hydrocarbon groups having from 2 to 10 carbon atoms, e.g. 2, 3, 4, 5, 6, 7, 8, 9, or 10 carbon atoms. The alkenyl group may have from 2 to 4, 2 to 6 or 2 to 8 carbon atoms. Examples of such alkenyl groups include but are not limited to vinyl, allyl, 1-methylvinyl, butenyl, iso-butenyl, 3-methyl-2-butenyl, 1-pentenyl, cyclopentenyl, 1-methyl-cyclopentyl, 1-hexenyl, 3-hexenyl, cyclohexenyl, 1,3-butadienyl, 1,4-pentadienyl, 1,3-cyclopentadienyl, 1,3-hexadienyl, 1,4-hexadienyl, 1,3-cyclohexadienyl and 1,4-cyclohexadienyl.

As used herein, the term "alkynyl" includes within its meaning monovalent and straight chain or branched chain unsaturated aliphatic hydrocarbon groups having from 2 to 10 carbon atoms (e.g, 2, 3, 4, 5, 6, 7, 8, 9, or 10 carbon atoms) and having at least one triple bond. The alkynyl group may have from 2 to 4, 2 to 6 or 2 to 8 carbon atoms. Examples of such alkynyl groups include but are not limited to: ethynyl,1-propynyl, 1-butynyl, 2-butynyl, 1-methyl-2-butynyl, 3-methyl-1-butynyl,1-pentynyl, 1-hexyl, methylpentynyl, 1-heptynyl, 2-heptynyl,1-octynyl, 2-octynyl,1-nonyl, 1-decynyl, and the like.

As used herein, the term "aryl" refers to monovalent single, polynuclear, conjugated and fused residues of aromatic hydrocarbons having from 6 and 20 carbon atoms. The aryl group may have from 6 to 10, 6 to 12, 6 to 14, 6 to 16 or 6 to 18 carbon atoms. Examples of such aryl groups include but are not limited to phenyl, biphenyl, naphthyl, tetrahydronaphthyl, indenyl, azulenyl, phenantryl, pyrenyl and the like. Any available position of the aromatic residue can be used for attachment to the remainder of the molecule of formula (I).

As used herein, the term "heteroaryl" refers to single, polynuclear, conjugated and fused aromatic radicals having between 6 and 20 ring atoms, wherein 1 to 6, or 1 to 5, or 1 to 4, or 1 to 3, or 1 or 2 of these rings atoms are heteroatoms independently selected from the group consisting of: N, NH, O and S. The heteroaryl group may have from 6 to 10, 6 to 12, 6 to 14, 6 to 16 or 6 to 18 carbon atoms. The heteroaryl group may have 1 to 2, 1 to 3, 1 to 4, 1 to 5 or 1 to 6 heteroatoms. The hetero atoms may be independently selected from the group consisting of: N and NH, N and O, NH and O, N and S, NH and S and S and O. Examples of such heteroaryl groups include but are not limited to pyridyl, pyridyl, pyridyl, thienyl, furyl, pyrryl, indolyl, pyridazinyl, pyrazolyl, pyrazinyl, thiazolyl, pyrimidinyl, quinolinyl, isoquinolinyl, benzofuranyl, benzothienyl, purinyl, quinazolinyl, phenazinyl, acridinyl, benzoxazolyl, benzothiazolyl and the like. Any available position of the heteroaromatic residue can be used for attachment to the remainder of the molecule of formula (I). Nitrogen-containing heteroaryl groups may be substituted at nitrogen with an oxygen atome to form an N-oxide. Sulfur-containing heteroaryl groups may be substituted at sulfur with one or two oxygen atoms to form a sulfoxide or a sulfone respectively.

### Synthesis of compounds

Compounds in accordance with the invention may be synthesised employing combinations of reactions which are generally known in the art. Starting materials for synthesis of compounds of the invention are either commercially available, or are known compounds, or are compounds that can be easily prepared by a person of ordinary skill in the art. Those skilled in the art will also appreciate that various protecting groups may be used throughout a synthesis where a functional group on a molecule may be incompatible with a desired synthetic step. Suitable protecting groups are known to those skilled in the art and have been described, for example, in Greene et al., "Protective Groups in Organic Synthesis"; John Wiley & Sons, 2nd Edition, New York (1991).

Scheme 1 shows one possible synthesis of functionalised imidazo[1,2-a]pyridines starting with an appropriately functionalised acetophenone derivative **1** and an appropriately functionalised aminopyridine **2.** In schemes 1 to 6, R₃ may be selected from the group consisting of: halogen, C₁-C₁₀ alkyl and O-(C₁-C₁₀ alkyl), wherein the C₁-C₁₀ alkyl group is optionally substituted. R₃ may be (R₃)ₘ, where m is a number between 0 and 3. If m is 2 or 3, the R₃ groups may be the same or may be different or, in the case where m=3, two may be the same and one may be different. The reaction proceeds in a solvent such as ethanol, or similar solvent, with heat to provide the imidazo[1,2-a]pyridines **3**.

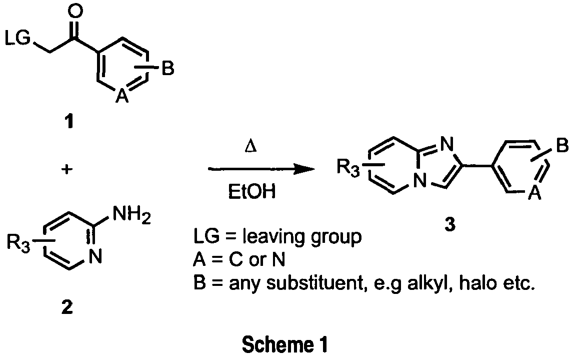

In an alternative synthesis shown in Scheme 2, the side chain at the 3-position of the imidazo[1,2-a]pyridine can be incorporated into the starting acetophenone derivative (compound **1a**). Reaction of **1a** with aminopyridine **2** in ethanol, or a similar solvent, gives compound **3a** which includes an amide side chain.

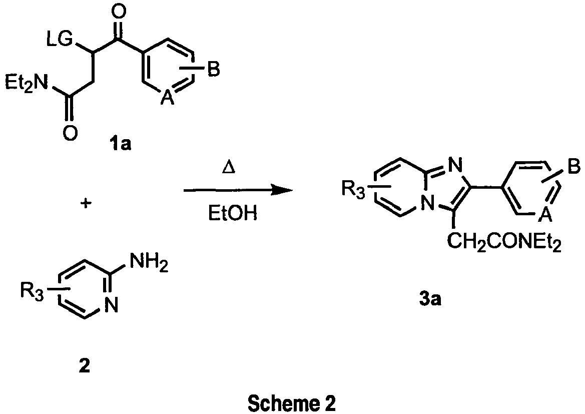

An alternative method for introducing an appropriate side chain, for example - CH₂C(O)NR₁R₂, is shown in Scheme 3. This procedure involves treating an imidazo[1,2-a]pyridine **3,** with formaldehyde and dimethylamine in an aqueous medium to give the methanamine derivative **4,** which is easily converted to the trimethylammonium salt **5** using methyl iodide. Substitution of the trimethylammonium group of **5** with cyanide ion provides the cyanomethyl compound **6,** which may be hydrolysed to the corresponding acid 7 by known methods. The acid **7** may then be converted to a range of amide derivatives **8**.

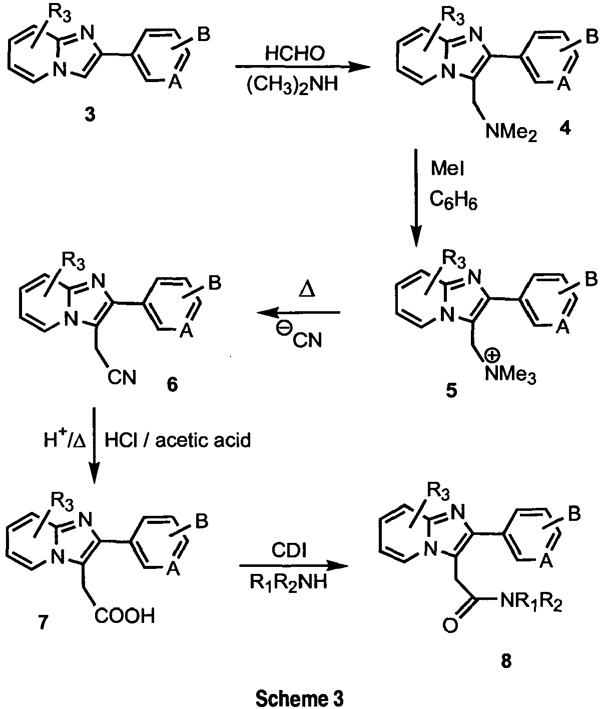

As an alternative, compounds of formula **4** may be demethylated by known methods and acylated to form compounds having a -CH₂NHC(O)R side chain.

Scheme 4 shows another method for preparing an imidazo[1,2-a]pyridine derivative from an appropriately functionalised aryl aldehyde derivative **1b,** and an appropriately functionalised aminopyridine **2,** wherein the resulting product **9** may possess a -NHCOOR side chain. The condensation reaction of compounds **1b** and **2** is carried out in the presence of an isocyanoacetate derivative and scandium(III) triflate. The procedure depicted in Scheme 4 provides compounds of formula **9,** which possess an -NHCOOEt side chain.

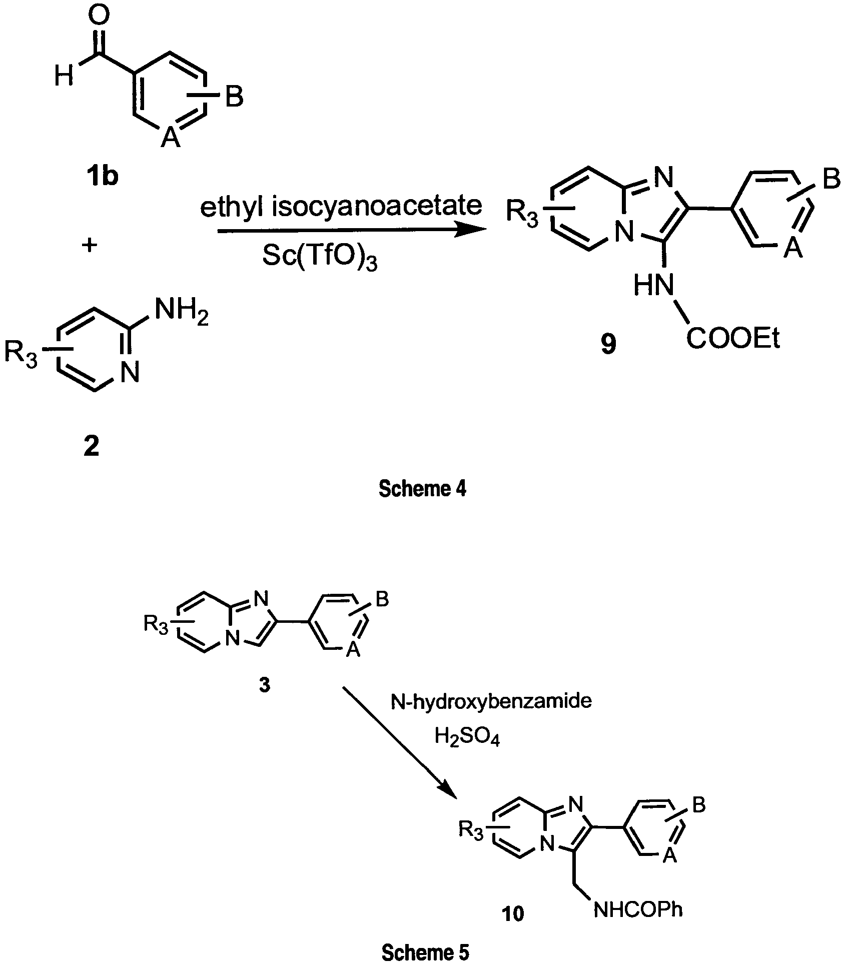

Scheme 5 shows a further alternative procedure for introducing a -CH₂NHC(O)-R group into an imidazo[1,2-a]pyridine compound of formula **3.** In this procedure, the imidazo[1,2-a]pyridine compound is treated with N-hydroxybenzamide in the presence of sulfuric acid.

Compounds of the type represented by formula **11** may be synthesised according to a literature method (Selleri, S. et al. Bioorganic and Medicinal Chemistry 9, 2661-2671 (2001). One example involves condensation of pentane-2,4-dione with 4-(2-(diethylamino) ethyl)-3-(4-methoxyphenyl)-1*H*-pyrazol-5-amine.

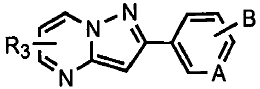

Compounds of the general formula **12** may be prepared by the methods described above in Schemes 1 to 5, except the compound of formula 2 is an appropriately functionalised amino pyridazine as opposed to an amino pyridine.

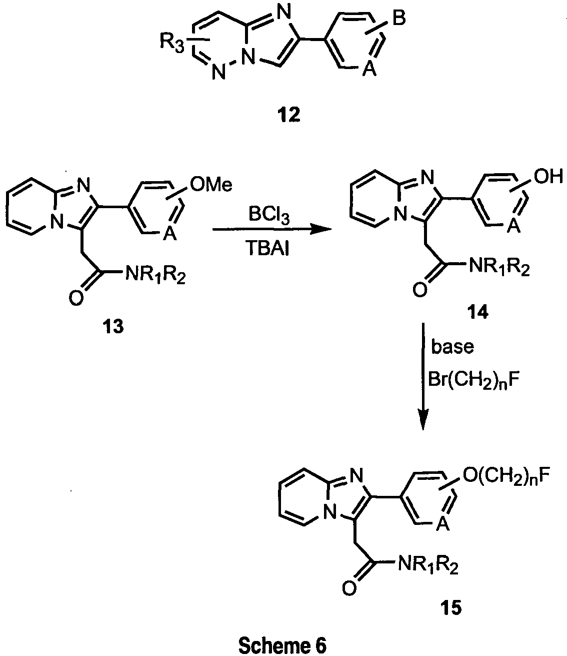

The substituent B depicted in schemes 1 to 5 can be manipulated using simple functional group interconversions known to those skilled in the art, so as to produce a wide range of derivatives once the initial condensation reaction has been performed. Scheme 6 shows one such possibility for preparing a fluorinated ether derivative. The methoxy compound **13** is cleaved by a standard procedure using BCl₃ and tetrabutylammonium iodide to give hydroxy compound **14.** Reaction of compound 14 with an appropriate alkylfluoride in the presence of a base gives the fluorinated compound **15.**

Schemes 7 to 10 set out synthetic routes to selected compounds of the invention. Examples of carrying out the various methods A to J referred to in schemes 7 to 10 can be found in Examples 3 to 17.

It should be noted that the synthesis of the compounds of the present invention is simpler than the synthesis disclosed in WO 99/51594 in that no iodo substituent is necessary on the 2-phenyl ring.

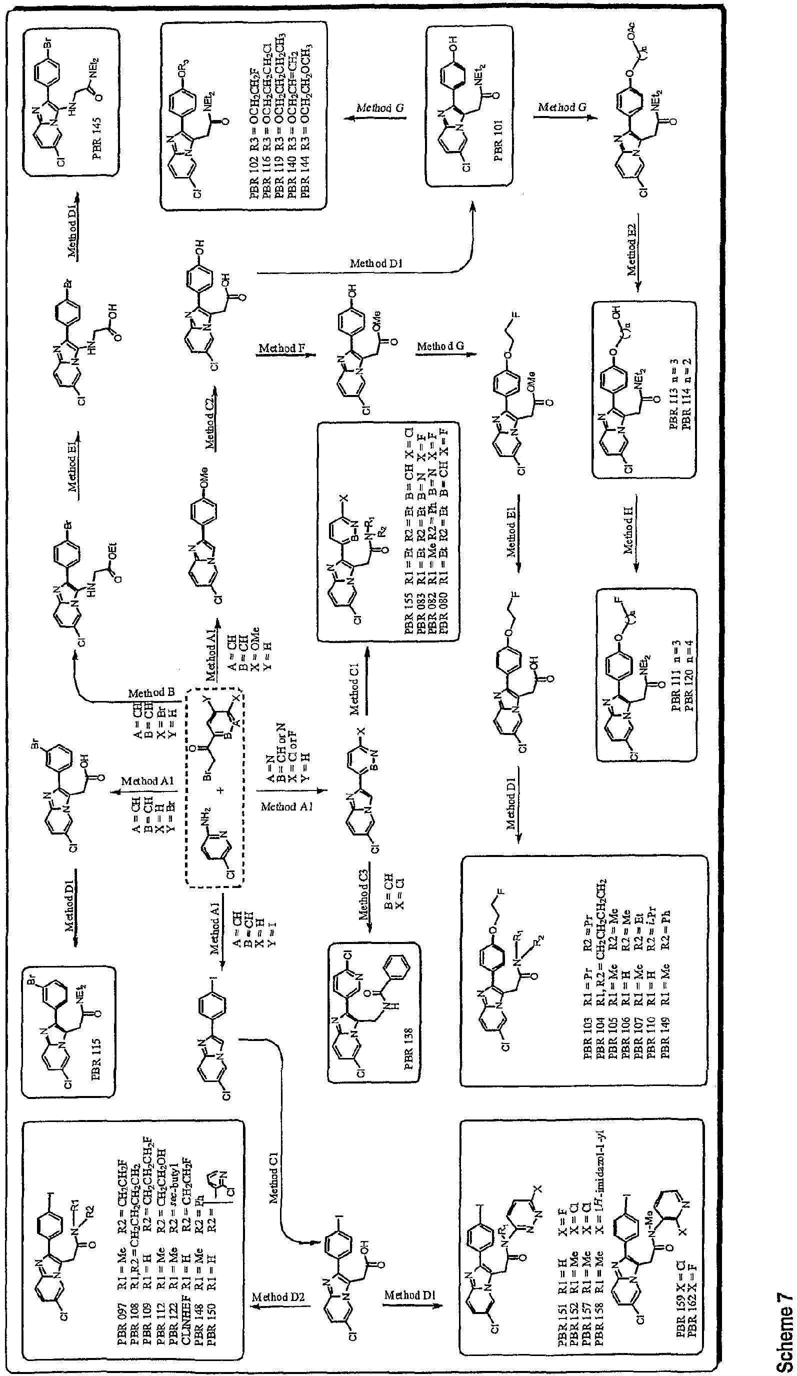

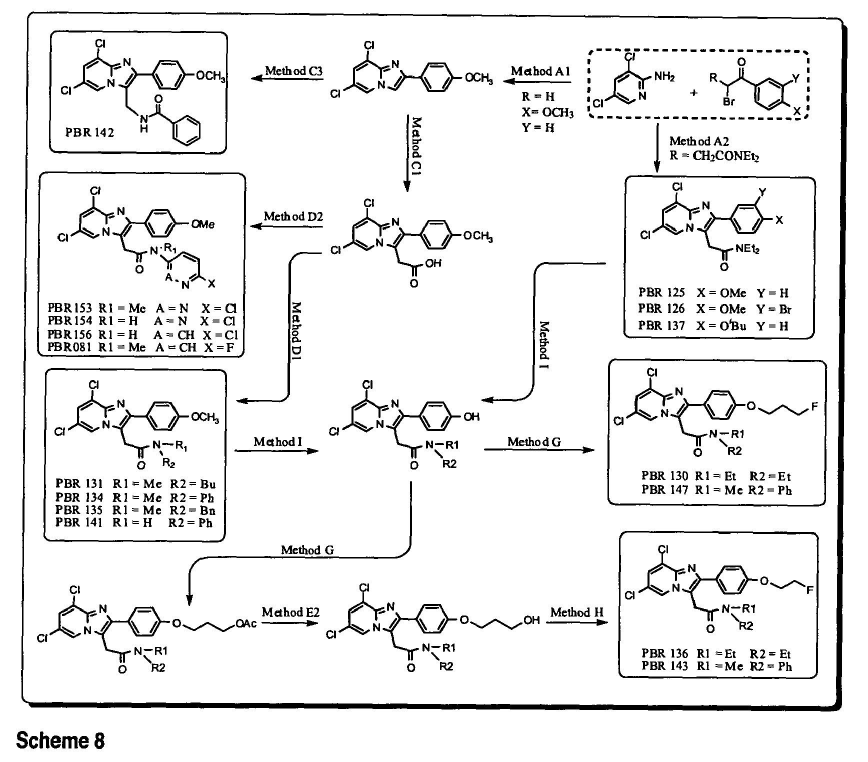

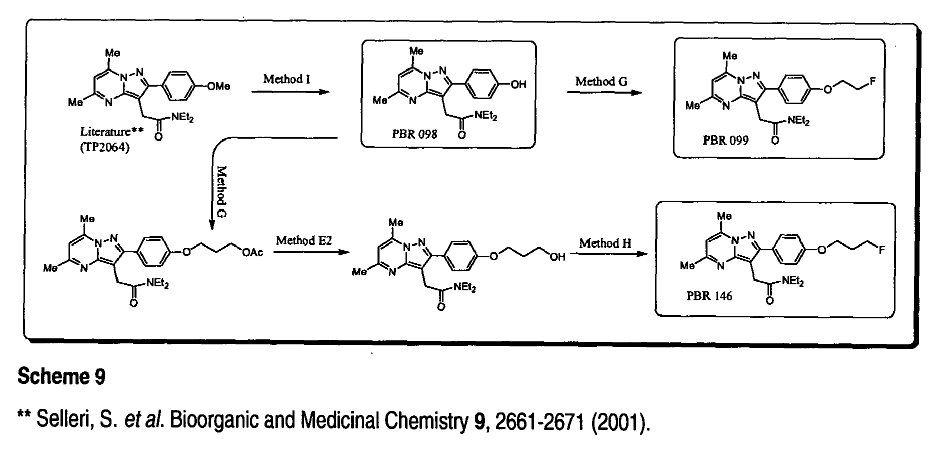

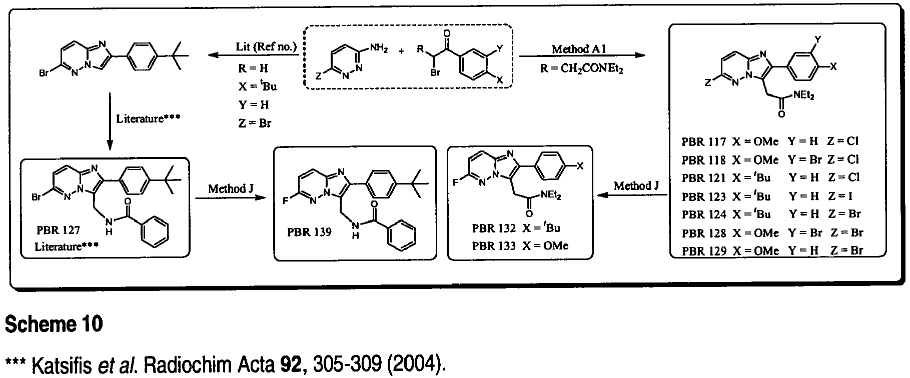

Compounds in accordance with the present invention include the following: 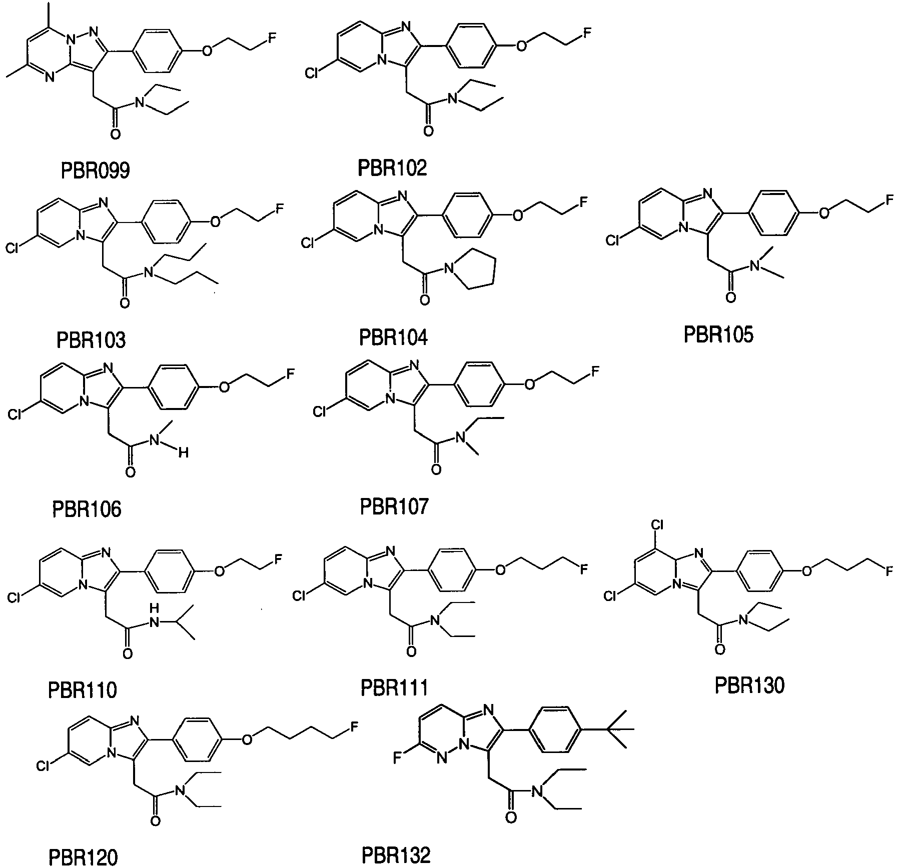 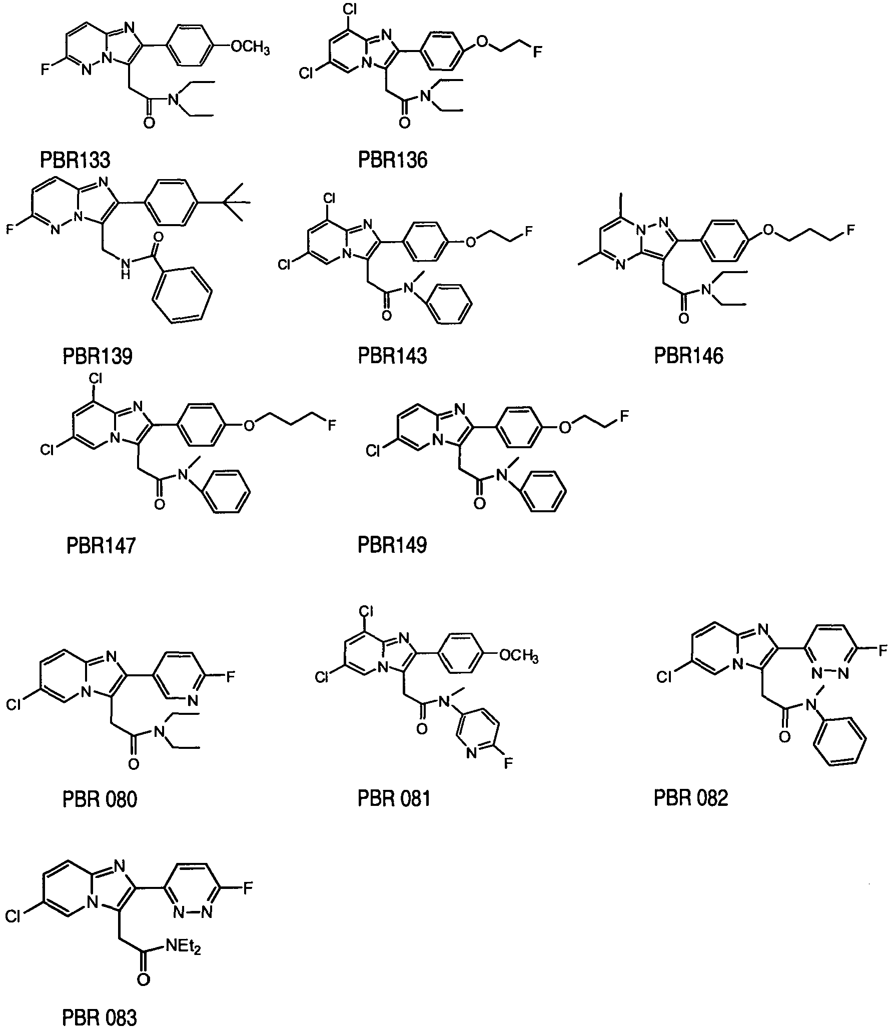 Intermediate compounds of the present invention also include the following: PBRs 115, 138, 145, 155, 142, 153, 154, 156, 131, 134, 135, 141, 125, 126, 137, 98, 139, 132, 133, 117, 118, 121, 123, 124, 128,129,116, 119, 140, 144, 101, 113, and 114 (see Schemes 7 to 10)

### Radiolabelling of compounds

Diagram 1 illustrates the general structure of the compounds of the invention, where A and C are unsaturated ring systems, B indicates the different configuration of the amide groups, and R₁ and R₂ designate various substituents.

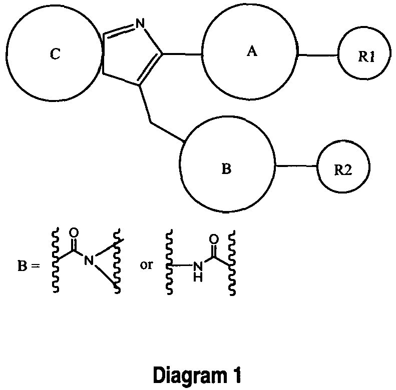

Radiolabelling is possible on rings B or C, or at positions R1 or R2 to form radiolabelled products. Three different paths to form ¹⁸F labelled products in the form of alkyl fluorides, fluoro pyridines and fluoro pyridazines are described below.

Alkyl fluorides can be introduced as substituents on rings A or C, or as part of the functional groups in R1 or R2, while fluoropyridines and fluoropyridazines can be introduced as part of rings A or C, or as a part of the group in R1 or R2.

Radiofluorination may be achieved by methods known to those skilled in the art. One such method involves the use of the [K2.2.2][K₂CO₃][¹⁸F⁻] complex, wherein the complex is reacted with a terminal alkyl group possessing a suitable leaving group, for example a halogen or O-tosylate or O-mesylate. This method is depicted in Schemes 11, 12 and 13 below.

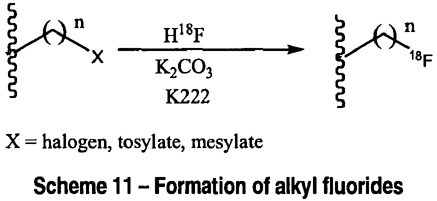

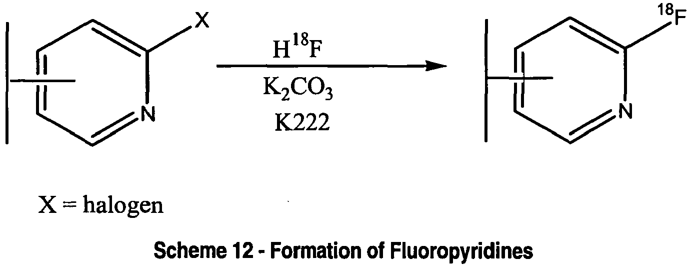

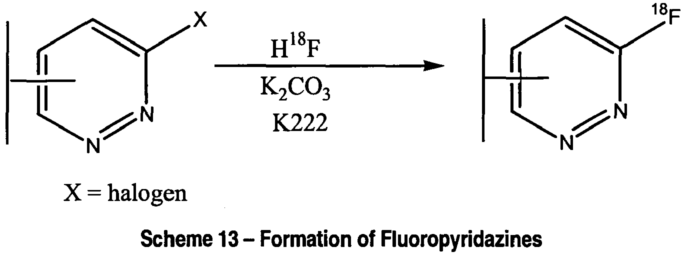

In a typical radiofluorination reaction, ¹⁸F-fluoride, in the form of H¹⁸F, is produced from an ¹⁸O(p,n)¹⁸F nuclear reaction by bombardment of an ¹⁸O-enriched water target. The aqueous ¹⁸F-fluoride solution is added to a 2.5 mL wheaton vial containing dry acetonitrile (1 mL), Kryptofix 2.2.2 and potassium carbonate. The solvent is evaporated under a stream of nitrogen at a temperature of about 80-120°C. This azeotropic drying is then repeated twice by further addition of acetonitrile (2 x 1 mL). The precursor compound is dissolved in an appropriate solvent, for example acetonitrile, DMF or DMSO (1 ml) and then added to the dried K222.K₂CO₃.KF complex. Stirring and heating is continued for about 5 to 15 mins before the reaction mixture is diluted with mobile phase and purified by reverse phase HPLC. The radioactive peak corresponding to the fluorinated product is collected, dried *in vacuo* and then formulated in saline (0.9%) for biological evaluation.

### Radiolabelled compounds

Radiolabelled compounds in accordance with the present invention include the following:

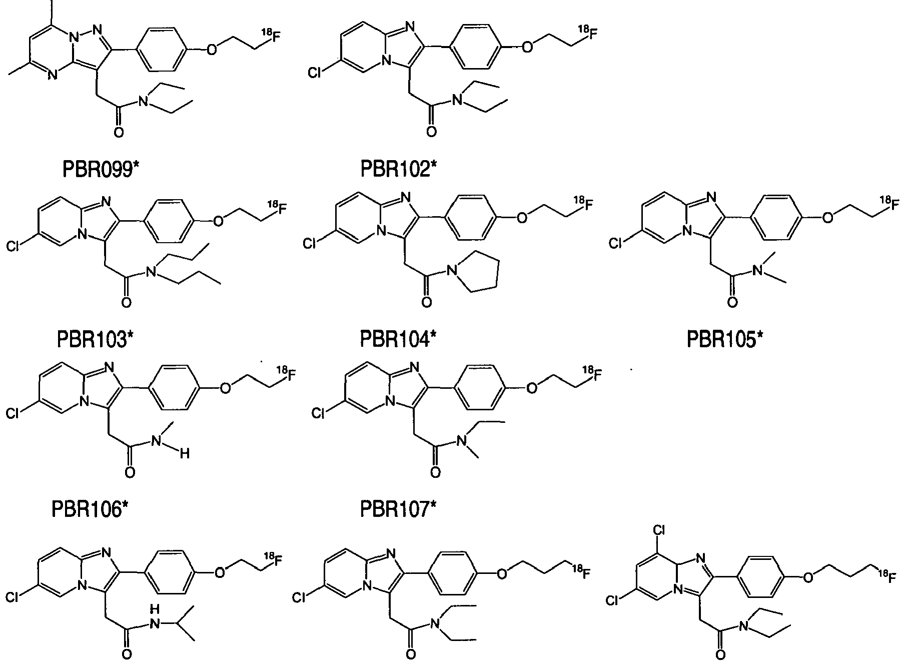

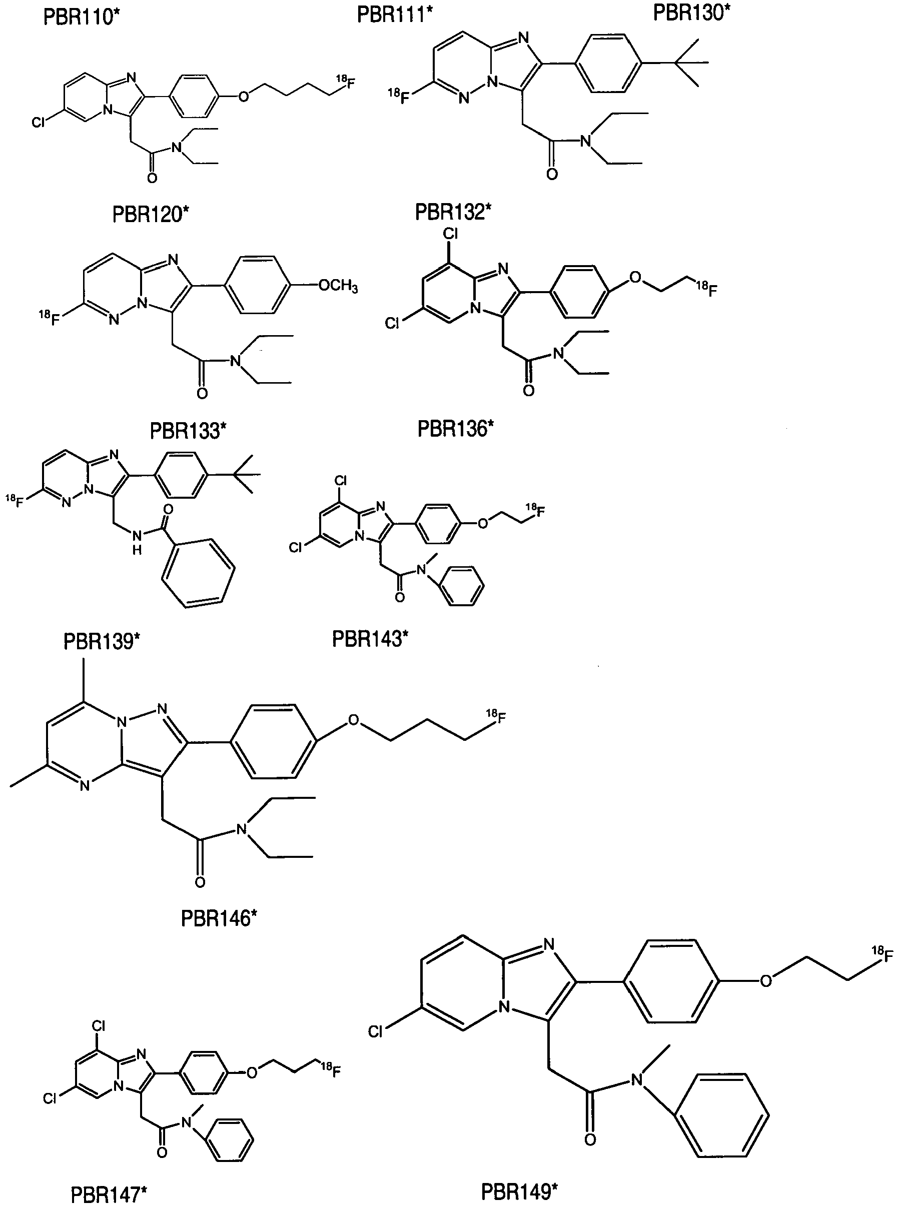

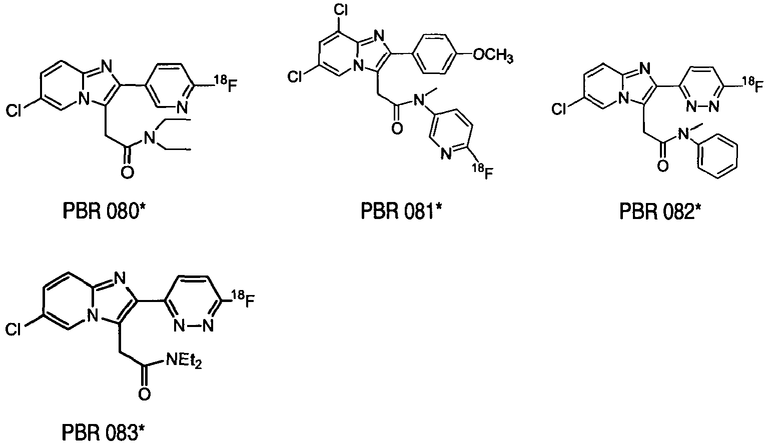

### Pharmaceutical compositions

Pharmaceutical compositions in accordance with the invention may be administered orally, topically, parenteral, or subcutaneous, e.g. by injection and by intra-arterial infusion, rectally or by inhalation spray.

For oral administration, the pharmaceutical composition may be in the form of tablets, lozenges, pills, troches, capsules, elixirs, powders, granules, suspensions, emulsions, syrups and tinctures. Slow-release or delayed-release forms may also be prepared, for example in the form of coated particles, multi-layer tablets or microgranules. Solid forms for oral administration may contain pharmaceutically acceptable binders, sweeteners, disintegrating agents, diluents, flavourings, coating agents, preservatives, lubricants and/or time delay agents. Suitable binders include gum acacia, gelatin, corn starch, gum tragacanth, sodium alginate, carboxymethylcellulose or polyethylene glycol. Suitable sweeteners include sucrose, lactose, glucose, aspartame or saccharine. Suitable disintegrating agents include corn starch, methylcellulose, polyvinylpyrrolidone, xanthan gum, bentonite, alginic acid or agar. Suitable diluents include lactose, sorbitol, mannitol, dextrose, kaolin, cellulose, calcium carbonate, calcium silicate or dicalcium phosphate. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

Liquid forms for oral administration may contain, in addition to the above agents, a liquid carrier. Suitable liquid carriers include water, oils such as olive oil, peanut oil, sesame oil, sunflower oil, safflower oil, arachis oil, coconut oil, liquid paraffin, ethylene glycol, propylene glycol, polyethylene glycol, ethanol, propanol, isopropanol, glycerol, fatty alcohols, triglycerides or mixtures thereof.

Suspensions for oral administration may further comprise dispersing agents and/or suspending agents. Suitable suspending agents include sodium carboxymethylcellulose, methylcellulose, hydroxypropylmethylcellulose, polyvinylpyrrolidone, sodium alginate or cetyl alcohol. Suitable dispersing agents include lecithin, polyoxyethylene esters of fatty acids such as stearic acid, polyoxyethylene sorbitol mono-or di-oleate,-stearate or -laurate, polyoxyethylene sorbitan mono-or di-oleate,-stearate or-laurate and the like. The emulsions for oral administration may further comprise one or more emulsifying agents. Suitable emulsifying agents include dispersing agents as exemplified above or natural gums such as gum acacia or gum tragacanth. For topical administration, the pharmaceutical composition may be in the form of a cream, ointment, gel, jelly, tincture, suspension or emulsion. The pharmaceutical composition may contain pharmaceutically acceptable binders, diluents, disintegrating agents, preservatives, lubricants, dispersing agents, suspending agents and/or emulsifying agents as exemplified above.

For parenteral administration, the compound of formula (I) or (Ia) or a salt thereof, may be prepared in sterile aqueous or oleaginous solution or suspension. Suitable non-toxic parenterally acceptable diluents or solvents include water, Ringer's solution, isotonic salt solution, 1,3-butanediol, ethanol, propylene glycol or polyethylene glycols in mixtures with water. Aqueous solutions or suspensions may further comprise one or more buffering agents. Suitable buffering agents include sodium acetate, sodium citrate, sodium borate or sodium tartrate, for example.

For rectal administration, the compound of formula (I) or (Ia) is suitably administered in the form of an enema or suppository. A suitable suppository may be prepared by mixing the active substance with a non-irritating excipient which is solid at ordinary temperatures but which will melt in the rectum. Suitable such materials are cocoa butter and polyethylene glycols. Suitable enemas may comprise agents as exemplified above with reference to forms for topical administration.

Suitably, an inhalation spray comprising a compound of formula (I) or (Ia) will be in the form of a solution, suspension or emulsion as exemplified above. The inhalation spray composition may further comprise an inhalable propellant of low toxicity. Suitable propellants include carbon dioxide or nitrous oxide.

Pharmaceutical compositions, diagnostic compositions and medicaments including compounds of the first, second or third aspects may be manufactured by methods which are generally known in the art. Compositions or medicaments may be prepared by grinding, crushing, blending, dispersing, dissolving, suspending, mixing, admixing, combining, emulsifying or homogenising a compound of the first or second aspect with one or more suitable carriers, adjuvants, diluents or excipients. Combinations of two or more of the foregoing steps may also be employed.

The dosage form of the compound of formula (I) or (Ia) will include from 0.01% to 99% by weight of the compound of formula (I) or (Ia). Usually, dosage forms according to the invention will comprise from 0.1% to about 10% by weight of the active substance.

In a diagnostic method in accordance with the sixth aspect of the invention, a dosage of typically from about 3 to about 10mCi of a compound of the third aspect is typically administered to a mammal, usually a human, in whom it is desired to diagnose the presence or absence of a disorder characterised by an abnormal density of peripheral benzodiazepine receptors. After a period of from 0.5 to 4 hours following administration of a compound of the second embodiment, or alternatively from 1 to 40 hours, an image of the distribution of radioactivity in the body, or part of the body, of the mammal may be obtained. It will be appreciated that the lower doses are appropriate for administration to a child, and the higher dosages are more appropriate for administration for diagnosis of a tumour where imaging is to be carried out 2 to 4 hours or more after administration of the substance. The presence of a concentration of radioactivity at a site where an abnormal density of peripheral benzodiazepine receptors occurs in a mammal suffering from the disorder indicates a positive diagnosis.

A method in accordance with the seventh aspect includes the administration to a mammal, typically a human, of a compound of the first or second aspects, or a pharmaceutical composition of the fourth aspect. Single or multiple administrations of the pharmaceutical compositions of the fourth aspect may be carried out. One skilled in the art would be able, by routine experimentation, to determine a therapeutically effective amount (which is non-toxic) of the compound and/or compositions of the invention and an administration pattern which would be suitable for treating the diseases to which the compounds and compositions are applicable.

Further, it will be apparent to one of ordinary skill in the art that the optimal course of treatment, such as the number of doses of therapeutically effective amounts of the compound or compositions of the invention given per day for a defined number of days, can be ascertained using conventional treatment determination tests. Generally, a therapeutically effective amount per 24 hours may be in the range of about 0.0001 mg to about 1000 mg per kg body weight; suitably, about 0.001 mg to about 750 mg per kg body weight; about 0.01 mg to about 500 mg per kg body weight; about 0.1 mg to about 500 mg per kg body weight; about 0.1 mg to about 250 mg per kg body weight; or about 1.0 mg to about 250 mg per kg body weight. More suitably, an effective dosage per 24 hours may be in the range of about 1.0 mg to about 200 mg per kg body weight; about 1.0 mg to about 100 mg per kg body weight; about 1.0 mg to about 50 mg per kg body weight; about 1.0 mg to about 25 mg per kg body weight; about 5.0 mg to about 50 mg per kg body weight; about 5.0 mg to about 20 mg per kg body weight; or about 5.0 mg to about 15 mg per kg body weight. The therapeutically effective amount per 24 hrs may be continued on a daily, or every second, third, fourth, fifth, sixth or seventh day, until treatment of the disorder in the subject is achieved.

A therapeutic dosage of a compound of the first or second aspects of the invention may be determined by the attending physician in any given circumstance, depending on factors such as the condition which is to be treated in the patient, and its severity.

Conditions for the treatment of which compounds in accordance with the invention are useful are conditions associated with abnormal density of peripheral benzodiazepine receptors, such as inflammation, neurodegenerative disorders, including Huntington's disease, Alzheimer's disease, multiple sclerosis, anxiety, stress, emotional disturbances, cognitive impairment, stroke, and cerebral ischemia; certain tumours, such as glioma, and carcinoma of the ovary, colon, breast, prostate, brain and adrenals; neurotoxic injury, including that associated with anoxia or ischemia which may result from stroke or cardiac arrest; cognitive disorders, in cognitive enhancement and in the modulation of apoptotic processes. A compound in accordance with the invention may be administered in a single dose, or in two doses, or in multiple doses, depending on the disorder, the stage of the disorder, its response to the treatment, and any undesirable effects which may become apparent.

### In vivo biodistribution studies

*In vivo* biodistribution of the radiolabelled compounds of the invention indicated high uptake in the tissues which have known PBR sites. Tables 1, 1A, 2, 2A, 3, 4, 5 and 6 show the biodistribution of the compounds of PBR111*, PBR102*, PBR132*, PBR146*, PBR147*, and PBR099* in which the fluorine atom is radiolabelled, when administered to rats. Pretreatment of the rats with known standards such as PK 11195 without radiolabel (1 mg/kg) significantly reduced the uptake of activity in the tissues of interest. Pretreatment with Flumazenil and other central acting benzodiazepines did not reduce the uptake of activity of the radiolabelled compounds of the invention, thereby indicating that the binding of the compounds of the invention *in vivo* is specific and selective for the peripheral benzodiazepine receptors.

**Table 1: Biodistribution of the PBR 111* in normal rodents (rats). The uptake of the tracer in the different organs is consistent with the normal known peripheral benzodiazepine receptor distribution^{†}.**

| Time\Organ | 15 min | 30 min | 1 hour | 4 hours |
|---|---|---|---|---|
| LIVER | 1.54±0.20 | 0.94±0.28 | 0.82±0.22 | 0.44±0.07 |
| SPLEEN | 8.25±2.25 | 6.54±1.35 | 6.72±1.91 | 3.45±0.43 |
| KIDNEY | 5.18±0.67 | 4.77±1.05 | 5.08±1.23 | 2.57±0.48 |
| MUSCLE | 0.68±0.16 | 0.62±0.15 | 0.55±0.11 | 0.19±0.05 |
| SKIN | 0.39±0.06 | 0.41±0.06 | 0.54±0.07 | 0.71±0.07 |
| BONE | 0.99±0.18 | 1.35±0.27 | 2.41±0.24 | 4.86±0.86 |
| LUNGS | 16.45±2.81 | 9.27±1.60 | 7.29±2.12 | 3.37±0.40 |
| HEART | 7.75±1.08 | 7.30±0.90 | 8.41±1.91 | 4.92±0.66 |
| BLOOD | 0.48±0.11 | 0.33±0.11 | 0.24±0.08 | 0.12±0.02 |
| BLADDER | 0.67±0.36 | 0.76±0.16 | 1.24±0.29 | 1.33±0.17 |
| STOMACH | 0.95±0.23 | 1.18±0.34 | 0.91±0.06 | 0.51±0.06 |
| GIT | 1.21±0.11 | 1.38±0.36 | 1.89±0.37 | 2.01±0.27 |
| BRAIN | 0.31±0.06 | 0.19±0.04 | 0.17±0.04 | 0.10±0.02 |
| OLF BULBS | 0.74±0.14 | 0.60±0.14 | 0.65±0.14 | 0.47±0.06 |
| THYROID | 11.40±0.33 | 13.24±4.62 | 13.04±3.22 | 8.91±1.19 |
| PANCREAS | 2.33±0.33 | 1.49±0.29 | 1.24±0.36 | 0.61±0.10 |
| THYMUS | 1.07±0.23 | 1.23±0.47 | 1.39±0.29 | 1.88±0.40 |
| ADRENALS | 8.36±2.02 | 9.06±3.47 | 12.11±3.32 | 17.81±3.61 |
| TESTES | 0.35±0.05 | 0.38±0.07 | 0.51±0.09 | 0.67±0.08 |

| | | | | |
|---|---|---|---|---|
| Results are mean ± SD percent injected dose per gram (% ID/g), n = 4 rats. † Results not corrected for the decay of ¹⁸F | | | | |

**Table 1 A: Biodistribution of PBR111* in rats. Results are mean ± SD percent injected dose per gram (% ID/g), n = 4 rats□.**

| | **15 min** | **30 min** | **60 min** | **4 hours** |
|---|---|---|---|---|
| Liver | 1.37±0.20 | 0.86±0.17 | 0.65±0.09 | 0.39±0.06 |
| Spleen | 7.25±1.57 | 5.98±0.63 | 5.27±0.85 | 3.12±0.37 |
| Kidney | 4.56±0.34 | 4.34±0.52 | 4.11±0.66 | 2.32±0.37 |
| Muscle | 0.61±0.16 | 0.57±0.10 | 0.44±0.04 | 0.17±0.05 |
| Skin | 0.34±0.03 | 0.37±0.02 | 0.46±0.02 | 0.63±0.04 |
| Bone | 0.87±0.12 | 1.22±0.10 | 2.04±0.09 | 4.32±0.33 |
| Lungs | 14.39±2.19 | 8.41±0.77 | 5.68±1.03 | 3.02±0.39 |
| Heart | 6.76±0.82 | 6.63±0.35 | 6.72±0.80 | 4.38±0.42 |
| Blood | 0.41±0.08 | 0.30±0.06 | 0.18±0.04 | 0.11±0.01 |
| Bladder | 0.60±0.28 | 0.68±0.08 | 1.01±0.28 | 1.20±0.23 |
| Stomach | 0.82±0.20 | 1.06±0.23 | 0.78±0.12 | 0.45±0.06 |
| GIT | 1.05±0.08 | 1.22±0.20 | 1.54±0.19 | 1.77±0.12 |
| Brain | 0.27±0.04 | 0.17±0.02 | 0.13±0.01 | 0.08±0.01 |
| Olfactory Bulbs | 0.64±0.10 | 0.54±0.07 | 0.54±0.10 | 0.41±0.04 |
| Thyroid | 9.82±0.74 | 10.33±0.99 | 10.72±2.14 | 8.25±1.10 |
| Pancreas | 1.99±0.18 | 1.28±0.12 | 0.92±0.10 | 0.53±0.09 |
| Thymus | 0.91±0.17 | 1.10±0.35 | 1.12±0.21 | 1.65±0.41 |
| Adrenals | 6.26±0.94 | 6.49±0.93 | 9.23±1.25 | 16.46±0.97 |
| Testes | 0.29±0.03 | 0.33±0.03 | 0.40±0.01 | 0.57±0.03 |

| | | | | |
|---|---|---|---|---|
| ^{†} Results corrected for the decay of ¹⁸F | | | | |

**Table 2: Biodistribution of PBR 102* in normal rats, expressed as a % of ID/g tissue over a 4 hour period^{‡}.**

| | 15 min | 30 min | 60 min | 4 hours |
|---|---|---|---|---|
| Liver | 0.75±0.21 | 0.63±0.21 | 0.40±0.04 | 0.34±0.05 |
| Spleen | 5.35±0.89 | 4.68±0.90 | 3.89±0.54 | 2.29±0.50 |
| Kidney | 3.90±0.53 | 3.95±1.01 | 3.10±0.51 | 1.61±0.29 |
| Muscle | 0.49±0.03 | 0.46±0.08 | 0.39±0.07 | 0.27±0.03 |
| Skin | 0.29±0.05 | 0.37±0.05 | 0.43±0.07 | 0.59±0.06 |
| Bone | 0.56±0.07 | 0.64±0.12 | 0.73±0.13 | 1.22±0.22 |
| Lungs | 17.25±1.70 | 14.80±3.67 | 13.44±2.71 | 10.26±1.09 |
| Heart | 5.23±0.33 | 5.41±0.71 | 5.32±0.74 | 3.38±0.44 |
| Blood | 0.25±0.06 | 0.21±0.03 | 0.20±0.03 | 0.26±0.03 |
| Bladder | 0.59±0.14 | 0.67±0.16 | 0.60±0.11 | 1.08±0.14 |
| Stomach | 0.80±0.19 | 0.71±0.21 | 0.52±0.13 | 0.49±0.13 |
| Git | 0.82±0.11 | 1.07±0.26 | 1.09±0.13 | 1.43±0.34 |
| Brain | 0.24±0.03 | 0.20±0.04 | 0.20±0.03 | 0.23±0.03 |
| Olf Bulbs | 0.68±0.12 | 0.44±0.05 | 0.52±0.05 | 0.50±0.13 |
| Thyroid | 7.74±1.90 | 10.70±2.08 | 11.55±4.24 | 8.29±3.22 |
| Pancreas | 1.74±0.22 | 1.42±0.45 | 0.88±0.18 | 0.49±0.12 |
| Thymus | 0.68±0.17 | 0.81±0.06 | 0.91±0.16 | 1.21±0.27 |
| Adrenals | 8.30±2.13 | 9.75±1.53 | 7.88±1.86 | 9.77±2.42 |
| Testes | 0.22±0.02 | 0.29±0.05 | 0.37±0.06 | 0.52±0.06 |

| | | | | |
|---|---|---|---|---|
| Results are mean ± SD percent injected dose per gram (% ID/g), n = 4 rats. ^{‡} Results not corrected for the decay of ¹⁸F. | | | | |

**Table 2A: Biodistribution of PBR102* in rats. Results are mean ± SD percent injected dose per gram (% ID/g), n = 4 rats^{#}**

| | **15 min** | **30 min** | **60 min** | **4 hours** |
|---|---|---|---|---|
| Liver | 0.63±0.15 | 0.52±0.14 | 0.35±0.01 | 0.29±0.02 |
| Spleen | 4.48±0.52 | 3.88±0.44 | 3.42±0.19 | 1.92±0.23 |
| Kidney | 3.26±0.27 | 3.25±0.50 | 2.72±0.23 | 1.35±0.11 |
| Muscle | 0.41±0.02 | 0.38±0.03 | 0.34±0.04 | 0.23±0.03 |
| Skin | 0.24±0.03 | 0.31±0.03 | 0.37±0.03 | 0.49±0.03 |
| Bone | 0.46±0.04 | 0.52±0.05 | 0.64±0.07 | 1.01±0.09 |
| Lungs | 14.30±1.45 | 12.10±1.98 | 11.63±1.52 | 8.09±1.14 |
| Heart | 4.35±0.33 | 4.44±0.26 | 4.62±0.27 | 2.82±0.15 |
| Blood | 0.20±0.04 | 0.17±0.02 | 0.18±0.01 | 0.21±0.01 |
| Bladder | 0.49±0.10 | 0.55±0.10 | 0.52±0.08 | 0.90±0.07 |
| Stomach | 0.66±0.15 | 0.57±0.12 | 0.45±0.08 | 0.41±0.12 |
| GIT | 0.67±0.06 | 0.86±0.13 | 0.94±0.05 | 1.17±0.17 |
| Brain | 0.20±0.01 | 0.16±0.02 | 0.17±0.01 | 0.19±0.01 |
| Olfactory bulbs | 0.55±0.07 | 0.35±0.01 | 0.44±0.02 | 0.40±0.07 |
| Thyroid | 6.28±1.36 | 8.77±2.27 | 8.24±0.99 | 5.75±0.89 |
| Pancreas | 1.41±0.09 | 1.13±0.26 | 0.75±0.11 | 0.40±0.06 |
| Thymus | 0.55±0.13 | 0.65±0.07 | 0.77±0.10 | 0.98±0.18 |
| Adrenals | 6.66±1.31 | 7.92±1.70 | 7.31±1.14 | 7.14±1.04 |
| Testes | 0.17±0.01 | 0.23±0.03 | 0.31±0.03 | 0.42±0.01 |

| | | | | |
|---|---|---|---|---|
| ^{#} Results corrected for the decay of ¹⁸F | | | | |

**Table 3: Biodistribution of PBR132* in SD rats. Results are mean ± SD percent injected dose per gram (% ID/g), n = 4 rats*.**

| | **15 min** | **30 min** | **60 min** | **4 hours** |
|---|---|---|---|---|
| Liver | 1.22±0.17 | 1.18±0.33 | 0.67±0.06 | 0.31±0.03 |
| Spleen | 5.85±0.93 | 6.89±0.80 | 5.56±0.67 | 2.64±0.36 |
| Kidney | 4.10±0.28 | 3.94±0.54 | 3.24±0.15 | 2.31±0.17 |
| Muscle | 0.89±0.28 | 0.91±0.10 | 0.50±0.03 | 0.24±0.06 |
| Skin | 0.60±0.09 | 0.65±0.08 | 0.64±0.07 | 0.67±0.17 |
| Bone | 1.04±0.14 | 1.25±0.18 | 2.06±0.10 | 3.28±0.65 |
| Lungs | 16.09±2.56 | 11.58±2.11 | 6.40±0.50 | 2.42±0.25 |
| Heart | 8.13±0.77 | 8.34±0.86 | 6.87±0.53 | 4.71±0.31 |
| Blood | 0.41±0.05 | 0.32±0.05 | 0.17±0.00 | 0.07±0.00 |
| Bladder | 0.73±0.33 | 0.87±0.36 | 0.84±0.08 | 0.99±0.30 |
| Stomach | 0.58±0.19 | 0.64±0.11 | 0.47±0.05 | 0.72±0.24 |
| GIT | 1.08±0.08 | 1.36±0.22 | 1.78±0.04 | 2.93±0.39 |
| Brain | 0.35±0.03 | 0.23±0.02 | 0.12±0.00 | 0.07±0.00 |
| Olfactory Bulbs | 0.63±0.09 | 0.46±0.11 | 0.36±0.05 | 0.25±0.03 |
| Thyroid | 8.87±1.06 | 9.15±0.87 | 18.30±1.00 | 7.41±0.68 |
| Pancreas | 2.01±0.26 | 1.97±0.32 | 1.20±0.19 | 0.57±0.10 |
| Thymus | 0.78±0.20 | 0.80±0.06 | 0.88±0.07 | 1.02±0.06 |
| Adrenals | 10.03±1.11 | 13.21±1.16 | 11.24±2.03 | 13.51±0.61 |
| Testes | 0.31±0.03 | 0.31±0.03 | 0.32±0.01 | 0.42±0.06 |

| | | | | |
|---|---|---|---|---|
| * Results corrected for the decay of ¹⁸F | | | | |

**Table 4: Biodistribution of PBR146* in rat. Results are mean ± SD percent injected dose per gram (% ID/g), n = 4 rats⁺.**

| | | | | |
|---|---|---|---|---|
| 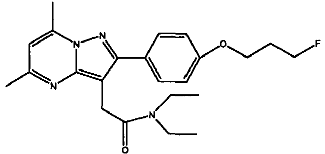 | | | | |

| | **15 min** | **30 min** | **60 min** | **4 hours** |
|---|---|---|---|---|
| Liver | 1.08±0.05 | 0.98±0.21 | 0.69±0.26 | 0.38±0.09 |
| Spleen | 5.49±0.90 | 5.53±0.36 | 5.14±0.38 | 3.61±0.19 |
| Kidney | 5.53±1.20 | 4.91±0.21 | 4.70±0.24 | 3.23±0.26 |
| Muscle | 1.10±0.15 | 0.82±0.13 | 0.67±0.04 | 0.31±0.06 |
| Skin | 0.53±0.11 | 0.55±0.05 | 0.67±0.06 | 0.71±0.07 |
| Bone | 1.06±0.17 | 1.46±0.27 | 2.07±0.27 | 4.12±0.23 |
| Lungs | 20.42±4.32 | 12.36±1.28 | 7.12±0.95 | 3.25±0.21 |
| Heart | 8.99±0.69 | 9.05±0.51 | 8.57±1.01 | 5.76±0.92 |
| Blood | 0.38±0.08 | 0.26±0.03 | 0.18±0.02 | 0.10±0.01 |
| Bladder | 0.63±0.14 | 0.71±0.22 | 0.89±0.25 | 1.26±0.16 |
| Stomach | 0.69±0.12 | 1.03±0.16 | 1.27±0.45 | 0.34±0.04 |
| GIT | 1.05±0.13 | 1.25±0.09 | 1.51±0.21 | 1.54±0.11 |
| Brain | 0.27±0.03 | 0.15±0.02 | 0.12±0.01 | 0.08±0.01 |
| Olfactory Bulbs | 0.41±0.10 | 0.45±0.07 | 0.25±0.06 | 0.31±0.08 |
| Thyroid | 7.71±2.92 | 7.69±2.21 | 13.37±0.46 | 10.68±1.44 |
| Pancreas | 2.20±0.68 | 1.59±0.10 | 1.41±0.22 | 0.77±0.16 |
| Thymus | 0.80±0.07 | 0.84±0.11 | 0.96±0.17 | 1.35±0.15 |
| Adrenals | 8.05±0.66 | 7.94±1.52 | 11.02±1.88 | 12.21±1.56 |
| Testes | 0.269±0.022 | 0.285±0.024 | 0.319±0.028 | 0.426±0.043 |

| | | | | |
|---|---|---|---|---|
| ⁺ Results corrected for the decay of ¹⁸F | | | | |

**Table 5: Biodistribution of PBR147* in SD rats. Results are mean ± SD percent injected dose per gram (% ID/g), n = 4 rats^{o}.**

| | **15 min** | **30 min** | **60 min** | **4 hours** |
|---|---|---|---|---|
| Liver | 1.88±0.16 | 1.37±0.38 | 1.08±0.14 | 0.57±0.02 |
| Spleen | 8.07±1.11 | 7.25±1.13 | 6.98±0.29 | 4.79±0.31 |
| Kidney | 4.75±0.75 | 3.82±0.45 | 3.68±0.28 | 3.26±0.16 |
| Muscle | 0.78±0.09 | 0.64±0.19 | 0.55±0.11 | 0.44±0.04 |
| Skin | 0.45±0.03 | 0.51±0.06 | 0.49±0.06 | 0.57±0.04 |
| Bone | 0.94±0.05 | 1.18±0.08 | 1.49±0.16 | 2.56±0.03 |
| Lungs | 26.34±3.04 | 18.00±2.29 | 11.36±0.97 | 5.62±0.24 |
| Heart | 6.53±0.38 | 5.69±0.42 | 5.82±0.24 | 6.09±0.54 |
| Blood | 0.63±0.08 | 0.34±0.06 | 0.23±0.03 | 0.13±0.01 |
| Bladder | 0.61±0.15 | 0.58±0.05 | 0.58±0.12 | 0.80±0.05 |
| Stomach | 0.41±0.12 | 0.40±0.06 | 0.64±0.27 | 0.83±0.17 |
| GIT | 0.98±0.15 | 1.14±0.04 | 1.44±0.29 | 2.71±0.10 |
| Brain | 0.43±0.05 | 0.26±0.04 | 0.16±0.01 | 0.09±0.01 |
| Olfactory Bulbs | 0.58±0.09 | 0.48±0.09 | 0.27±0.03 | 0.24±0.09 |
| Thyroid | 7.23±1.35 | 7.43±1.00 | 8.17±1.71 | 6.44±0.45 |
| Pancreas | 2.12±0.16 | 1.88±0.24 | 1.66±0.13 | 0.95±0.14 |
| Thymus | 0.76±0.12 | 0.63±0.11 | 0.69±0.08 | 0.81±0.08 |
| Adrenals | 7.59±1.30 | 8.47±1.46 | 8.45±0.84 | 9.30±0.55 |
| Testes | 0.23±0.02 | 0.21±0.02 | 0.22±0.01 | 0.28±0.02 |

| | | | | |
|---|---|---|---|---|
| ^{o} Results corrected for the decay of ¹⁸F | | | | |

**Table 6: Biodistribution of PBR099* in SD rats. Results are mean ± SD percent injected dose per gram (% ID/g).**

| | **15 min** | **30 min** | **60 min** | **4 hours** |
|---|---|---|---|---|
| Liver | 0.63 ± 0.06 | 0.54 ± 0.18 | 0.40 ± 0.05 | 0.29 ± 0.04 |
| Spleen | 4.60 ± 0.29 | 4.71 ± 0.61 | 4.51 ± 0.70 | 2.88 ± 0.56 |
| Kidney | 3.85 ± 0.24 | 3.62 ± 0.53 | 3.53 ± 0.24 | 2.41 ± 0.40 |
| Muscle | 0.65 ± 0.14 | 0.55 ± 0.03 | 0.43 ± 0.02 | 0.30 ± 0.05 |
| Skin | 0.35 ± 0.04 | 0.49 ± 0.02 | 0.48 ± 0.04 | 0.65 ± 0.05 |
| Bone | 0.64 ± 0.03 | 0.70 ± 0.02 | 0.79 ± 0.03 | 1.07 ± 0.05 |
| Lungs | 14.55 ± 1.35 | 8.82 ± 0.53 | 5.66 ± 0.56 | 2.80 ± 0.51 |
| Heart | 5.86 ± 0.44 | 5.59 ± 0.26 | 6.45 ± 0.43 | 4.29 ± 0.86 |
| Blood | 0.23 ± 0.03 | 0.16 ± 0.01 | 0.14 ± 0.01 | 0.16 ± 0.01 |
| Bladder | 0.46 ± 0.18 | 0.42 ± 0.08 | 0.72 ± 0.17 | 0.80 ± 0.11 |
| Stomach | 0.45 ± 0.18 | 0.32 ± 0.04 | 0.41 ± 0.09 | 0.79 ± 0.16 |
| GIT | 0.83 ± 0.09 | 0.90 ± 0.03 | 1.31 ± 0.08 | 1.90 ± 0.09 |
| Brain | 0.21 ± 0.01 | 0.14 ± 0.01 | 0.13 ± 0.02 | 0.15 ± 0.01 |
| Olfactory Bulbs | 0.35 ± 0.03 | 0.28 ± 0.03 | 0.29 ± 0.03 | 0.31 ± 0.05 |
| Thyroid | 6.94 ± 1.04 | 7.99 ± 1.83 | 9.90 ± 3.67 | 7.97 ± 1.37 |
| Pancreas | 1.70 ± 0.13 | 1.33 ± 0.16 | 1.08 ± 0.19 | 0.56 ± 0.09 |
| Thymus | 0.62 ± 0.17 | 0.66 ± 0.14 | 0.77 ± 0.12 | 0.91 ± 0.05 |
| Adrenals | 5.64 ± 0.53 | 6.56 ± 1.20 | 7.82 ± 1.73 | 9.69 ± 1.49 |
| Testes | 0.184 ± 0.006 | 0.209 ± 0.007 | 0.266 ± 0.019 | 0.349 ± 0.011 |

### In vitro binding studies

The inhibition constant (IC₅₀) of selected compounds for the peripheral benzodiazepine binding site was determined by incubating, in triplicate, aliquots (0.1 ml) of diluted kidney membrane preparation (200 µg), at 4°C for 1 h with 7 concentrations of the compound (10⁻⁵ to 10⁻¹⁰ M) and [³H]-PK11195 (2 nM) in a final volume of 0.5 ml. Non-specific binding was determined with PK11195 (10 µM).

Similarly, the IC₅₀ of the compounds for central benzodiazepine receptors was determined by incubating aliquots (0.1 ml), in triplicate, of diluted brain cortex membrane preparation (200 µg) at 25°C for 45 min with 7 concentrations of the compounds (10⁻⁵ to 10⁻¹⁰ M) and of [³H]-Flumazenil (2 nM) in a final volume of 0.5 mL. Non-specific binding was determined with Flumazenil (20 µM). In both cases, incubations were terminated by rapid filtration through Whatman GF/B glass fibre. Filters were immediately washed 3 times with 5 mL ice-cold 50 mM Tris/HCl at pH 7.4. Filters were counted in a ß-scintillation counter (Packard) to measure the amount of bound radioactivity. The IC₅₀ of the compounds for the peripheral benzodiazepine binding site and the central benzodiazepine receptor were calculated using Kell 6 software. These results are shown in Table 7.

**Table 7: Binding Affinity for PBR and CBR of all compounds**

| Compound | PBR IC₅₀ (nM) | CBR IC₅₀ (nM) | LogP HPLC |
|---|---|---|---|
| PBR099 | 14.95 | >5000 | 2.54±0.08 |
| PBR102 | 13.2 | 1560 | 2.70±0.09 |
| PBR103 | 51.3 | 1843.6 | 3.44±0.11 |
| PBR104 | 575.2 | 1635.5 | 2.45±0.08 |
| PBR105 | 674.9 | 1759.9 | 2.04±0.06 |
| PBR106 | 169.6 | 547.7 | 2.05±0.07 |
| PBR107 | 54.8 | 1393.6 | 2.34±0.07 |
| PBR110 | 14.1 | 2735.7 | 2.74±0.09 |
| PBR111 | 7.5 | 1588.1 | 3.20±0.10 |
| PBR 120 | 35 | 941.6 | 3.53±0.11 |
| PBR 130 | 20 | 50% @ 10⁻⁵M >5000 | 3.43±0.10 |
| PBR 132 | 38 | 343.2 | 3.60±0.11 |
| PBR 133 | 32 | 18.8 | 2.35±0.07 |
| PBR 136 | 17.6 | 588.1 | 2.99±0.09 |
| PBR 139 | 800 | 2616.7 | 4.23±0.13 |
| PBR 143 | 6.4 | 50% @ 10⁻⁵M | 3.85±0.12 |
| PBR 146 | 10.5 | 8000 50% @10⁻⁵M | 3.16±0.10 |
| PBR 147 | 7.4 | 4087.3 | 4.31±0.13 |
| PBR 149 | 4.7 | 357.0 | 3.57±0.11 |

### Examples

The invention will now be described in more detail, by way of illustration only, with respect to the following examples. The examples are intended to serve to illustrate this invention and should not be construed as limiting the generality of the disclosure of the description throughout this specification.

### Comparative example

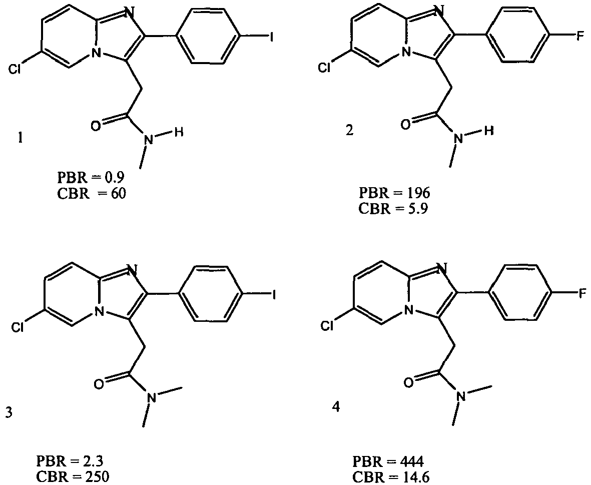

Compounds 1 to 4 were prepared in order to assess the effect on selectivity of replacing the 2-iodo substituent with a 2-fluoro substituent. As can be seen from the IC₅₀ values, where a 2-fluoro substituent is substituted for the 2-iodo substituent, the selectivity for the peripheral benzodiazepine receptor is lost. The above results surprisingly show that the simple replacement of the iodo substituent of the compounds disclosed in WO 99/51594 with a fluoro substituent results in compounds that are selective for the central benzodiazepine receptor as opposed to the peripheral benzodiazepine receptor. Unexpectedly, removing the iodo substituent from the phenyl ring and attaching a fluoro substituted alkoxy group results in fluorinated compounds that retain selectivity for the peripheral benzodiazepine receptor such that they are useful for PET imaging and also for therapeutic applications of disorders characterised by an abnormal density of peripheral benzodiazepine receptors.

### Example 1 - Preparation of precursor bromoacetophenones

A solution of bromine (2.52 g, 15.75 mmoles) in carbon tetrachloride (10 ml) was added over 1 h to a stirred, ice-cooled solution of *N,N*-diethyl-4-(4-methoxyphenyl)-4-oxobutanamide (3.945 g, 15.0 mmoles) in carbon tetrachloride (50 ml) containing 1 drop of conc. HBr. Stirring was continued at this temperature for a further 2 h, and then at room temperature for 30 min. The pale yellow solution was evaporated and the residue stirred with ethyl acetate (100 ml) and water (50 ml). Solid sodium bicarbonate was added cautiously to neutralize hydrogen bromide, the organic phase separated, washed (brine, 3 x 10 ml), dried (Na₂SO₄), and evaporated to give the crude bromoketone, 3-bromo-*N,N*-diethyl-4-(4-methoxyphenyl)-4-oxobutanamide as a colourless oil (5.15 g).

### Example 2 - Preparation of pyridine-based bromoacetophenones

In one reaction vessel, 2-chloro-5-acetylpyridine (1.55 g, 10 mmol) was dissolved in 45% HBr in acetic acid (30 ml) and left stirring in an ice-water bath for 20 min to form a dark brown solution. In a separate reaction vessel, bromine (1.61 g, 10 mmol) was added to HBr in acetic acid (45%) (6 ml) and the resulting solution was added to the first reaction vessel. The combined reaction mixture was left stirring in an ice-water bath for 1 h. After this time, anhydrous diethyl ether was added to the reaction solution, which resulted in precipitation of the reaction. The diethyl ether was added until no further precipitate was formed (- 60 mls). The solid was collected by filtration and washed with cold diethyl ether (dried over sodium) to yield a light yellow powder of 2-bromo-1-(6-chloropyridin-3-yl)ethanone as a HBr salt (2.44 g, 77%).

### Example 3 - METHOD A1 - Condensation 1

A mixture of 2-bromo-4'-methoxyacetophenone (1.4 g, 6.13 mmol), 2-amino-3,5-dichloropyridine (1.0 g, 6.13 mmol) in absolute ethanol (15 mls) was heated to reflux. After 2 h, NaHCO₃ (309 mg, 3.68 mmol) was slowly added to the dark brown solution. The reaction mixture continued to reflux for another 15 h before another portion of NaHCO₃ was slowly added (206 mg, 2.45 mmol). After 15 mins, the reaction was cooled and kept in the fridge for 20 h. The resulting precipitate was collected by filtration, washed with cold ethanol followed by water. The solid was then boiled in hot ethanol for 10 mins before it was cooled to RT and then collected by filtration to give 6,8-dichloro-2-(4-methoxyphenyl)imidazo[1,2-a]pyridine as beige coloured crystals (1.34 g, 75%).

### Example 4 - METHOD A2 - Condensation 2

A solution of 3-bromo-*N,N-*diethyl-4-(4-methoxyphenyl)-4-oxobutanamide (60%), 3-bromo-4-(3-bromo-4-methoxyphenyl)-*N,N*-diethyl-4-oxobutanamide (20%) and *N,N*-diethyl-4-(4-methoxyphenyl)-4-oxobutanamide (20%) (total weight 4 g) in dry DMF (10 ml) was added portionwise over 5 h to a stirred solution of 3,5-dichloro-2-aminopyridine (1.63 g, 10.0 mmoles) in DMF at 110°C; heating was continued at this temperature for 6 h, and then at 100°C for 15 h. The cooled mixture was diluted with ethyl acetate (75 ml), washed (water, 3 x 20 ml; 10% aqueous tartaric acid, 20 ml; 2% aqueous NaHCO₃, 10 ml; water, 2 x 10 ml; brine, 2x10 ml), dried (Na₂SO₄) and run through a short silica column (ca, 4 g) with elution being continued with ethyl acetate (30 ml). The solvent was evaporated to give a red-brown tar, which was purified by preparative HPLC on an Alltech Alltima C18, 10µ, 250 x 22mm column eluting with 80% acetonitrile and 20% ammonium bicarbonate (20mM) at 10mLs /min The major product, eluting at 11.0 mins, was recrystallized from ethyl acetate / hexane to give white needles of 2-(6,8-dichloro-2-(4-methoxyphenyl)imidazo[1,2-*a*]pyridin-3-yl)-*N,N*-diethylacetamide (465 mg, 9.54% overall). The minor product, eluting at 13.8 mins gave white woolly needles of 2-(2-(3-bromo-4-methoxyphenyl)-6,8-dichloroimidazo[1,2-*a*]pyridin-3-yl)-*N,N*-diethylacetamide (209 mg, 3.59%) from the same solvents.

### Example 5 - METHOD B - UGI reaction

A mixture of 2-amino-5-chloropyridine (200 mg, 1.56 mmol), 4-bromobenzaldehyde (346 mg, 1.87 mmol) and scandium (III) triflate (38mg, 0.078 mmol) was dissolved in methanol dichloromethane (1:3, 3 mls). After 30 mins of stirring at RT, ethyl isocyanoacetate (212 mg, 1.87 mmol) was added and the reaction continued to stir at RT for 72 hrs. After this time the reaction mixture was diluted with DCM (10 mls) and then washed with water. The water phase was extracted with DCM (2x 10 ml) and the combined organic extracts was then vigorously stirred with a solution of sodium bisulfite (2M, 25mls) for 10 mins before the organic extract was separated, dried (Na₂SO₄) filtered and the filtrate concentrated *in vacuo* to give a yellow oil. The yellow oil was crystallised from hot ethyl acetate with addition of hexane. The yellow crystals were collected by filtration to give ethyl 2-(2-(4-bromophenyl)-6-chloroimidazo[1,2-a]pyridin-3-ylamino)acetate (411 mg, 65%).

### Example 6 - METHOD C1

**Step 1.** To a solution of 2-(3-bromophenyl)-6-chloroimidazo[1,2-a]pyridine (1.93 g, 6.3 mmol) in acetic acid (25 ml) was slowly added aqueous dimethylamine (40%, 11.8 ml, 1.2 equv.) with stirring. The temperature rose and a precipitate formed. Aqueous formaldehyde (37%, 4.7 ml) was then added, and the mixture stirred at 55-60°C for 20h. After cooling down, the solvent was concentrated and the brown coloured residue was dissolved in a mixture of water (10 ml) and chloroform (20ml) and then 2.5 M aqueous NaOH was slowly added, with stirring, until the pH was 12. The chloroform layer was separated and the aqueous phase was further extracted with chloroform (10 ml + 2× 5 ml). The combined chloroform solutions was then extracted with 2N HCl (3 ×10 ml). The extract was then basified with NaOH to pH 12. A yellow gummy solid formed. The solid was redissoved in EtOH and recrystallised from ethanol / water to give (2-(3-bromophenyl)-6-chloroimidazo[1,2-*a*]pyridin-3-yl)-*N,N-*dimethylmethanamine as a beige crystalline solid (1.36 g, 60%).
**Step** 2. The product from Step 1 [(2-(3-bromophenyl)-6-chloroimidazo[1,2-*a*]pyridin-3-yl)-*N,N-*dimethylmethanamine (1.3 g, 3.6 mmol)] was dissolved in benzene (15 ml) and iodomethane (0.8 ml (1.8 g), 13 mmol) was added. The reaction was left stirring in the absence of light for 18h. A white solid precipitated out, and collected by filtration and washed with benzene to give a trimethyl ammonium iodide salt (0.8g).
**Step 3.** The trimethyl ammonium iodide salt from obtained from Step 2 (0.51 g, 1.1 mmol) was dissolved in EtOH/H₂O (1/1, 10 ml) and KCN (0.50 g, 7.8 mmol) was added to form a suspension. The mixture was heated to reflux for 24 h. Upon cooling, the suspension was blown with N₂ for 1h, and then evaporated to remove EtOH. Water (10 ml) was added and resulting brown solid was filtered and collected. The solid was dried in a desiccator to give the acetamide 2-(2-(3-bromophenyl)-6-chloroimidazo[1,2-a]pyridin-3-yl)acetamide as a light yellow solid (0.4 g).
**Step 4.** The acetamide from step 3 (0.4 g) was added to a solution of concentrated HCl (6 ml) and acetic acid (6 ml). The suspension was heated to reflux over night and an off-white precipitate formed upon cooling. The mixture was then evaporated to dryness and water (10 ml) and 10% NaOH (5 ml) was added. The resulting solution was heated for 40 min at 80°C and then filtered. The brown coloured filtrate was cooled and acidified with concentrated HCl to pH 4 to precipitate the hydrolysed product 2-(2-(3-bromophenyl)-6-chloroimidazo[1,2-a]pyridin-3-yl)acetic acid as a creamy solid (0. 24 g, 60%).

### Example 7 -METHOD C2

Steps 1, 2 and 3 are the same as described above in METHOD C1, however the hydrolysis in Step 4 was modified. Instead of using a solution of HCl / Acetic acid, a solution of HBr (48%, 200 ml) and acetic acid (100 ml) was added to the amide, for example 2-(6-chloro-2-(4-methoxyphenyl)imidazo[1,2-a]pyridin-3-yl)acetamide (9.96g, 0.031 mol). The reaction mixture was heated to reflux for 48h where upon a white precipitate formed after cooling. The resulting crystalline solid was collected by filtration and washed with water to give the acid 2-(6-chloro-2-(4-methoxyphenyl)imidazo[1,2-*a*]pyridin-3-yl)acetic acid as a hydrobromide salt (10.8g, HBr salt, 89%).

### Example 8 - METHOD C3 - Reverse amide reaction

Concentrated sulfuric acid (18 µl) was added dropwise to a vigorously stirred mixture of (6-chloro-2-(6-chloropyridin-3-yl)imidazo[1,2-*a*]pyridine) (40 mg, 0.15 mmole), *N*-hydroxybenzamide (22.7 mg, 0.15 mmole) and acetic acid (1.0 ml). The cloudy solution was then heated in an oil bath at 110°C for 12 h, cooled, diluted with ethyl acetate (15 ml) and washed with excess sodium carbonate solution (2.5%). The organic layer was washed with water (2 x 3 ml) and saturated brine (2 ml), dried (Na₂SO₄) and evaporated to give an oily residue which was separated by preparative HPLC on an Alltech Alltima Column (250x22mm, 10µ), eluting at 10mls/min with 50% acetonitrile and 50% ammonium bicarbonate (20 mM). The product that eluted at 14.8 mins gave *N*-((6-chloro-2-(6-hydroxypyridin-3-yl)imidazo[1,2-*a*]pyridin-3-yl)methyl)benzamide as fine white needles (8.2 mg, 14.3% yield) after crystallisation from chloroform/ethanol. The product that eluted at 16.9 mins gave *N*-((6-chloro-2-(6-chloropyridin-3-yl)imidazo[1,2-*a*]pyridin-3-yl)methyl) benzamide (10.2 mg, 17.0%) as white needles after crystallisation from ethyl acetate / hexane.

### Example 9 - METHOD D1 - Coupling (condensation) using EDCI

Diisopropylethylamine (620 µl, 3.6 mmoles) was added to a stirred mixture of (2-(6,8-dichloro-2-(4-methoxyphenyl)imidazo[1,2-*a*]pyridin-3-yl)acetic acid) (702 mg, 2.0 mmoles), *N*-methylaniline (260 ul, 2.4 mmoles) and HOBT (270 mg, 2.0 mmoles) in dry DMF (6 ml). After all solids were dissolved, EDCI (500 mg, 2.6 mmoles) was then added. Four days later, acetic acid (350 ul, 6 mmoles) and water (ca. 15 ml) were added slowly with stirring. The mixture was cooled to 5 °C for 3 h before the resulting precipitate was collected by filtration and recrystallised from chloroform / ethanol to give 2-(6,8-dichloro-2-(4-methoxyphenyl)imidazo[1,2-*a*]pyridin-3-yl)-*N*-methyl-*N*-phenylacetamide (842 mg, 95.6% yield) as white woolly needles.

### Exception

Diisopropylethylamine (2.09 ml, 12.0 mmoles) was added to a stirred mixture of (2-(6-chloro-2-(4-hydroxyphenyl)imidazo[1,2-*a*]pyridin-3-yl)acetic acid) (hydrobromide, 1.1505 g, 3.0 mmoles), diethylamine (372 ul, 3.6 mmoles) and HOBT (405 mg, 3.0 mmoles) in dry DMF (8 ml). After all solids had dissolved, EDCI (748 mg, 3.9 mmoles) was added. After 18 h, acetic acid (500 ul) and water (50 ml) were added slowly with stirring. The solid product collected, after cooling the mixture to 5°C for 6 h, was washed well with water and dried. This crude product (901 mg), which contained dimers and trimers formed from condensation of carboxylic acid and phenol moieties, was stirred with dry DMF (3 ml) and diethylamine (0.5 ml) at ca. 100 °C in a sealed container for 2 h. It was heated further, with the lid removed, until all solids dissolved and most of the excess diethylamine evaporated. Ethyl acetate (10 ml) was added rapidly, and the product was allowed to crystallise. The crystals were collected after 4 h at 5 °C, washed with a small volume of cold ethyl acetate and dried to give 2-(6-chloro-2-(4-hydroxyphenyl)imidazo[1,2-*a*]pyridin-3-yl)-*N,N*-diethylacetamide (816 mg, 76.1% yield) as white woolly needles.

### Example 10 - METHOD D2 - Coupling (condensation) using CDI

A mixture of 2-(6,8-dichloro-2-(4-methoxyphenyl)imidazo[1,2-*a*]pyridin-3-yl)acetic acid (176 mg, 0.50 mmole), carbonyl diimidazole (122 mg, 0.75 mmole) and dry DMF (1.5 ml) was stirred at room temperature for 6 h. After this time, 3-Amino-2-chloropyridine (71 mg, 0.55 mmole) was added and the reaction mixture heated at 85°C (oil bath temperature) for 16 h. Water (ca. 10 ml) was added slowly to the cooled mixture with vigorous stirring to precipitate the product as a purple-red solid, which was collected 2 h later, washed with water and recrystallized from ethanol to give 2-(6,8-dichloro-2-(4-methoxyphenyl)imidazo[1,2-*a*]pyridin-3-yl)-*N*-(6-chloropyridazin-3-yl)-*N-*methylacetamide (145.4 mg, 63.0%) as pale pink woolly needles.

### Example 11 - METHOD E1- Hydrolysis 1

A solution of sodium hydroxide (0.40 g, 10 mmoles) in water (10 ml) was added to a stirred mixture of methyl 2-(2-(4-(2-fluoroethoxy)phenyl)-6-chloroimidazo[1,2-*a*]pyridin-3-yl)acetate (1.45 g, 4.0 mmoles) in methanol (40 ml). The mixture was boiled under reflux for 1 h. Most of the methanol was distilled off (at atmospheric pressure) to a final volume of ca. 20 ml. The solution was cooled slightly, acetic acid (1 ml) added rapidly, and the mixture left undisturbed to crystallize the product. This was collected after being refrigerated overnight, washed with cold 1:1 methanol / water and dried to give 2-(2-(4-(2-fluoroethoxy)phenyl)-6-chloroimidazo[1,2-a]pyridin-3-yl)acetic acid (1.371 g, 98.4%) as white flakes.

### Example 12 - METHOD E2 - Hydrolysis 2

To a solution of 2-(2-(4-(4-acetoxybutoxy)phenyl)-6-chloroimidazo[1,2-*a*]pyridin-3-yl)-*N,N-*diethylacetamide (180 mg, 0.38 mmol) in MeOH (3 ml) was slowly added a solution of cesium carbonate (276 mg, 0.76 mmol) in H₂O (1 ml). After 2 days of stirring at ambient temperature, a precipitate was observed and was collected by filtration. The solid was collected by filtration and washed with water. The solid was then recrystallised from hot ethyl acetate and hexanes to give 2-(2-(4-(4-hydroxybutoxy)phenyl)-6-chloroimidazo[1,2-*a*]pyridin-3-yl)-*N,N*-diethylacetamide as colourless crystals (146 mg, 90%).

### Example 13 - METHOD F - Esterification

To stirring methanol (50 ml) was added concentrated sulphuric acid (1.5 ml) followed by 2-(6-chloro-2-(4-hydroxyphenyl)imidazo[1,2-*a*]pyridin-3-yl)acetic acid (hydrobromide, 1.918 g, 5.0 mmoles). The mixture was heated at 50°C for 1.25 h, cooled to room temperature and trimethyl orthoformate (530 mg, 5.0 mmoles) was added. The following day, the mixture was poured slowly into a vigorously stirred mixture of ethyl acetate (400 ml), water (100 ml) and sodium bicarbonate (6.0 g). The separated organic layer was washed (water, 2 x 50 ml; 5% aqueous sodium carbonate, 10 ml; water, 2 x 50 ml; brine, 25 ml), dried (Na₂SO₄) and concentrated (Rotavapor) until crystallization was well advanced (ca. 20 ml remaining). Hexane (20 ml) was then added slowly. The resulting product was collected 2 h later, washed with ethyl acetate / hexane (1:1, 20 ml) and dried to give methyl 2-(6-chloro-2-(4-hydroxyphenyl)imidazo[1,2-*a*]pyridin-3-yl)acetate (1.4673 g, 92.7%) as white needles.

### Example 14 - METHOD G - Alkylation of a phenol

A mixture of 2-(6-chloro-2-(4-hydroxyphenyl)imidazo[1,2-*a*]pyridin-3-yl)-*N,N*-diethylacetamide (71.5 mg, 0.2 mmole), 2-bromoethyl methyl ether (55.6mg, 38 µl, 0.4 mmole), potassium carbonate (83 mg, 0.6 mmole), potassium iodide (5 mg), tetrabutylammonium iodide (1.4 mg) and dry DMF (1 ml) was stirred vigorously for 2 days. The mixture was diluted with ethyl acetate (15 ml), washed (water, 4 x 3ml; brine, 3 ml), dried (Na₂SO₄ and evaporated. The resulting residue was recrystallized from ethyl acetate / hexane to give 2-(2-(4-(2-methoxyethoxy)phenyl)-6-chloroimidazo[1,2-*a*]pyridin-3-yl)-*N,N*-diethylacetamide (76 mg, 91.5% yield) as pale yellow flakes.

### Example 15 - METHOD H - Fluorination of an alkyl alcohol

Diisopropylethylamine (210 ul, 1.2 mmoles), perfluoro-1-butanesulfonyl fluoride (69 µl, 0.40 mmole) and triethylamine trihydrofluoride (67 µl, 0.40 mmole) were added to a stirred mixture of 2-(2-(4-(3-hydroxypropoxy)phenyl)-5,7-dimethylpyrazolo[1,5-*a*]pyrimidin-3-yl)-*N,N*-diethylacetamide (82 mg, 0.20 mmole) and dry acetonitrile (0.80 ml). The mixture was warmed to 40°C for 30 min and then left overnight at room temperature. Volatiles were removed, and a solution of the residual oil in ethyl acetate (10 ml) was washed (water, 3 x 3ml; 10% aqueous tartaric acid, 3 ml; water, 2 x 3 ml; brine, 3 ml), dried (Na₂SO₄) and evaporated. The residue was purified by preparative HPLC on an Alltech Alltima C18 250x22mm column eluting with 70% Acetonitrile 30% water + 0.1% TFA at 10mls/min. The peak at 15.4 mins was collected, concentrated and recrystallization from ethyl acetate / hexane gave 2-(2-(4-(3-fluoropropoxy)phenyl)-5,7-dimethylpyrazolo[1,5-*a*]pyrimidin-3-yl)-*N,N*-diethylacetamide (48.6 mg, 59.0%) as chunky needles.

### Example 16 - METHOD I - Demethylation

A stirred solution of (*N,N*-diethyl-2-(2-(4-methoxyphenyl)-5,7-dimethylpyrazolo[1,5-a]pyrimidin-3-yl)acetamide) (732 mg, 2.0 mmoles) and tetrabutylammonium iodide (812 mg, 2.2 mmoles) in dry dichloromethane (10 ml) was cooled in an ethanol / liquid nitrogen bath (ca. -70°C) while being flushed with N₂. A solution of boron trichloride in hexane (1.0 M, 9.0 ml, 9.0 mmoles) was then added drop-wise *via* syringe over 5 min, with vigorous stirring. Five mins later, the ethanol / N₂ bath was replaced with ice, and stirring continued for 2.5 h. The reaction was quenched by careful addition of ice and water (ca. 30 ml total) and the mixture stirred until all solid had dispersed (ca. 1 h). The semi-solid mass was shaken vigorously with water (50 ml), chloroform (50 ml) and ethanol (5 ml); solid sodium bicarbonate was added in small portions to neutralize the acids from hydrolysis of boron compounds, which was complete when the mixture was no longer purple in the presence of excess bicarbonate. The organic layer was separated, and the aqueous phase extracted with more chloroform (2 x 20 ml). The combined organic solution was washed with dilute brine (50 ml), dried (Na₂SO₄) and evaporated to give a crystalline semi-solid, which was slurried with acetonitrile (4 ml), the crystals filtered off, washed with minimal cold acetonitrile and dried to give *N,N*-diethyl-2-(2-(4-hydroxyphenyl)-5,7-dimethylpyrazolo[1,5-*a*]pyrimidin-3-yl)acetamide (620 mg, 88.1% yield) as white needles.

### Example 17 - METHOD J - Fluorination of halopyridazines

A mixture of 2-(2-(4-*tert*-butylphenyl)-6-bromoimidazo[1,2-*b*]pyridazin-3-yl)-*N,N*-diethylacetamide (66.5 mg, 0.15 mmole), potassium fluoride (87 mg, 1.50 mmoles) dimethyl sulfoxide (0.60 ml) and toluene (1.5 ml) in a 4-ml container was stirred and heated slowly to 170°C (oil bath temperature) to evaporate the toluene and remove water. The container was flushed with N₂, sealed, and kept at 165-170°C for 7 h. The cooled mixture was diluted with ethyl acetate (3 ml), washed (water, 3 x 1 ml; brine, 1 ml), dried (Na₂SO₄) and evaporated. The semi-crystalline residue was purified by preparative HPLC on an Alltech Alltima C18 250x22mm column eluting with 80% Acetonitrile 20% water + 0.1% TFA at 20mls/min. The peak at 7.4 mins was collected, concentrated and recrystallization of the resulting solid from ethyl acetate / hexane gave 38.5 mg of 2-(2-(4-*tert-*butylphenyl)-6-fluoroimidazo[1,2-*b*]pyridazin-3-yl)-*N,N*-diethylacetamide (67.2%) as colourless plates.

### Example 18 - Radiosynthesis to form alkyl fluorides.

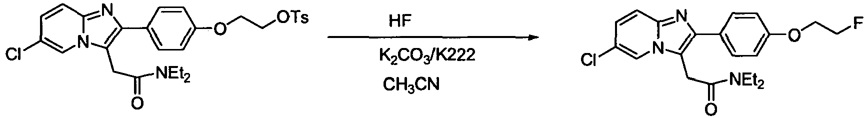

[¹⁸F]Fluoride, in the form of H¹⁸F, was produced by the ¹⁸O(p,n)¹⁸F nuclear reaction by bombardment of ¹⁸O-enriched water target. The aqueous [¹⁸F]fluoride solution was added to a 2.5-mL wheaton vial containing dry acetonitrile (1 mL), Kryptofix 2.2.2 (5 mg,13.3 µmol) and potassium carbonate (2 mg, 14.5 µmol). The solvent was evaporated under a stream of nitrogen at 90°C with a reducing vacuum. This azeotropic drying was repeated twice by further addition of acetonitrile (2 x 1 mL). The tosylate precursor 2-(2-(4-(2-tosylethoxy)phenyl)-6-chloroimidazo[1,2-a]pyridin-3-yl)-*N,N*-diethylacetamide (2 mg, 3.6 µmol) was dissolved in acetonitrile (1 ml) and added to the dried K₂₂₂.K₂CO₃.KF complex. Stirring and heating at 90°C continued for 10 to 15 mins before the reaction mixture was diluted with mobile phase and purified by reverse phase HPLC on an Alltech Alltima, C18, 10µ, 250 x 22 mm column eluting at 10 mLs/min with 45% acetonitrile, 55% water with 0.1% TFA. The radioactive peak between 13 to 14 mins was collected, dried *in vacuo* and then formulated in saline for biological evaluation, typically 25 µCi/100µl saline solution per rat for biodistribution studies.

### Example 19 - Characterisation data for various compounds

By the general procedures depicted in Schemes 1 to 4, and by utilising methods A - J, the following compounds were synthesised and characterised.

### PBR098

### N,N-diethyl-2-(2-(4-hydroxyphenyl)-5,7-dimethylpyrazolo[1,5-a]pyrimidin-3-yl)acetamide

m.p.: 234-235°C.
¹H NMR (400 MHz, DMSO) δ 1.02 (t, J = 7.0 Hz, 3H, CH₃), 1.17 (t, J = 7.0 Hz, 3H, CH₃), 2.48 (s, 3H, CH₃), 2.69 (s, 3H, CH₃), 3.28 (q, *J* = 7.0 Hz, 1H, NCH₂), 3.52 (q, *J* = 7.0 Hz, 1H, NCH₂), 3.82 (s, 2H, CH₂), 6.82 (d, *J* = 8.8 Hz, 2H, ArCH), 6.83 (s, 1H, ArCH), 7.58 (d, *J* = 8.8 Hz, 2H, ArCH), 9.63 (br s, 1H, OH).
MS: ES(+ve) *m*/*z* 353 (M+1).
Elemental Analysis: Calculated for C₂₀H₂₄N₄O₂, C = 68.16; H = 6.86; N = 15.90. Found C = 67.97, H = 6.95, N =15.85.

### PBR099

### 2-(2-(4-(2-fluoroethoxy)phenyl)-5,7-dimethylpyrazolo[1,5-a]pyrimidin-3-yl)-N,N-diethylacetamide

m.p.: 148°C.
¹H NMR (400 MHz, DMSO) δ 1.02 (t, *J* = 7.0 Hz, 3H, CH₃), 1.18 (t, *J* = 7.0 Hz, 3H, CH₃), 2.48 (s, 3H, CH₃), 2.69 (s, 3H, CH₃), 3.28 (q, *J =* 7.0 Hz, 1H, NCH₂), 3.52 (q, *J* = 7.0 Hz, 1H, NCH₂), 3.83 (s, 2H, CH₂), 4.29 (dm *J* = 30.1 Hz, 2H, OCH₂), 4.76 (dm, *J* = 47.6 Hz, 2H, CH₂F), 6.84 (s, 1H, ArCH), 7.06 (d, *J* = 8.8 Hz, 2H, ArCH), 7.72 (d, *J* = 8.8 Hz, 2H, ArCH).
¹³C NMR (100 MHz, DMSO) δ 13.1 (CH₃), 14.2 (CH₃), 16.3 (CH₃), 24.2 (CH₃), 27.5 (CH₂), 39.81 (NCH-₂), 41.6 (NCH₂), 67.1 (d, *J* = 18.9 Hz, OCH₂), 82.1 (d, *J* = 66.0 Hz, CH₂F), 100.6 (C), 108.3 (ArCH), 114.5 (ArCH), 126.3 (C), 129.3 (ArCH), 144.5 (C), 147.2 (C), 153.4 (C), 157.3 (C), 158.3 (C), 169.0 (CO).
Elemental Analysis: Calculated for C₂₂H₂₇FN₄O₂, C = 66.31; H = 6.83; N = 14.06. Found C = 66.47, H = 6.89, N=14.17.

### PBR101

### 2-(6-chloro-2-(4-hydroxyphenyl)-imidazo[1,2-a]pyridin-3-yl)-N,N-diethylacetamide

¹H NMR (400 MHz, DMSO), δ 1.06 (t, *J* = 7.1 Hz, 3H, CH₃), 1.16 (t, *J* = 7.1 Hz, 3H, CH₃), 3.32 (q, *J* = 7.1 Hz, 2H, NCH₂), 3.44 (q, *J* = 7.1 Hz, 2H, NCH₂), 4.18 (s, 2H, CH₂), 6.83 (d, *J* = 8.8 Hz, 2H, ArCH), 7.26 (dd, *J* = 9.4, 1.8 Hz, 1H, ArCH), 7.41 (d, *J* = 8.8 Hz, 2H, ArCH), 7.57 (d, *J* = 9.4 Hz, 1H, ArCH), 8.49 (d, *J* 1.8 Hz, 1H, ArCH), 9.61 (br s, 1H, OH).
¹³C NMR (100 MHz, DMSO) δ 13.0 (CH₃), 14.11 (CH₃), 28.7 (CH₂), 39.9 (NCH₂), 41.6 (NCH₂), 115.4 (ArCH), 116.1 (C), 117.0 (ArCH), 118.3 (C), 122.9 (ArCH), 124.5 (ArCH), 125.0 (C), 129.1 (ArCH), 142.1 (C), 143.9 (C), 157.2 (C), 167.1 (CO).
MS: ES(+ve) *m*/*z* 358 (M+1).

### PBR102

### 2-(2-(4-(2-fluoroethoxy)phenyl)-6-chloroimidazo[1,2-a]pyridin-3-yl)-N,N-diethylacetamide

m.p.: 142°C.
¹H NMR (400 MHz, DMSO), δ 1.06 (t, *J* = 7.0 Hz, 3H, CH₃), 1.18 (t, *J* = 7.0 Hz, 3H, CH₃), 3.34 (q, *J* = 7.0 Hz, 2H, NCH₂), 3.46 (q, *J* = 7.0 Hz, 2H, NCH₂), 4.22 (s, 2H, CH₂), 4.30 (dm, *J* = 30.1 Hz, 2H, OCH₂), 4.78 (dm, *J* = 47.6 Hz, 2H, CH₂F), 7.08 (d, *J* = 9.1 Hz, 2H, ArCH), 7.29 (dd, *J* = 9.4, 2.0 Hz, 1 H, ArCH), 7.55 (d, *J* = 9.1 Hz, 2H, ArCH), 7.62 (d, *J* = 9.4 Hz, 1H, ArCH), 8.52 (d, *J* = 2.0 Hz, 1H, ArCH).
¹³C NMR (100 MHz, DMSO) δ 13.0 (CH₃), 14.1 (CH₃), 28.7 (CH₂), 39.9 (NCH₂), 41.7 (NCH₂), 67.1 (d, *J* = 18.8 Hz, OCH₂), 82.3 (d, *J* = 166.8 Hz, CH₂F), 114.7 (ArCH), 116.5 (C), 117.1 (ArCH), 118.4 (C), 123.0 (ArCH), 124.7 (ArCH), 127.0 (C), 129.0 (ArCH), 142.2 (C), 143.3 (C), 157.9 (C), 167.0 (CO).
MS: ES(+ve) *m*/*z* 404 (M+1), 442 (M+K).
Elemental Analysis: Calculated for C₂₁H₂₃ClFN₃O₂, C = 62.45; H = 5.74; N = 10.40. Found C = 62.51, H = 5.79, N =10.43.

### PBR103

### 2-(2-(4-(2-fluoroethoxy)phenyl)-6-chloroimidazo[1,2-a]pyridin-3-yl)-N,N-dipropylacetamide

m.p.: 158-160°C.
¹H NMR (400 MHz, DMSO) δ 0.81 (t, *J* = 7.3 Hz, 3H, CH₃), 0.85 (t, *J* = 7.3 Hz, 3H, CH₃), 1.5 (m, 2H, CH₂), 1.6 (m 2H, CH₂), 3.25 (m, 2H, NCH₂), 3.32 (m, 2H, NCH₂), 4.23 (s, 2H, CH₂), 4.29 (dm, *J* = 30.1 Hz, 2H, OCH₂), 4.76 (dm, *J* = 47.9 Hz, 2H, CH₂F), 7.06 (d, *J* = 9.0 Hz, 2H, ArCH), 7.29 (dd, *J* = 9.6, 2.0 Hz, 1 H, ArCH), 7.56 (d, *J* = 9.0 Hz, 2H, ArCH), 7.61 (d, *J* = 9.6 Hz, 1 H, ArCH), 8.50 (d, *J* = 2.0 Hz, 1H, ArCH).
¹³C NMR (100 MHz, DMSO), δ 11.0 (CH₃), 11.2 (CH₃), 20.5 (CH₂), 21.6 (CH₂), 28.8 (CH₂), 47.1 (NCH₂), 49.0 (NCH₂), 67.1 (d, *J* = 19 Hz, OCH₂), 82.3 (d, *J* = 165.7 Hz, CH₂F), 114.6 (ArCH₂), 116.4 (C), 117.1 (ArCH), 118.4 (C), 123.0 (ArCH), 124.7 (ArCH), 127.0 (C), 129.1 (ArCH), 142.2 (C), 143.3 (C), 157.9 (C), 167.5 (CO).
MS: ES(+ve) *m*/*z* 432 (M+1).
Elemental Analysis: Calculated for C₂₃H₂₇ClFN₃O₂ ; C = 63.96, H = 6.30, N = 9.73. Found, C = 64.15, H = 6.36, N = 9.78.

### PBR104

### 2-(2-(4-(2-fluoroethoxy)phenyl)-6-chloroimidazo[1,2-a]pyridin-3-yl)-1-(pyrrolidin-1-yl)ethanone

m.p.: 150-152°C.
¹H NMR (400 MHz, DMSO) δ 1.84 (m, 2H, CH₂), 1.95 (m, 2H, CH₂), 3.36 (t, *J* = 6.7 Hz, 2H, NCH₂), 3.58 (t, *J* = 6.7 Hz, 2H, NCH₂), 4.15 (s, 2H, CH₂), 4.29 (dm, *J* = 30.1 Hz, 2H, OCH₂), 4.77 (dm, *J* = 47.9 Hz, 2H, CH₂F), 7.07 (d, *J* = 9.0 Hz, 2H, ArCH), 7.29 (dd, *J* = 9.4, 2.0 Hz, 1H, ArCH), 7.59 (d, *J* = 9.0 Hz, 2H, ArCH), 7.61 (d, *J* = 9.0 Hz, 1H, ArCH), 8.55 (d, *J* = 2.0 Hz, 1H, ArCH).
¹³C NMR (100 MHz, DMSO) δ 24.0 (CH₂), 25.7 (CH₂), 29.9 (CH₂), 45.7 (NCH₂), 46.1 (NCH₂), 67.1 (d, *J* = 19.0 Hz, OCH₂), 82.3 (d, *J* = 165.7 Hz, CH₂F), 114.7 (ArCH), 116.1 (C), 117.1 (ArCH), 118.5 (C), 123.1 (ArCH), 124.8 (ArCH), 127.0 (C), 129.1 (ArCH), 142.2 (C), 143.5 (C), 157.8 (C), 166.1 (CO).
MS: ES(+ve) *m*/*z* 402 (M+1).
Elemental Analysis: Calculated for C₂₁H₂₁ClFN₃O₂; C = 62.76; H = 5.27; N = 10.46. Found C = 63.02, H = 5.38, N = 10.41.

### PBR105

### 2-(2-(4-(2-fluoroethoxy)phenyl)-6-chloroimidazo[1,2-a]pyridin-3-yl)-N,N-dimethylacetamide

m.p.: 194-196°C.
¹H NMR (400 MHz, DMSO), δ 2.90 (s, 3H, CH₃), 3.13 (s, 3H, CH₃), 4.21 (s, 2H, CH₂), 4.29 (dm, *J* = 30.1 Hz, 2H, OCH₂), 4.76 (dm, *J* = 47.9 Hz, 2H, CH₂F), 7.08 (d, *J* = 8.8 Hz, 2H, ArCH), 7.28 (dd, *J* = 9.6, 2.0 Hz, 1 H, ArCH), 7.57 (d, *J* = 8.8 Hz, 2H, ArCH), 7.61 (d, *J* = 9.6 Hz, 1H, ArCH), 8.54 (d, *J* = 2.0 Hz, 1 H, ArCH).
¹³C NMR (100 MHz, DMSO), δ 28.9 (CH₂), 35.3 (NCH₃), 37.0 (NCH₃), 66.1 (d, *J* = 19.0 Hz, OCH₂), 82.3 (d, *J* = 165.7 Hz, CH₂F), 114.7 (ArCH), 116.3 (C), 117.1 (ArCH), 118.5 (C), 123.0 (ArCH), 124.7 (ArCH), 127.0 (C), 129.0 (ArCH), 142.2 (C), 143.5 (C), 157.8 (C), 167.9 (CO).
MS: ES(+ve) *m*/*z* 376 (M+1).
Elemental Analysis: Calculated for C₁₉H₁₉ClFN₃O₂; C = 60.72; H = 5.10; N = 11.18. Found C = 60.60, H = 5.09, N =11.15

### PBR106

### 2-(2-(4-(2-fluoroethoxy)phenyl)-6-chloroimidazo[1,2-a]pyridin-3-yl)-N-methylacetamide

m.p.: 255°C.
¹H NMR (400 MHz, DMSO) δ 2.65 (d, *J* = 4.7 Hz, 3H, NCH₃), 3.99 (s, 2H, CH₂), 4.30 (dm, *J* = 30.1 Hz, 2H, OCH₂), 4.77 (dm, *J* = 47.6 Hz, 2H, CH₂F), 7.08 (d, *J* = 9.0 Hz, 2H, ArCH), 7.29 (dd, *J* = 9.6, 2.0 Hz, 1 H, ArCH), 7.62 (d, *J =* 9.6 Hz, 1 H, ArCH), 7.73 (d, *J =* 9.0 Hz, 1H, ArCH), 8.21 (q, *J* = 4.7 Hz, 1H, NH), 8.61 (d, *J* = 2.0 Hz, 1H, ArCH).
¹³C NMR (100 MHz, DMSO) δ 25.8 (CH₃), 30.7 (CH₂), 67.1 (d, *J* = 19.2 Hz, OCH₂), 82.3 (d, *J* = 165.8 Hz, CH₂F), 114.6 (ArCH), 115.8 (C), 117.2 (ArCH), 118.6 (C), 122.9 (ArCH), 124.9 (ArCH), 126.9 (C), 129.1 (ArCH), 142.2 (C), 143.4 (C), 157.9 (C), 168.6 (CO).
MS: ES(+ve) *m*/*z* 362 (M+1).
Elemental Analysis: Calculated for C₁₈H₁₇ClFN₃O₂; C = 59.76; H = 4.74; N = 11.61. Found C = 59.85, H = 4.77, N = 11.67.

### PBR107

### 2-(2-(4-(2-fluoroethoxy)phenyl)-6-chloroimidazo[1,2-a]pyridin-3-yl)-N-ethyl-N-methylacetamide m.p.: 117-118°C.

Two rotamers observed in NMR.
¹H NMR (400 MHz, DMSO), δ 1.06 (t, *J* = 7.0 Hz,1.5 H, CH₃), 1.17 (t, *J* = 7.0 Hz, 1.5 H, CH₃), 2.87 (s, 1.5H, NCH₃), 3.10 (s, 1.5H, NCH₃), 3.37 (q, *J* = 7.0 Hz, 1 H, NCH₂), 3.47 (q, *J* = 7.0 Hz, 1H, NCH₂), 4.19 (s, 1 H, CH₂), 4.23 (s, 1H, CH₂), 4.29 (dm, *J* = 29.8 Hz, 2H, OCH₂), 4.76 (dm, *J* = 47.9 Hz, 2H, CH₂F), 7.08 (dm, *J* = 8.8 Hz, 2H, ArCH), 7.28 (dd, *J* = 9.4, 2.0 Hz, 0.5H, CH), 7.29 (dd, *J* = 9.4, 2.0 Hz, 0.5H, CH), 7.55 (d, *J* = 8.8 Hz, 1 H, ArCH), 7.57 (d, *J* = 8.8 Hz, 1H, ArCH), 7.61 (d, *J* = 9.4 Hz, 0.5H, CH), 7.61 (d, *J* = 9.4 Hz, 0.5H, CH), 8.52 (d, *J* = 2.0 Hz, 0.5H, CH), 8.53 (d, *J* = 2.0 Hz, 0.5H, CH).
¹³C NMR (100 MHz, DMSO), δ 12.3, 13.3 (CH₃), 28.5, 29.0 (CH₂), 32.5, 34.5 (NCH₃), 42.0, 43.8 (NCH₂), 67.1 (d, *J* = 19.0 Hz, OCH₂), 81.3 (d, *J* = 165.6 Hz, CH₂F), 114.7 (ArCH), 116.4 (C), 117.1 (ArCH), 118.4 (C), 123.0 (ArCH), 124.7 (ArCH), 127.0 (C), 129.0 (ArCH), 142.2 (C), 143.4 (C), 157.8 (C), 167.4 (CO).
Elemental Analysis: Calculated for C₂₀H₂₁ClFN₃O₂; C = 61.62; H = 5.43; N = 10.78. Found C = 61.34, H = 5.48, N =10.70.

### PBR110

### 2-(2-(4-(2-fluoroethoxy)phenyl)-6-chloroimidazo[1,2-a]pyridin-3-yl)-N-isopropylacetamide

m.p.: 205-206 °C.
¹H NMR (400 MHz, DMSO) δ 1.11 (d, *J* = 6.7 Hz, 6H, 2 x CH₃), 3.89 (m, 1 H, NCH), 3.96 (s, 2H, CH₂), 4.29 (dm, *J* = 30.1 Hz, 2H, OCH₂), 4.76 (dm, *J* = 47.6 Hz, 2H, CH₂F), 7.07 (d, *J* = 8.8 Hz, 2H, ArCH), 7.29 (dd, *J* = 9.6, 2.0 Hz, 1H., CH), 7.62 (d, *J* = 9.6 Hz, 1H, CH), 7.74 (d, *J* = 8.8 Hz, 2H, ArCH), 8.29 (d, *J* = 7.9 Hz, 1 H, CH), 8.61 (d, *J* = 2.0 Hz, 1H, CH).
¹³C NMR (100 MHz, DMSO) δ 22.3 (CH₃), 30.7 (CH₂), 40.7 (NCH), 67.1 (d, *J* = 19.0 Hz, OCH₂), 81.3 (d, *J* = 165.6 Hz, CH₂F), 114.6 (ArCH), 116.2 (C), 117.2 (CH), 118.6 (C), 122.8 (CH), 124.8 (CH), 126.9 (C), 129.2 (ArCH), 142.1 (C), 143.5 (C), 157.9 (C), 167.1 (CO).
MS: ES(+ve) *m*/*z* 390 (M+1).
Elemental Analysis: Calculated for C₂₀H₂₁ClFN₃O₂, C = 61.62; H = 5.43; N = 10.78. Found C = 61.75, H = 5.51, N =10.89.

### PBR111

### 2-(2-(4-(3-fluoropropoxy)phenyl)-6-chloroimidazo[1,2-a]pyridin-3-yl)-N,N-diethylacetamide

m.p.: 110-114°C
¹H NMR (400 Hz, CD₃CN) δ 1.13 (t, *J* = 7.1 Hz, 3H,CH₃); 1.18 (t, *J* = 7.1 Hz, 3H, CH₃); 2.20 (m, 2H, CH₂CH₂*CH₂*CH₂CH₂); 3.40(q, *J* = 7.1 Hz, 2H,*CH₂*CH₃); 3.44(q, *J* = 7.1 Hz, 2H, C*H₂*CH₃); 4.11 (s, 2H, CH₂C=O); 4.17 (t, *J* = 6.2 Hz, 2H, OCH₂); 4.59-4.75 (dt, *J* = 5.9, 47.3 Hz, 2H, FCH₂); 7.05 (d, *J* = 8.8 Hz, 2H, ArCH×2); 7.24 (dd, *J* = 2.0, 9.5 Hz, 1H, CH), 7.54 (dd, *J* = 0.8, 9.5 Hz, 1H, CH), 7.58 (d, *J* = 8.8 Hz, 2H, ArCH×2); 8.24 (dd, *J* = 0.8, 2.0 Hz, 1H, CH).

### PBR113

### 2-(2-(4-(3-hydroxypropoxy)phenyl)-6-chloroimidazo[1,2-a]pyridin-3-yl)-N,N-diethylacetamide

m.p.: 119-120°C.
¹H NMR (400 Hz, DMSO) δ1.08 (t, *J* = 6.9 Hz, 3H, CH₃), 1.23 (t, *J* = 6.9 Hz, 3H, CH₃), 1.90 (m, 2H, CH₂), 3.36 (q, *J* = 6.9 Hz, 2H, CH₂CH₃, superimposed with H₂O peak), 3.45 (q, *J* = 6.9 Hz, 2H, C*H₂*CH₃), 3.58 (t, *J* = 6.1 Hz, 2H, HOC*H₂*CH₂), 4.10 (t, *J* = 6.2 Hz, 2H, OCH₂CH₂), 4.22 (s, 2H, COCH₂), 7.05 (d, *J* = 8.6 Hz, 2H, ArCH×2), 7.31 (dd, *J* = 1.7, 9.5 Hz, 1H, CH), 7.55 (d, *J* = 8.6 Hz, 2H, ArCH×2), 7.62(d, *J* = 9.5 Hz, 1H, CH), 8.52 (d, *J* = 1.7 Hz, 1H, CH).

### PBR114

### 2-(-2-(4-(2-hydroxyethoxy)phenyl)-6-chloroimidazo[1,2-a]pyridin-3-yl)-N,N-diethylacetamide

¹H NMR (400 Hz, DMSO) δ 1.07 (t, *J* = 7.1 Hz, 3H, CH₃), 1.17 (t, *J* = 7.1 Hz, 3H, CH₃), 3.34 (q, *J* = 7.1 Hz, 2H, CH₂), 3.45 (q, *J* = 7.1 Hz, 2H, CH₂), 3.73 (dt, , *J* = 4.9, 5.3 Hz, 2H, CH₂OH), 4.03 (t, *J* = 5.3Hz, 2H, CH₂O), 4.20 (s, 2H, CH₂CO), 4.87 (t, *J* = 5.5 Hz, 1 H, OH), 7.03 (d, *J* = 8.8Hz, 2H, ArCH×2), 7.29 (dd, *J* = 9.5, 2.0Hz, 1 H, CH), 7.52 (d, *J* = 8.8Hz, 2H, ArCH×2), 7.61 (dd, *J* = 0.7, 9.5Hz, 1H, CH), 8.50 (dd, *J* = 0.7, 2.0Hz, 1 H, CH).
¹³C NMR (400 MHz, DMSO) δ 13.06, 14.16 (CH₃), 28.72, 39.90, 41.68 (CH₂), 59.57 (OHCH₂), 69.60 (OCH₂),114.62 (ArCH×2), 116.41 (C), 117.13 (CH), 118.43 (C), 123.02, 124.72 (CH), 126.51 (C), 129.03 (ArCH×2), 142.22, 143.47, 158.44 (C), 167.06 (C=O).
MS: ES(+ve) *m*/*z* 402 (M+1).

### PBR115

### 2-(2-(3-bromophenyl)-6-chloroimidazo[1,2-a]pyridin-3-yl)-N,N-diethylacetamide

m.p.: 154-155°C.
¹H NMR (400 MHz, DMSO) δ 1.10 (t, *J* = 7.1 Hz, 3H, CH₃), 1.23 (t, *J* = 7.1 Hz, 3H, CH₃), 3.36 (q, *J* = 7.1 Hz, 2H, CH₂), 3.52 (q, J *=* 7.1 Hz, 2H, CH₂), 4.28 (s, 2H, COCH₂), 7.35 (dd, *J* = 1.9, 9.5 Hz, 1H, CH), 7.46 (m, 1H, ArCH), 7.60 (m, 1H, ArCH), 7.68 (d, *J* = 9.5 Hz, 1 H, CH), 7.66 (m, 1 H, ArCH, superimposed), 7.72 (m, 1H, ArCH), 8.65 (d, *J=1.9* Hz, CH).
¹³C NMR (400 MHz, DMSO) δ 13.03, 14.14 (CH₃), 28.49, 39.90 (CH₂), 41.68 (CO*C*H₂), 119.19, 119.65, 120.66, 123.58, 124.91, 127.09, 128.26, 131.68, 132.04, 132.51, 138.24, 143.34, 144.04 (C), 168.60 (C=O).
MS: ES(+ve) *m*/*z* 420,422 (M+1).

### PBR 117

### 2-(6-chloro-2-(4-methoxyphenyl)imidazo[1,2-b]pyridazin-3-yl)-N,N-diethylacetamide

m.p.: 138-139°C.
¹H NMR (400 MHz, CD₃CN) δ 1.11 (t, *J* = 7.1 Hz, 3H, CH₃); 1.28 (t, *J* = 7.1 Hz, 3H, CH₃): 3.40 (q, *J* = 7.1 Hz, 2H, NCH₂); 3.55 (q, *J* = 7.1 Hz, 2H, NCH₂); 3.84 (s, 3H, OCH₃); 4.17 (s, 2H, CH₂C=O); 7.03 (d, *J* = 8.9 Hz, 2H, 2 × ArCH); 7.15 (d, *J* = 9.4 Hz, 1 H, CH); 7.73 (d, *J* = 8.9 Hz, 2H, 2 × ArCH); 7.93 (d, *J* = 9.4 Hz, 1H, CH).
MS: ES(+ve) *m*/*z* 373 (M+1), 395 (M+Na).

### PBR118

### 2-(2-(3-bromo-4-methoxyphenyl)-6-chloroimidazo[1,2-b]pyridazin-3-yl)-N,N-diethylacetamide

m.p.: 188°C.
¹H NMR (400 MHz, DMSO) δ 1.07 (t, *J* = 7.1 Hz, 3H, CH₃); 1.26 (t, *J* = 7.1 Hz, 3H, CH₃); 3.33 (q, *J* = 7.1 Hz, 2H, NCH₂); 3.57 (q, *J* = 7.1 Hz, 2H, NCH₂); 3.90 (s, 3H, OCH₃); 4.21 (s, 2H, CH₂C=O); 7.24 (d, *J* = 8.7 Hz, 1H, ArCH), 7.38 (d, *J* = 9.4 Hz, 1H, CH); 7.78 (dd, *J* = 8.7, 2.1 Hz, 1H, ArCH); 7.90 (d, *J* = 2.1 Hz, 1H, ArCH); 8.22 (d, *J* = 9.4 Hz, 1H, CH).

### PBR119

### 2-(2-(4-butoxyphenyl)-6-chloroimidazo[1,2-a]pyridin-3-yl)-N,N-diethylacetamide

m.p.: 118-119°C.
¹H NMR (400 MHz, CD₃CN) δ 0.98 (t, *J* = 7.4Hz, 3H, CH₃); 1.11 (t, *J* = 7.1Hz, 3H, CH₃); 1.15 (t, *J* = 7.1 Hz, 3H, CH₃); 1.50 (m, *J* = 7.4 Hz, 2H, CH₂CH₂CH₃); 1.76 (m, *J* = 7.2 Hz, 2H, OCH₂CH₂CH₂); 3.38 (m, *J* = 7.2 Hz, 4H, 2 X 2H, NCH₂CH₃); 4.03 (t, *J=* 6.5 Hz, 2H, OCH₂); 4.09 (s, 2H, CH₂C=O), 7.0 (d, J = 8.8 Hz, 2H, 2 X ArCH); 7.2 (dd, *J* = 9.6, 2.0 Hz, 1H, CH); 7.5 (dd, *J* = 9.6, 0.7 Hz, 1H, NCCH); 7.5 (d, *J =* 8.8 Hz, 2H, 2 X ArCH); 8.21 (dd, *J* = 2.0, 0.7 Hz, 1H, NCH).

### PBR120

### 2-(2-(4-(4-fluorobutoxy)phenyl)-6-chloroimidazo[1,2-a]pyridin-3-yl)-N,N-diethylacetamide

m.p.: 102-103°C.
¹H NMR (400 MHz, CD₃CN) δ 1.15 (t, *J* = 7.1Hz, 3H,CH₃); 1.20 (t, 3H, *J* = 7.1Hz, 3H, CH₃); 1.9 (m, 4H, 2×CH2, OCH₂*CH₂CH₂*); 3.4 (q, *J* = 7.1 Hz, 2H,CH₂CH₃); 3.45(q, *J* = 7.1 Hz, 2H, CH₂CH₃); 4.10 (t, *J* = 6.1 Hz, 2H, OCH₂); 4.14 (s, 2H, CH₂C=O); 4.48-4.64 (dm, *J =* 47.5 Hz, 2H, FCH₂); 7.00 (d, *J* = 8.8Hz, 2H, ArCH×2); 7.20 (dd, *J* = 9.5, 2.1Hz, 1H, CH); 7.54 (d, *J* = 9.5Hz, 1 H, NCCH); 7.60 (d, *J* = 8.8Hz, 2H, ArCH×2); 8.25 (d, *J* = 2.1 Hz, 1 H, NCH).
MS: ES(+ve) *m*/*z* 432 (M+1).

### PBR121

### 2-(2-(4-tert-butylphenyl)-6-chloroimidazo[1,2-b]pyridazin-3-yl)-N,N-diethylacetamide

m.p.: 184-185°C.
¹H NMR(400MHz, DMSO) δ 1.07 (t, *J* = 7.08 Hz, 3H, CH₂C*H*₃); 1.27 (t, *J* = 7.08 Hz, 3H, CH₂C*H*₃); 1.32 (s, 9H, 3×CH₃); 3.33 (q, *J* = 7.08 Hz, 2H, C*H*₂CH₃); 3.55 (q, *J* = 7.08 Hz, 2H, C*H*₂CH₃); 4.21 (s, 2H, CH₂C=O); 7.36 (d, *J* = 9.40 Hz,1H, CH=CH-C=N); 7.50 (d, *J* = 8.5 Hz, 2H, 2×ArCH); 7.5 (d, *J* = 8.5 Hz, 2H, 2×ArCH); 8.2 (d, *J* = 9.40 Hz, 1H, CH=CH-C=N).

### PBR123

### 2-(2-(4-tert-butylphenyl)-6-iodoimidazo[1,2-b]pyridazin-3-yl)-N,N-diethylacetamide

m.p.: 175-176°C.
¹H NMR(400MHz, DMSO) δ 1.07 (t, *J* = 7.08 Hz, 3H, CH₂C*H*₃); 1.29 (t, *J* = 7.08 Hz, 3H, CH₂C*H*₃); 1.32 (s, 9H, 3×CH₃); 3.33 (q, *J* = 7.08 Hz, 2H, C*H*₂CH₃); 3.55 (q, *J* = 7.08 Hz, 2H, C*H*₂CH₃); 4.19 (s, 2H, CH₂C=O); 7.50 (d, *J* = 8.5 Hz, 2H, 2×ArCH); 7.52 (d, *J* = 9.40 Hz, 1H, CH=CH-C=N); 7.7 (d, *J* = 8.5 Hz, 2H, 2×ArCH); 7.9 (d, *J* = 9.40 Hz, CH=CH-C=N).

### PBR124

### 2-(2-(4-tert-butylphenyl)-6-bromoimidazo[1,2-b]pyridazin-3-yl)-N,N-diethylacetamide

m.p.: 174-175°C.
¹H NMR(400MHz, DMSO) δ 1.04 (t, *J* = 7.08 Hz, 3H, CH₂C*H*₃); 1.24 (t, *J* = 7.08 Hz, 3H, CH₂C*H*₃); 1.30 (s, 9H, 3×CH₃); 3.33 (q, *J* = 7.08 Hz, 2H, C*H*₂CH₃); 3.52 (q, *J* = 7.08 Hz, 2H, C*H*₂CH₃); 4.20 (s, 2H, CH₂C=O); 7.40 (d, *J* = 9.40 Hz,1H, CH=CH-C=N); 7.48 (d, *J* = 8.5 Hz, 2H, 2×ArCH); 7.7 (d, *J* = 8.5 Hz, 2H, 2×ArCH); 8.10 (d, *J* = 9.40 Hz, 1H, CH=CH-C=N).

### PBR125

### 2-(6,8-dichloro-2-(4-methoxyphenyl)imidazo[1,2-a]pyridin-3-yl)-N,N-diethylacetamide

m.p.: 180-181°C.
¹H NMR(400MHz, DMSO) δ 1.07 (t, J = 7.1 Hz, 3H, CH₂C*H*₃), 1.17 (t, *J* = 7.1 Hz, 3H, CH₂C*H*₃), 3.33 (q, *J* = 7.1 Hz, 2H, C*H*₂CH₃), 3.44 (q, *J* = 7.1 Hz, 2H, C*H*₂CH₃); 3.81 (s, 3H, OCH₃), 4.22 (s, 2H, CH₂C=O), 7.04 (d, *J* = 8.9 Hz, 2H, 2×ArCH), 7.55 (d, *J* = 8.9 Hz, 2H, 2×ArCH), 7.63 (d, *J* = 1.7 Hz, 1 H, CH), 8.59 (d, *J* = 1.7 Hz, 1 H, CH).
¹³C NMR (400 MHz, DMSO) δ 13.05, 14.16 (CH₃), 28.91 (CH₂), 39.91, 41.69(*C*H₂CH₃), 55.23 (OCH₃), 114.14 (ArCH×2), 117.54, 118.60, 121.48 (C), 122.55, 123.63 (CH), 126.01 (C), 129.15 (ArCH×2), 139.52,143.75 (C), 159.21 (ArC-O), 166.84(C=O)
MS: ES(+ve) *m*/*z* 406 (M+1).

### PBR126

### 2-(2-(3-bromo-4-methoxyphenyl)-6,8-dichloroimidazo[1,2-a]pyridin-3-yl)-N,N-diethylacetamide m.p.: 190-191°C.

¹H NMR(400MHz, DMSO) δ 1.09 (t, *J* = 7.1 Hz, 3H, CH₂C*H*₃), 1.20 (t, *J* = 7.1 Hz, 3H, CH₂C*H*₃), 3.35 (q, *J* = 7.1 Hz, 2H, C*H*₂CH₃), 3.50 (q, *J* = 7.1 Hz, 2H, C*H*₂CH₃), 3.90 (s, 3H, CH₃), 4.25 (s, 2H, CH₂C=O), 7.24 (d, *J* = 8.7 Hz, 2H, ArCH), 7.63 (dd, *J* = 8.7, 2.2 Hz, 1H, ArCH), 7.66 (d, *J* = 1.7 Hz, CH), 7.72 (d, *J* = 2.2 Hz, 1H, ArCH), 8.67 (d, *J* = 1.7 Hz, CH).
MS: ES(+ve) *m*/*z* 484,486 (M+1).

### PBR128

### 2-(6-bromo-2-(3-bromo-4-methoxyphenyl)imidazo[1,2-b]pyridazin-3-yl)-N,N-diethylacetamide m.p.:180°C.

¹H NMR(400MHz, DMSO) δ 1.07 (t, *J* = 7.1 Hz, 3H, CH₂C*H*₃), 1.25 (t, *J* = 7.1 Hz, 3H, CH₂C*H*₃), 3.33 (q, *J* = 7.1 Hz, 2H, C*H*₂CH₃), 3.57 (q, *J* = 7.1 Hz, 2H, C*H*₂CH₃), 3.90 (s, 3H, OCH₃), 4.20 (s, 2H, CH₂C=O), 7.22 (d, *J* = 6.8 Hz, 1 H, ArCH), 7.43 (d, *J* = 9.4 Hz, 2H, CH), 7.78 (dd, *J* = 6.8, 2.2 Hz, 2H, 2×ArCH), 7.90 (d, *J* = 2.2 Hz, 1H, ArCH), 8.12 (d, *J* = 9.4Hz, 1H, CH).
MS: ES(+ve) *m*/*z* 495,497 (M+1).

### PBR129

### 2-(6-bromo-2-(4-methoxyphenyl)imidazo[1,2-b]pyridazin-3-yl)-N,N-diethylacetamide m.p.: 124-125°C.

¹H NMR(400MHz, CD₃CN) δ 1.13 (t, *J* = 7.1 Hz, 3H, CH₂C*H*₃); 1.32 (t, *J* = 7.1 Hz, 3H, CH₂C*H*₃); 3.40 (q, *J* = 7.1 Hz, 2H, C*H*₂CH₃); 3.57 (q, *J* = 7.1 Hz, 2H, C*H*₂CH₃); 3.85 (s, 3H, OCH₃), 4.15 (s, 2H, CH₂C=O); 7.04 (d, *J* = 8.9 Hz, 2H, 2×ArCH); 7.24 (d, *J* = 9.40 Hz, 1H, CH); 7.74 (d, *J* = 8.9 Hz, 2H, 2×ArCH); 7.84 (d, *J* = 9.40 Hz, 1H, CH).
MS: ES(+ve) *m*/*z* 417 (M+1).

### PBR130

### 2-(2-(4-(3-fluoropropoxy)phenyl)-6,8-dichloroimidazo[1,2-a]pyridin-3-yl)-N,N-diethylacetamide

m.p.: 132-134°C.
¹H NMR (400 MHz, CD3CN) δ 1.07 (t, *J* = 7.05Hz, 3H,CH₃); 1.18 (t, *J* = 7.05Hz, 3H, CH₃); 2.1 (m, 2H, CH₂CH₂*CH₂* CH₂CH₂); 3.34(q, *J* = 7.05Hz, 2H,C*H₂*CH₃); 3.45(q, *J* = 7.05Hz, 2H, C*H*₂CH₃); 4.1 (t, *J* = 6.3Hz, 2H, OCH₂); 4.22 (s, 2H, CH₂C=O); 4.55-4.70 (dt, *J* = 6.1, 47.2 Hz, 2H, FCH₂); 7.1 (d, *J* = 8.8Hz, 2H, ArCH×2); 7.54 (d, *J* = 8.8Hz, 2H, ArCH×2); 7.63 (d, *J* = 1.8Hz, 1H, CH); 8.59 (d, *J* = 1.8Hz, 1H, CH).
¹³C NMR (400 MHz, DMSO) δ 13.02 (CH₃), 14.14 (CH₃), 28.88 (CH₂CH₂*CH₂* CH₂CH₂), 29.65, 29.84 (*CH₂*CH₃), 41.66 (*C*H₂C=O), 63.64 (OCH₂), 80.01, 81.62 (FCH₂), 114.64 (ArCH × 2), 117.52, 118.59,121.46 (C), 122.52, 123.60 (CH), 139.49,143.68 (C), 158.33 (ArC-O), 166.81 (C=O).

### PBR131

### N-butyl-2-(6,8-dichloro-2-(4-methoxyphenyl)imidazo[1,2-a]pyridin-3-yl)-N-methylacetamide

m.p.: 118-120°C.
Rotamers were observed in the NMR, 58:42 based on ¹H NMR integration.

### Rotamer 1 (major):

¹H NMR (400 MHz, DMSO) δ 0.89 (t, *J* = 7.2Hz, 3H, CH₂C*H*₃), 1.26 (m, 2H, C*H₂*CH₃), 1.50 (m, 2H, C*H*₂CH₂CH₃), 3.10 (s, 3H, NCH₃), 3.35 (m, 2H, NCH₂), 3.81 (s, 3H, OCH₃), 4.22 (s, 2H, CH₂), 4.45 (m, 1H, CHCH₃), 7.05 (d, *J* = 8.8 Hz, 2H, ArCH×2), 7.57 (d, *J* = 8.8 Hz, 2H , ArCH×2), 7.6 (d, *J* = 1.6 Hz, 1H, CH), 8.58(d, *J* = 1.6 Hz, 1H, CH).
¹³C NMR (100 MHz, DMSO) δ 15.37 (CH₂*C*H₃), 21.02 (*C*H₂CH₃), 30.49 (*CH₂*CH₂CH₃), 36.62 (NCH₃), 48.39 (CH₂), 55.88 (OCH₃), 115.78 (ArCH×2), 119.24 (C), 120.17 (CH), 123.16 (ArC), 124.11 (C-Cl), 125.05 (C-Cl), 127.5 (CH), 130.79 (ArCH×2), 145.55 (C-phenyl), 160.82 (ArC-OMe), 169.02 (C=O).

### Rotamer 2 (minor):

¹H NMR (400 MHz, DMSO) δ 0.91 (t, *J* = 7.2Hz, 3H, CH₂C*H*₃); 1.26 (m, 2H, C*H*₂CH₂CH₃); 1.50 (m, 2H, C*H*₂CH₃); 2.86 (s, 3H, NCH₃), 3.35 (m, NCH₂), 3.81 (s, 3H, OCH₃), 4.23 (s, 2H, CH₂), .32 (dd, *J* = 2.0, 9.5 Hz, 1H, CH), 7.03 (d, *J* = 8.8 Hz, 2H, ArCH×2), 7.55 (d, *J* = 8.8 Hz, 2H , ArCH×2), 7.64 (d, *J* = 2.0 Hz, 1H, CH), 8.59 (d, *J* = 2.0 Hz, 1H, CH).
¹³C NMR (100 MHz, DMSO) δ 15.50 (CH₂*C*H₃), 21.10 (*C*H₂CH₃), 31.64 (*CH*₂CH₂CH₃), 34.8 (NCH₃), 48.39 (CH₂), 55.88 (OCH₃), 118.80 (ArCH×2), 119.24 (C), 120.17 (CH), 123.16 (ArC), 124.11 (C-Cl), 125.05 (C-Cl), 127.5 (CH), 130.93 (ArCH×2), 145.55 (C-phenyl), 160.82 (ArC-OMe), 169.02 (C=O).
MS: ES(+ve) *m*/*z* 420 (M+1).

### PBR132

### 2-(2-(4-tert-butylphenyl)-6-fluoroimidazo[1,2-b]pyridazin-3-yl)-N,N-diethylacetamide

m.p.: 160-162°C
¹H NMR(400MHz, CD3CN) δ 1.12 (t, *J* = 7.1 Hz, 3H, CH₂C*H*₃); 1.31 (t, *J* = 7.1 Hz, 3H, CH₂C*H*₃); 1.37 (s, 9H, 3×CH₃); 3.4 (q, *J* = 7.1 Hz, 2H, C*H*₂CH₃); 3.56 (q, *J* = 7.1 Hz, 2H, C*H₂*CH₃); 4.17 (s, 2H, CH₂C=O); 7.02 (d, *J* = 9.6 Hz, 1H, CH=CH-C=N); 7.56 (d, *J* = 8.5 Hz, 2H, 2×ArCH); 7.72 (d, *J* = 8.5 Hz, 2H, 2×ArCH); 8.10 (d, *J* = 9.6 Hz, 1H, CH=CH-C=N).

### PBR133

### 2-(6-chloro-2-(4-methoxyphenyl)imidazo[1,2-b]pyridazin-3-yl)-N,N-diethylacetamide m.p.: 132-134°C.

¹H NMR (400 MHz, DMSO) δ1.05 (t, *J* = 7.1 Hz, 3H, CH₂C*H*₃), 1.25 (t, *J* = 7.1 Hz, 3H, CH₂C*H*₃), 3.30 (q, *J* = 7.1 Hz, 2H, C*H*₂CH₃), 3.50 (q, *J* = 7.1 Hz, 2H, C*H*₂CH₃), 3.80 (s, 3H, OCH₃), 4.14 (s, 2H, CH₂C=O), 7.05 (d, *J* = 8.9 Hz, 2H, 2×ArCH), 7.24 (d, *J* = 9.6 Hz, 1H, CH), 7.69 (d, *J* = 8.9 Hz, 2H, 2×ArCH), 8.32 (dd, *J* = 9.6, 7.5 Hz, CH-CF).
¹³C NMR (400 MHz, DMSO) δ 13.06, 14.22 (CH₃), 28.46, 39.95, 41.69 (CH₂), 55.21(OCH₃), 106.93, 107.30 (*C*HCF), 114.18 (ArCH×2), 120.85 (C), 126.31 (C), 128.72 (ArCH×2), 129.26, 129.37 (*C*HCHCF), 136.82 (C), 144.00 (*C*CHCHCF), 158.72, 161.00 (CF), 166.57 (C=O).

### PBR134

### 2-(6,8-dichloro-2-(4-methoxyphenyl)imidazo[1,2-a]pyridin-3-yl)-N-methyl-N-phenylacetamide

m.p.: 186-188°C.
¹H NMR (400 MHz, DMSO) δ 3.24 (s, 3H, NCH₃), 3.80 (s, 3H, OCH₃), 3.88 (s, 2H, CH₂), 7.03 (d, *J* = 8.7 Hz, 2H, ArCH×2), 7.43 (d, *J* = 8.7 Hz, 2H , ArCH×2), 7.3-7.57 (m, 5H, ArCH), 7.61 (d, *J* = 1.6 Hz, 1H, CH), 8.72(d, *J*= 1.6 Hz, 1H, CH)
MS: ES(+ve) *m*/*z* 440 (M+1).

### PBR135

### N-benzyl-2-(6,8-dichloro-2-(4-methoxyphenyl)imidazo[1,2-a]pyridin-3-yl)-N-methylacetamide

m.p.: 164-167°C.
Rotamers were observed in the NMR, 60:40 based on ¹H NMR integration.

### Rotamer 1 (major):

¹H NMR (400 MHz, DMSO) δ 3.09 (s, 3H, NCH₃), 3.80 (s, 3H, OCH₃), 4.33 (s, 2H, CH₂), 4.56 (CH₂-phenyl), 7.01 (d, *J* = 8.8 Hz, 2H, ArCH×2), 7.2-7.45 (m, 5H, ArCH), 7.57 (d, *J* = 8.8 Hz, 2H , ArCH×2), 7.62 (d, *J* = 1.7 Hz, 1H, CH), 8.65(d, *J*= 1.7 Hz, 1H, CH).

### Rotamer 2 (minor):

¹H NMR (400 MHz, DMSO) δ 2.94 (s, 3H, NCH₃), 3.78 (s, 3H, OCH₃), 4.23 (s, 2H, CH₂), 4.72 (CH₂-phenyl), 6.93 (d, *J* = 8.8 Hz, 2H, ArCH×2), 7.2-7.45 (m, 5H, ArCH), 7.42 (d, *J* = 8.8 Hz, 2H, ArCH×2), 7.62 (d, *J* = 1.7 Hz, 1H, CH), 8.57(d, *J* = 1.7 Hz, 1H, CH).
MS: ES(+ve) *m*/*z* 454 (M+1).

### PBR136

### 2-(2-(4-(2-fluoroethoxy)phenyl)-6,8-dichloroimidazo[1,2-a]pyridin-3-yl)-N,N-diethylacetamide

m.p.: 144-146°C.
¹H NMR (400 MHz, CD₃CN) δ 1.07 (t, *J* = 7.1Hz, 3H,CH₃), 1.18 (t, *J* = 7.1Hz, 3H, CH₃), 3.34(q, *J* = 7.1 Hz, 2H,C*H*₂CH₃), 3.45(q, *J* = 7.1 Hz, 2H, C*H*₂CH₃), 4.23 (s, 2H, CH₂C=O); 4.30 (dm, *J* = 30.1 Hz, 2H, OCH₂), 4.82 (dm, *J* = 47.9 Hz, 1H, FCH₂), 7.08 (d, *J* = 8.9Hz, 2H, ArCH×2), 7.56 (d, *J* = 8.9Hz, 2H, ArCH×2), 7.63 (d, *J* = 1.7 Hz, 1H, CH), 8.59 (d, *J* = 1.7Hz, 1H, CH).

### PBR137

### 2-(2-(4-tert-butylphenyl)-6,8-dichloroimidazo[1,2-a]pyridin-3-yl)-N,N-diethylacetamide

m.p.: 160°C.
¹H NMR(400MHz, DMSO) δ 1.06 (t, *J* = 7.1 Hz, 3H, CH₂C*H*₃), 1.17 (t, *J* = 7.1 Hz, 3H, CH₂C*H*₃), 1.32 (s, 9H, 3×CH₃), 3.30 (q, *J* = 7.1 Hz, 2H, C*H*₂CH₃), 3.45 (q, *J* = 7.1 Hz, 2H, C*H*₂CH₃), 4.25 (s, 2H, CH₂C=O), 7.50 (d, *J* = 8.6 Hz, 2H, 2×ArCH), 7.55 (d, *J* = 8.6 Hz, 2H, 2×ArCH); 7.64 (d, *J* = 1.7 Hz, 1H, CH); 8.60 (d, *J* = 1.7 Hz, CH).
¹³C NMR (400 MHz, DMSO) δ 13.06, 14.14 (CH₃), 28.90 (CH₂), 31.08 (CH₃×3), 34.39 (CH₂), 41.69 (CH₂), 117.63, 119.16, 121.62 (C), 122.62 (ArCH×2), 123.76 (ArCH×2), 130.81, 143.71, 150.57 (ArC-O), 166.81 (C=O).

### PBR138

### N-((6-chloro-2-(6-chloropyridin-3-yl)imidazo[1,2-a]pyridin-3-yl)methyl)benzamide

m.p.: 288-290°C.
¹H NMR(400MHz, DMSO) δ 5.0 (d, *J* = 5.4 Hz, 2H, C*H*₂NH); 7.29 (dd, *J* =2.0, 9.6 Hz, 1H, Cl-C-CH); 7.4-7.6 (m, 5H, ArCH), 7.8 (d, *J* = 9.6 Hz, 1H, Cl-C-CH=C*H*; superimposed with d, *J* = 8.3 Hz, 1H, pyridyl-CH), 8.27 (dd, *J* = 2.5, 8.3 Hz, 1H, pyridyl-CH), 8.64 (d, *J* =2.0 Hz, 1H, Cl-C=CH), 8.9 (d, *J*=2.5 Hz, 1H, pyridyl-CH-N).
MS: ES(+ve) *m*/*z* 397 (M+1).

### PBR139

### N-((2-(4-tert-butylphenyl)-6-fluoroimidazo[1,2-b]pyridazin-3-yl)methyl)benzamide

m.p.: 237°C.
¹H NMR(400MHz, DMSO) δ 1.35 (s, 9H, CH₃×3), 5.06 (d, *J* = 5.0 Hz, 2H, C*H*₂NH); 7.06 (d, *J* = 9.6 Hz, 1H, CH); 7.4-7.5 (m, 3H, ArCH), 7.55 (d, *J* = 8.5 Hz, 2H, ArCH×2), 7.72 (m, 2H, ArCH), 7.9 (d, *J* = 8.5 Hz, 2H, ArCH×2), 8.1 (dd, *J* = 7.4, 9.6 Hz, F-C-CH)
MS: ES(+ve) *m*/*z* 303 (M+1).

### PBR140

### 2-(2-(4-(allyloxy)phenyl)-6-chloroimidazo[1,2-a]pyridin-3-yl)-N,N-diethylacetamide

m.p.: 126°C.
¹H NMR (400 MHz, DMSO) δ 1.05 (t, *J* = 7.1 Hz, 3H, CH₃), 1.15 (t, *J* = 7.1 Hz, 3H, CH₃), 3.3 (q, *J* = 7.1 Hz, 2H, CH₂CH₃), 3.4 (q, *J* = 7.1 Hz, 2H, CH₂CH₃), 4.2 (s, 2H, CH₂), 4.6 (ddd, *J* = 1.46, 1.46, 5.3 Hz, 2H, OCH₂), 5.25 (ddt, *J* = 1.46, 1.75, 10.5 Hz, 1H, CH), 5.4 (ddt, *J* = 1.46, 17.2Hz, 1H, CH), 6.05 (ddt, *J* = 10.5,17.2, 5.2 Hz, 1H, CH).

### PBR141

### 2-(6,8-dichloro-2-(4-methoxyphenyl)imidazo[1,2-a]pyridin-3-yl)-N-phenylacetamide

m.p.: 220-222°C.
¹H NMR (400 MHz, DMSO) δ 3.80 (s, 3H, OCH₃), 4.29 (s, 2H, CH₂), 7.05 (d, *J* = 8.8 Hz, 2H, ArCH×2), 7.06 (m, 1H, ArCH, superimposed), 7.3-7.60 (m, 4H, ArCH), 7.66 (d, *J* = 1.7 Hz, 1H, CH), 7.70 (d, *J* = 8.8 Hz, 2H, ArCH×2), 8.72(d, *J* = 1.7 Hz, 1H, CH), 10.40 (s, 1H, NH).
¹³C NMR (100 MHz, DMSO) δ 31.79 (CH₂), 55.20 (OCH₃), 114.21 (ArCH×2), 117.65, 117.78 (C), 119.29 (ArCH×2), 121.55 (C), 122.58, 123.52, 123.91 (CH), 125.88 (C), 128.80, 129.18 (ArCH×2), 138.90,139.63 (C), 143.99 (ArC-NH), 159.23 (ArC-O), 166.91 (C=O).

### PBR142

### N-((6,8-dichloro-2-(4-methoxyphenyl)imidazo[1,2-a]pyridin-3-yl)methyl)benzamide

m.p.: 260-263°C.
¹H NMR (400 MHz, DMSO δ 3.80 (s, 3H, OCH₃), 4.94 (s, 2H, CH₂), 7.05 (d, *J* = 8.8 Hz, 2H, ArCH×2), 7.4-7.6 (m, 3H, ArCH), 7.68 (d, *J* = 1.6 Hz, CH), 7.85 (m, 2H, ArCH), 7.88 (d, *J* = 8.8 Hz, 2H, ArCH×2), 8.82 (d, *J* = 1.7 Hz, 1H, CH).
MS: ES(+ve) *m*/*z* 426 (M+1).

### PBR143

### 2-(2-(4-(2-fluoroethoxy)phenyl)-6,8-dichloroimidazo[1,2-a]pyridin-3-yl)-N-methyl-N-phenylacetamide

m.p.: 189-190°C
¹H NMR (400 MHz, DMSO) δ 3.25 (s, 3H, NCH₃), 3.90(s, 2H, CH₂C=O), 4.32 (dm, *J* = 30.1 Hz, 2H, OCH₂), 4.70-4.85 (dm, *J* = 47.8 Hz, 2H, FCH₂), 7.07 (d, *J* = 8.7 Hz, 2H, ArCH×2), 7.42 (d, *J* = 8.7 Hz, 2H, ArCH×2), 7.32-7.58 (m, 5H, ArCH), 7.63 (d, *J* = 1.8 Hz, 1H, CH);); 8.74 (d, *J* = 1.8 Hz, 1H, CH).

### PBR144

### 2-(2-(4-(2-methoxyethoxy)phenyl)-6-chloroimidazo[1,2-a]pyridin-3-yl)-N,N-diethylacetamide

m.p.: 134-135°C.
¹H NMR (400 MHz, DMSO), δ 1.06 (t, *J* = 7.1 Hz, 3H, CH₃), 1.16 (t, *J* = 7.1 Hz, 3H, CH₃), 3.29 (s, 3H, OCH₃), 3.32 (q, *J* = 7.1 Hz, 2H, NCH-₂), 3.47 (q, *J* = 7.1 Hz, 2H, NCH₂),3.67 (m, 2H, OCH₂), 4.14 (m, 2H, OCH₂), 4.21 (s, 2H, CH₂), 7.03 (d, *J* = 8.9 Hz, 2H, ArCH×2), 7.30 (dd, *J* = 9.5, 2.0 Hz, 1H, CH), 7.50 (d, *J* = 8.9 Hz, 2H, ArCH), 7.60 (dd, *J* = 9.4, 0.7 Hz, 1H, CH), 8.46 (dd, *J* = 2.0, 0.7 Hz, 1H, CH).

### PBR145

### 3-(2-(4-bromophenyl)-6-chloroimidazo[1,2-a]pyridin-3-yl)-N,N-diethylpropanamide

m.p.: 119-120°C.
¹H NMR (400 MHz, DMSO), δ 0.90 (t, *J* = 7.1 Hz, 3H, CH₃), 0.98 (t, *J* = 7.1 Hz, 3H, CH₃), 3.15 (q, *J* = 7.1 Hz, 2H, NCH-₂), 3.25 (q, *J* = 7.1 Hz, 2H, NCH-₂), 3.82 (d, *J* = 5.6 Hz, 2H, NHC*H*₂), 5.23 (t, *J* = 5.6 Hz, 1H, N*H*CH₂), 7.20 (dd, *J* = 9.5, 2.0 Hz, 1H, CH), 7.50 (dd, *J* = 9.5, 0.8 Hz, 1H, CH), 7.61 (d, *J* = 8.6 Hz, 2H, ArCH×2), 8.05 (d, *J* = 8.6 Hz, 2H, ArCH×2), 8.65 (dd, *J* = 2.0, 0.8 Hz, 1H, CH).

### PBR146

### 2-(2-(4-(3-fluoropropoxy)phenyl)-5,7-dimethylpyrazolo[1,5-a]pyrimidin-3-yl)-N,N-diethylacetamide m.p.: 106-108°C.

¹H NMR (400 MHz, DMSO) δ 1.02 (t, *J* = 7.1 Hz, 3H, CH₃), 1.18 (t, *J* = 7.1 Hz, 3H, CH₃), 2.14 (dm, *J* = 25.7Hz, 2H,CH₂), 2.49 (s, 3H, CCH₃), 2.69 (s, 3H, CCH₃), 3.29 (q, *J* = 7.1 Hz, 2H, NCH-₂), 3.52 (q, *J* = 7.1 Hz, 2H, NCH-₂), 3.84 (s, 2H, CH₂C=O), 4.13 (t, *J* = 6.3Hz, 2H, OCH₂), 4.55-4.70 (dt, *J* = 47.3, 5.9 Hz, 2H, FCH₂), 6.84 (s, 1H, CH), 7.05 (d, *J* = 8.9Hz, 2H, ArCH×2), 7.70 (d, *J* = 8.9Hz, 2H, ArCH×2).
¹³C NMR (400 MHz, DMSO) δ 13.08, 14.25 (*C*H₃CH₂), 16.27, 24.20 (*C*H₃C), 27.50 (CH₂C=O), 29.66, 29.86 (OC(*C*H₂)CF), 39.82, 41.57 (CH₃*C*H₂), 63.56, 63.61 (O CH₂), 80.04, 81.65 (F*C*H₂), 100.61 (C), 108.33 (CH), 114.45 (ArCH×2), 126.07 (C), 129.28 (ArCH×2), 144.55, 147.19, 153.49, 157.34,158.57 (C), 169.05 (C=O).

### PBR147

### 2-(2-(4-(3-fluoropropoxy)phenyl)-6,8-dichloroimidazo[1,2-a]pyridin-3-yl)-N-methyl-N-phenylacetamide

m.p.: 128°C.
¹H NMR (400 MHz, DMSO) δ 2.14 (dm, *J* = 25.8Hz, 2H,CH₂), 3.30 (s, 3H, NCH₃), 3.90(s, 2H, CH₂C=O), 4.14 (t, *J* = 6.3Hz, 2H, OCH₂), 4.48-4.64 (dt, *J* = 47.3, 5.9 Hz, 2H, FCH₂), 7.07 (d, *J* = 8.7Hz, 2H, ArCH×2), 7.45 (d, *J* = 8.7Hz, 2H, ArCH×2), 7.34-7.60 (m, 5H, ArCH), 7.63 (d, *J* = 1.7Hz, 1H, CH);); 8.74 (d, *J* = 1.7Hz, 1H, CH).
¹³C NMR (400 MHz, DMSO) δ 29.66, 29.86 (CH₂CF), 30.24 (CH₂), 37.12 (NCH₃), 63.61, 63.67 (OCH₂CCF ), 80.06, 81.67 (FCH₂), 114.71 (ArCH), 117.51, 118.12, 121.40 (C), 122.84 (CH), 123.79 (CH), 125.78 (C), 127.34 (CH), 127.98 (CH), 128.93 (ArCH), 129.86 (ArCH), 139.58 (C), 143.26 (N-ArC), 158.30 (O-ArC), 167.59 (C=O).

### PBR149

### 2-(2-(4-(2-fluoroethoxy)phenyl)-6-chloroimidazo[1,2-a]pyridin-3-yl)-N-methyl-N-phenylacetamide

m.p.: 198-200°C
¹H NMR (400 MHz, DMSO) δ 3.26 (s, 3H, NCH₃), 3.90 (s, 2H, CH₂), 4.3 (dm, *J* = 30 Hz, 2H, OCH₂), 4.8 (dm, *J* = 48 Hz, 2H, FCH₂), 7.06 (d, *J* = 8.5 Hz, 2H, ArCH×2), 7.28 (dd, *J* = 1.9, 9.5 Hz, 1H, CH), 7.3-7.55 (m, 5H, ArCH), 7.57 (d, *J* = 8.5 Hz, 2H, ArCH×2), 7.57 (d, *J* = 8.5 Hz, 1H, CH, superimposed), 8.68 (d, *J*= 1.9 Hz, 1H, CH).
¹³C NMR (400 MHz, DMSO) δ 29.95 (CH₃), 37.12 (CH₂), 67.03, 67.22 (OCH₂), 81.33, 82.99 (FC H₂), 114.68 (ArCH×2), 116.06 (ArCH×2), 117.06 (CH), 118.445 (C), 123.32 (CH), 124.94 (CH), 126.60 (C), 127.34 (CH), 127.95 (C), 128.83 (ArCH×2), 129.86 (ArCH×), 142.28 (N-ArC), 142.90 (C), 143.34 (C), 157.81 (O-ArC), 167.85 (C=O).

### PBR153

### 2-(6,8-dichloro-2-(4-methoxyphenyl)imidazo[1,2-a]pyridin-3-yl)-N-(6-chloropyridazin-3-yl)-N-methylacetamide

m.p.: 178-179°C.
¹H NMR (400 MHz, DMSO) δ 3.57 (s, 3H, CH₃), 4.43 (s, 2H, CH₂), 7.04 (d, *J* = 8.9 Hz, 2H, ArCH×2), 7.59 (d, *J* = 8.9 Hz, 2H, ArCH×2), 7.65 (d, *J* = 1.7 Hz, 1H, CH), 7.93 (d, *J* = 9.2, Hz, 1H, CH), 8.03 (d, *J* = 9.2 Hz, 1H, CHCCI), 8.79 (d, *J*= 1.7 Hz, 1H, CH).

### PBR154

### 2-(6,8-dichloro-2-(4-methoxyphenyl)imidazo[1,2-a]pyridin-3-yl)-N-(2-chloropyridin-3-yl)acetamide

m.p.: 110-112°C.
¹H NMR (400 MHz, DMSO) δ 3.82 (s, 3H, CH₃), 4.47 (s, 2H, CH₂), 7.06 (d, *J* = 8.9 Hz, 2H, ArCH×2), 7.44 (dd, *J* = 4.7, 8.0, Hz, 1H, CH), 7.67 (d, *J* = 1.7 Hz, 1H, CH), 7.75 (d, *J* = 8.9 Hz, 2H, ArCH×2), 8.18 (dd, *J*= 1.8, 8.0 Hz, 1H, CH), 8.23 (dd, *J* = 1.8, 4.7 Hz, 1H, CH), 8.81 (d, *J* = 1.7 Hz, 1H, CH), 10.20 (s, 1H, NH).

### PBR155

### 2-(6-chloro-2-(6-chloropyridin-3-yl)imidazo[1,2-a]pyridin-3-yl)-N,N-diethylacetamide

¹H NMR (400 MHz, DMSO) δ 1.05 (t, *J*= 7.1 Hz, 3H, CH₃), 1.16 (t, *J*= 7.1 Hz, 3H, CH₃), 3.36 (q, *J* = 7.1 Hz, 2H, CH₂), 3.52 (q, *J* = 7.1 Hz, 2H, CH₂), 4.33 (s, 2H, COCH₂), 7.37 (dd, *J* = 2.0, 9.6 Hz, 1H, CH), 7.66 (d, *J* = 8.3 Hz, 1H, CH), 7.69 (d, *J* = 9.6, Hz, 1H, CH), 8.10 (dd, *J* = 2.5, 8.3 Hz, 1H, CH), 8.62 (s, 1H, CH, superimposed), 8.62 (s, 1H, CH, super-imposed).
¹³C NMR (400 MHz, DMSO) δ 12.99, 14.14 (CH₃), 28.36, 39.88, 41.67 (CH₂), 117.55 (CH), 118.45, 119.12 (C), 123.29, 124.39, 125.68 (CH), 129.58 (C), 138.40 (CH), 139.25, 142.67 (C), 148.10 (CH), 149.21 (C), 166.69 (C=O)
MS: ES(+ve) *m*/*z* 377 (M+1).

### PBR156

### 2-(6,8-dichloro-2-(4-methoxyphenyl)imidazo[1,2-a]pyridin-3-yl)-N-(6-chloropyridin-3-yl)acetamide m.p.: 150-154°C.

¹H NMR (400 MHz, DMSO) δ 3.80 (s, 3H, OCH₃), 4.32 (s, 2H, CH₂), 7.06 (d, *J* = 8.9 Hz, 2H, ArCH×2), 7.48 (d, *J* = 8.7, Hz, 1H, CH), 7.66 (d, *J* = 1.8 Hz, 1H, CH), 7.67 (d, *J* = 8.9 Hz, 2H, ArCH×2), 8.09 (dd, *J* = 2.8, 8.7 Hz, 1H, CH), 8.61 (d, *J* = 2.8 Hz, 1H, CH), 8.81 (d, *J* = 1.8 Hz, 1H, CH).
¹³C NMR (400 MHz, DMSO) δ 31.76 (CH₂), 55.21 (OCH₃), 114.26 (ArCH×2), 117.11, 117.88, 121.57 (C), 122.63, 124.04, 124.28 (CH), 125.80 (C), 129.20 (ArCH×2), 130.02 (CH), 135.19, 139.71 (C), 140.50 (CH), 143.83, 144.15, 159.26 (C), 167.65 (C=O)
MS: ES(+ve) *m*/*z 461,* 463 (M+1).

### PBR158

### N-(6-(1H-imidazol-1-yl)pyridazin-3-yl)-2-(6-chloro-2-(4-iodophenyl)imidazo[1,2-a]pyridin-3-yl)-N-methylacetamide

m.p.: 218-219 °C.
¹H NMR (400 MHz, DMSO) δ 3.55 (s, 3H, NCH₃), 4.48 (s, 2H, CH₂), 7.21 (b, 1H, CH-imidazole), 7.32 (dd, *J* = 2.0, 9.6 Hz, 1H, CH-pyridine), 7.46 (d, *J* = 8.4 Hz, 2H, ArCH×2), 7.62 (dd, *J* = 9.6 Hz, 1H, CH-pyridine), 7.81 (d, *J* = 8.4 Hz, 2H, ArCH×2), 8.05 (dd, *J* = 1.2, 1.6 Hz, 1H, CH-imidazole, not fully resolved), 8.10 (d, *J* = 9.4 Hz, 1H, CH-pyridazine), 8.25 (d, *J* = 9.4 Hz, 1H, CH-pyridazine), 8.62 (b, 1H, CH-imidazole), 8.74 (d, *J* = 2.0 Hz, 1H, CH-pyridine, not fully resolved).

### PBR159

### 2-(6-chloro-2-(4-iodophenyl)imidazo[1,2-a]pyridin-3-yl)-N-(2-chloropyridin-3-yl)-N-methylacetamide m.p.: 258 °C.

### Rotamers were observed in NMR.

### Major:

¹H NMR (400 MHz, DMSO) δ 3.17 (s, 3H, NCH₃), 4.46 (ABq, *J* = 17.5, 2H, CH₂), 7.32 (dd, *J* = 2.0, 9.6 Hz, 1H, CH), 7.36 (d, *J* = 8.5 Hz, 2H, ArCH×2),7.55 (dd, *J* = 4.7, 7.9, Hz, 1H, Cl-Py-CH), 7.64 (d, *J* = 9.6 Hz, 1H, CH), 7.80 (d, *J* = 8.5 Hz, 2H, ArCH×2), 8.31 (dd, *J* = 1.8, 7.9, Hz, 1H, Cl-Py-CH), 8.44 (dd, *J* = 1.8, 4.7 Hz, 1H, Cl-Py-CH), 8.60 (d, *J* = 2.0 Hz, 1H, CH).
¹³C NMR (400 MHz, DMSO) δ 30.39 (CH₂), 35.72 (NCH₃), 94.15 (ArC-I), 116.03 (C), 117.48 (CH), 118.93 (C), 123.22, 124.65, 125.56 (CH), 129.76 (ArCH×2), 133.27, 136.63 (C), 137.35 (ArCH×2), 140.00(CH), 142.52, 142.65, 148.71 (C), 149.63 (CH), 167.38 (C=O).

### minor:

¹H NMR (400 MHz, DMSO) δ 3.48 (s, 3H, NCH₃), 4.53 (m, 2H, CH₂), 7.34 (m, 1H, CH, superimposed), 7.51 (d, *J* = 8.5 Hz, 2H, ArCH×2), 7.53 (m, 1H, Cl-Py-CH, superimposed), 7.65 (d, *J* = 9.6 Hz, 1H, CH), 7.85 (d, *J* = 8.5 Hz, 2H, ArCH×2), 7.94 (dd, *J* = 1.8, 7.9, Hz, 1H, Cl-Py-CH), 8.38 (dd, *J* = 1.8, 4.7 Hz, 1H, Cl-Py-CH), 8.62 (m, 1H, CH).
¹³C NMR (400 MHz, DMSO) δ 29.24 (CH₂), 37.62 (NCH₃), 94.15 (ArC-I), 116.35 (C), 117.48 (CH), 118.93 (C), 123.16, 124.23, 125.44 (CH), 129.86 (ArCH×2), 133.61(C), 137.44 (ArCH×2), 137.95 (C), 139.05(CH), 142.49, 143.00 (C), 148.35 (CH), 148.53 (C), 168.49 (C=O).

### Example 20

### IN VIVO EVALUATION OF THE [¹⁸F] LABELLED IMIDAZOPYRIDAZINE PBR-132*,

Synthesis and evaluation of the imidazopyridazine: 2-(2-(4-*tert*-butylphenyl)-6-fluoroimidazo[1,2-*b*]pyridazin-3-yl)-*N,N*-diethylacetamide **PBR132*.**

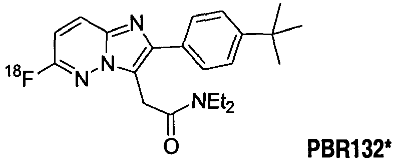

[[¹⁸F]**PBR132*has** been prepared by nucleophilic substitution of the Br precursor with ¹⁸F-fluoride in the presence of K222 and K₂CO₃ in DMF at 150°C for 5 mins.

The biodistribution of **PBR132*** was undertaken in SD rats and analysis up to 4h p.i. in the brain and peripheral tissues was performed. The specificity and selectivity of the tracer were assessed by pre-treatment with PBR and Central benzodiazepine receptor (CBR) specific ligands (1 mg/kg) 5 min prior to injection of **PBR132*.**

In vitro binding of **1** indicated an IC₅₀ of 29 nM for PBR and 340 nM for the CBR. [¹⁸F]1 was synthesised in 40-50% radiochemical yield (unoptimised) >95% radiochemical purity and specific activity of 40-80 GBq/µmol. The in vivo biodistribution of **PBR132*** showed high uptake in tissues of known PBR. In the adrenals was found an uptake of 13 % ID/g at 30 min p.i and maintained over the 4 h. In kidney, heart and lung the activity peaked (4, 8 and 16 % ID/g) at 15 min p.i. and decreased over time to less than 2.3% ID/g at 4h. Bone uptake ranged from 1 (at 15 min) to 3.3% ID/g at 4h. The uptake in the olfactory bulbs ranged from an initial 0.63 % at 15 min to 0.25 % at 4h p.i.. The concentration in the blood was low throughout the time of measurement. Pre-treatment with PK 11195 and Ro 5-4864 decreased the uptake in the brain and peripheral organs except in the adrenals which showed an activity increase. Flumazenil had no effect in the uptake of **PBR132*** in the brain or peripheral organs.

### Example 21

### SYNTHESIS AND EVALUATION OF THE [¹⁸F] LABELLED IMIDAZOPYRIDINE PBR 147*

Synthesis and evaluation of the fluorine-18 imidazopyridine analogue 2-(2-(4-(3-fluoropropoxy)phenyl)-6,8-dichloroimidazo[1,2-a]pyridin-3-yl)-N-methyl-N-phenylacetamide 1.

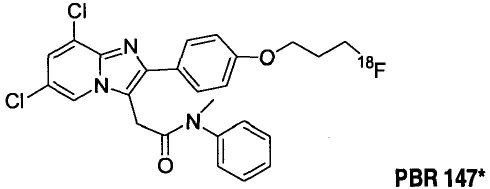

**PBR 147*** has been prepared by nucleophilic substitution of the tosylate precursor with ¹⁸F-fluoride in the presence of K₂₂₂, K₂CO₃ in ACN at 100°C for 5 mins.

The biodistribution of **PBR 147*** was performed in SD rats and brain and peripheral tissues were analysised at 15, 30 min, 1, and 4 h p.i.. The specificity and selectivity of the tracer was assessed by pre-treatment of the animals with the PBR ligands PK11195 and Ro 5-4864 and with Flumazenil for CBR at 1 mg/kg 5 min prior to injection of **PBR 147***

In vitro binding of **PBR147** indicated an IC₅₀ of 7.4 nM for PBR and a low IC₅₀ of >4000 nM for the CBR. [¹⁸F]**147*** was synthesised in 40-55 % radiochemical yield non-decay corrected and with > 95 % radiochemical purity. The specific activity ranged from 37-80 GBq/µmol. The in vivo biodistribution showed uptake of **PBR 147*** in tissue rich in PBR. The adrenals showed high uptake, increasing from 9 % ID/g at 30 min p.i to 11 % at 4 h. In the kidneys the activity peaked at 15 min p.i.(5 % ID/g) and decreased over time to 4 and 3.6 % ID/g at 4h. In the heart the uptake was maintained at 7% througout of the time of the experiment (15 min to 4 h). Bone uptake ranged from 1 (at 15 min) to 2.9 % ID/g at 4h. The uptake in the olfactory bulbs ranged from an initial 0.66% at 15 min to 0.26% at 4h p.i. while the concentration in the blood was significantly lower. Pre-treatment with PK 11195 and Ro 5-4864 decreased the uptake in the brain and peripheral organs except in the adrenals which showed an increase of **PBR 147*** uptake. Flumazenil had no effect in the uptake of **PBR 147*** in the brain or peripheral organs.

### Example 22

### SYNTHESIS AND EVALUATION OF [¹⁸F] LABELLED IMIDAZOPYRIDINES PBR102* and PBR111*

Synthesis and evaluation of two fluorine-18 imidazopyridine analogues **PBR102* and PBR111*.** Tracers **PBR102* and PBR111*** were prepared by nucleophilic substitution of the corresponding ethyl and propyl tosylate precursors with ¹⁸F-fluoride in the presence of K₂₂₂, K₂CO₃ in ACN at 100°C for 5 mins.

The biodistribution of **PBR102* and PBR111*** were performed in SD rats and brain and peripheral tissues analysed at 15, 30 min, 1, and 4 h p.i. The specificity and selectivity of the tracers were assessed by pre-treatment with the PBR ligands PK11195 and Ro 5-4864 and with Flumazenil for CBR at 1 mg/kg 5 min prior to injection of the corresponding tracers.

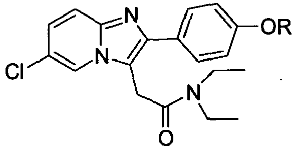

### PBR102* R = CH₂CH₂¹⁸F

### PBR111* R = CH₂CH₂CH₂¹⁸F

The IC₅₀ values of **PBR102 and PBR111** are 13.2 and 7.5 for the PBR and >1500 nM for the CBR. **PBR102* and PBR111*** were synthesised in 40-55% radiochemical yield non-decay corrected and with > 95 % radiochemical purity. The specific activity ranged from 37-80 GBq/µmol (un-optimised). The in vivo biodistribution of both tracers showed radioactivity uptake in tissue rich in PBR. The uptake of **PBR102***

in the olfactory bulbs ranged from 0.56 % at 15 min to 0.40 % ID/g at 4 h p.i whilst for **PBR111*** it ranged from 0.64 % to 0.41 % at 15 min and 4 h respectively. Whilst blood activity levels for **PBR102***remained constant (0.20%) over the 4 h study period **PBR111*** showed a decrease from 0.4 at 15 min to 0.1 at 4 h. The adrenals showed high uptake of **PBR102*** increasing from 9 % ID/g at 30 min p.i to 11 % at 4 h whilst **PBR111*** increased from 6.5 at 30 min to 16 % at 4 h. In the kidneys the activity peaked at 15 min p.i. for both tracers (4.5% ID/g) for **PBR102*** and (3.2 % ID/g) for **PBR111** * decreasing to 1.3 and 2.3% at 4 h. Bone uptake for **PBR102*** ranged from 0.4 % at 15 min to 1.0 % at 4 h whereas for **PBR111*** was 0.87 to 4.3 % for the same period. Pre-treatment with PK 11195 and Ro 5-4864 decreased the uptake in peripheral organs except in the adrenals which showed an increase of **PBR102***and **PBR111*** uptake. In the olfactory regions a non-significant decrease was observed with **PBR111*** Flumazenil had no effect in the uptake of **PBR102*** or **PBR111*** in the brain or peripheral organs.

### Example 23

### SYNTHESIS AND EVALUATION OF [¹⁸F] LABELLED PYRAZOLOPYRIMIDINES PBR099* and PBR146*

Tracers **PBR099* and PBR146*** were prepared by nucleophilic substitution of the corresponding ethyl and propyl tosylate precursors with ¹⁸F-fluoride in the presence of K₂₂₂, K₂CO₃ in ACN at 100°C for 5 mins.

The biodistribution of **PBR099* and PBR146*** were performed in SD rats and brain and peripheral tissues analysed at 15, 30 min, 1, and 4 h p.i. The specificity and selectivity of the tracers were assessed by pre-treatment with the PBR ligands PK11195 and Ro 5-4864 and with Flumazenil for CBR at 1 mg/kg 5 min prior to injection of the corresponding tracers.

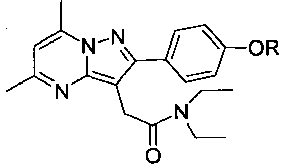

### PBR099* R = CH₂CH₂¹⁸F

### PBR146* R = CH₂CH₂CH₂¹⁸F

The IC₅₀ values of **PBR099 and PBR146** are 14.9 and 10.5 for the PBR and >5000 nM for the CBR. **PBR099* and PBR146*** were synthesised in 40-50% radiochemical yield non-decay corrected and with > 95 % radiochemical purity. The specific activity ranged from 40-80 GBq/µmol (un-optimised). The in vivo biodistribution of both tracers showed radioactivity uptake in tissue rich in PBR. The uptake of **PBR099*** in the olfactory bulbs ranged from 0.35 % at 15 min to 0.31 % ID/g at 4 h p.i whilst for **PBR146*** it ranged from 0.41 % to 0.31 % at 15 min and 4 h respectively. Whilst blood activity levels for **PBR099*remained** constant (0.23%) over the 4 h study period **PBR146*** showed a decrease from 0.4 at 15 min to 0.1 at 4 h. The adrenals showed high uptake of **PBR099*** increasing from 6.6 % ID/g at 30 min p.i to 9.7 % at 4 h whilst **PBR146*** increased from 7.9 at 30 min to 12.2 % at 4 h. In the kidneys the activity peaked at 15 min p.i. for both tracers (3.9% ID/g) for **PBR099*** and (5.5 % ID/g) for **PBR146*** decreasing to 2.4 and 3.2% at 4 h. Bone uptake for **PBR099*** ranged from 0.4 % at 15 min to 1.0 % at 4 h whereas for **PBR146*** was 1.0 to 5.0 % for the same period. Pre-treatment with PK 11195 and Ro 5-4864 decreased the uptake in peripheral organs except in the adrenals which showed an increase of **PBR099*and PBR146*** uptake. In the olfactory regions a small decrease was observed with **PBR146*** Flumazenil had no effect in the uptake of **PBR099*** or **PBR146*** in the brain or peripheral organs.

## Claims

1. A fluorinated compound of formula (1): 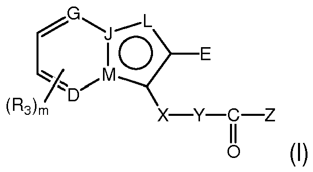 wherein,
D, G, and L are independently selected from the group consisting of: CH, C and N, and J and M are independently selected from the group consisting of C and N provided that one of J and M is C and the other is N, wherein at least two of D, G, M, J and L are N;
X is CH₂;
Y is absent;
Z is NR₁R₂;
R₁ and R₂ are independently selected from the group consisting of: hydrogen, C₁-C₁₀ alkyl, C₂-C₁₀ alkenyl, C₂-C₁₀ alkynyl, aryl and heteroaryl, each being optionally substituted with one or more of the following substituents: halogen, an C₁-C₆ alkyl;
or R₁ and R₂, together with the nitrogen to which they are attached, form a heterocyclic ring having between 3 and 7 ring members, optionally substituted with one or more of the following substituents: halogen and C₁-C₆ alkyl;
R₃ is a fluoro substituent;
E is an aryl group or a heteroaryl group, wherein each is optionally substituted with one or more fluoro substituents, or with one or more of the following substituents: C₁-C₆ alkyl, C₂-C₁₀ alkenyl, C₂-C₁₀ alkynyl, QC₁-C₁₀ alkyl, QC₂-C₁₀ alkenyl, QC₂-C₁₀ alkynyl, Q(CH₂)ₚ-Q-(CH₂)_{q}CH₃ or Q(CH₂)ₚ-Q-(CH₂)_{q}-Q-(CH₂)ᵣCH₃, each of which is optionally substituted with one or more fluoro substituents, and wherein p, q and r are, independently, integers between 1 and 3, and wherein Q is selected from the group consisting of: NH, O and S;
m is a number between 1 and 3;
with the proviso that E is not -PhF.

2. The compound of formula (1) of claim 1 wherein E is an aryl group or a heteroaryl group, which is substituted with of or with ¹⁹F.

3. The compound of formula (1) of claim 1 or claim 2 wherein Q is oxygen.

4. The compound of formula (1) of any one of claims 1 to 3 wherein m is 1 or 2.

5. The compound of formula (1) of any one of claims 1 to 4 wherein p is 1 or 2, q is 1 or 2, r is 1, and Q is oxygen.

6. The compound of formula (1) of any one of claims 1 to 5 wherein:
M and L are N, D and G are CH and J is C, or
D, M and L are N, J is C and G is CH; or
D, J and L are N, M is C and G is CH.

7. The compound of formula (1) of any one of claims 1 to 6 wherein R₁ and R₂ are independently selected from the group consisting of: hydrogen, C₁-C₆ alkyl, phenyl, pyridyl, pyrimidinyl, pyridazinyl and where R₁ and R₂, together with the nitrogen to which they are attached, form a heterocyclic ring having between 4 and 6 ring members, wherein the C₁-C₆ alkyl group, the phenyl group, the pyridyl group, the pyrimidinyl group, the pyridazinyl group or the heterocyclic ring are optionally substituted with between one and three fluoro substituents.

8. A compound according to any one of claims 1 to 7 that is radiolabelled with ¹⁸F.

9. A pharmaceutical composition comprising a compound according to any one of claims 1 to 8, together with at least one pharmaceutically acceptable carrier, diluent, excipient or adjuvant.

10. A compound according to claim 8 for use in the diagnosis of a disorder in a mammal **characterised by** an abnormal density of peripheral benzodiazepine receptors.

11. A compound according to any one of claims 1 to 7 for use in the treatment of a disorder in a mammal **characterised by** an abnormal density of peripheral benzodiazepine receptors, said compound being not radiolabelled with ¹⁸F.

## Patentansprüche

1. Fluorierte Verbindung der Formel (1): 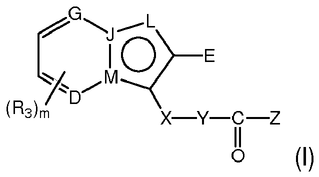 wobei
D, G und L unabhängig voneinander aus der Gruppe ausgewählt sind, die aus CH, C und N besteht, und J und M unabhängig voneinander aus der Gruppe ausgewählt sind, die aus C und N besteht, vorausgesetzt, dass entweder J oder M C ist und das jeweils andere N, wobei mindestens zwei von D, G, M, J und L N sind,
X CH₂ ist,
Y abwesend ist,
Z NR₁R₂ ist,
R₁ und R₂ unabhängig voneinander aus der Gruppe ausgewählt sind, die aus Folgendem besteht: Wasserstoff, C₁-C₁₀-Alkyl, C₂-C₁₀-Alkenyl, C₂-C₁₀-Alkynyl, Aryl und Heteroaryl, wobei jedes optional durch einen oder mehrere der folgenden Substituenten substituiert ist: Halogen, ein C₁-C₆-Alkyl,
oder R₁ und R₂ zusammen mit dem Stickstoff, mit dem sie verknüpft sind, einen heterocyclischen Ring bilden, der zwischen 3 und 7 Ringelemente aufweist, die optional durch einen oder mehrere der folgenden Substituenten substituiert sind: Halogen und C₁-C₆-Alkyl,
R₃ ein Fluorsubstituent ist,
E eine Arylgruppe oder eine Heteroarylgruppe ist, wobei jedes optional durch einen oder mehrere Fluorsubstituenten substituiert ist oder durch einen oder mehrere der folgenden Substituenten: C₁-C₆-Alkyl, C₂-C₁₀-Alkenyl, C₂-C₁₀-Alkynyl, QC₁-C₁₀-Alkyl, QC₂-C₁₀-Alkenyl, QC₂-C₁₀-Alkynyl, Q(CH₂)ₚ-Q-(CH₂)_{q}CH₃ oder Q(CH₂)ₚ-Q-(CH₂)_{q}-Q-(CH₂)ᵣCH₃, von denen jedes optional durch einen oder mehrere Fluorsubstituenten substituiert ist und wobei p, q und r unabhängig voneinander Ganzzahlen zwischen 1 und 3 sind und wobei Q aus der Gruppe ausgewählt ist, die aus Folgendem besteht: NH, O und S,
m eine Zahl zwischen 1 und 3 ist,
mit der Maßgabe, dass E nicht -PhF ist.

2. Verbindung der Formel (1) nach Anspruch 1, wobei E eine Arylgruppe oder eine Heteroarylgruppe ist, die durch ¹⁸F oder durch ¹⁹F substituiert ist.

3. Verbindung der Formel (1) nach Anspruch 1 oder Anspruch 2, wobei Q Sauerstoff ist.

4. Verbindung der Formel (1) nach einem der Ansprüche 1 bis 3, wobei m 1 oder 2 ist.

5. Verbindung der Formel (1) nach einem der Ansprüche 1 bis 4, wobei p 1 oder 2 ist, q 1 oder 2 ist, r 1 ist und Q Sauerstoff ist.

6. Verbindung der Formel (1) nach einem der Ansprüche 1 bis 5, wobei:
M und L N sind, D und G CH sind und J C ist oder
D, M und L N sind, J C ist und G CH ist oder
D, J und L N sind, M C ist und G CH ist.

7. Verbindung der Formel (1) nach einem der Ansprüche 1 bis 6, wobei R₁ und R₂ unabhängig voneinander aus der Gruppe ausgewählt sind, die aus Folgendem besteht: Wasserstoff, C₁-C₆-Alkyl, Phenyl, Pyridyl, Pyrimidinyl, Pyridazinyl, und wobei R₁ und R₂ zusammen mit dem Stickstoff, mit dem sie verknüpft sind, einen heterocyclischen Ring bilden, der zwischen 4 und 6 Ringelemente aufweist, wobei die C₁-C₆-Alkylgruppe, die Phenylgruppe, die Pyridylgruppe, die Pyrimidinylgruppe, die Pyridazinylgruppe oder der heterocyclische Ring optional durch zwischen ein und drei Fluorsubstituenten substituiert sind.

8. Verbindung nach einem der Ansprüche 1 bis 7, die mit ¹⁸F radioaktiv markiert ist.

9. Pharmazeutische Zusammensetzung, eine Verbindung nach einem der Ansprüche 1 bis 8 zusammen mit mindestens einem pharmazeutisch unbedenklichen Träger, Verdünnungsmittel, Hilfsmittel oder Zusatzstoff umfassend.

10. Verbindung nach Anspruch 8 zur Verwendung bei der Diagnose einer Funktionsstörung bei einem Säuger, die durch eine anormale Dichte von peripheren Benzodiazepinrezeptoren gekennzeichnet ist.

11. Verbindung nach einem der Ansprüche 1 bis 7 zur Verwendung bei der Behandlung einer Funktionsstörung bei einem Säuger, die durch eine anormale Dichte von peripheren Benzodiazepinrezeptoren gekennzeichnet ist, wobei die Verbindung nicht mit ¹⁸F radioaktiv markiert ist.

## Revendications

1. Composé fluoré de formule (1) : 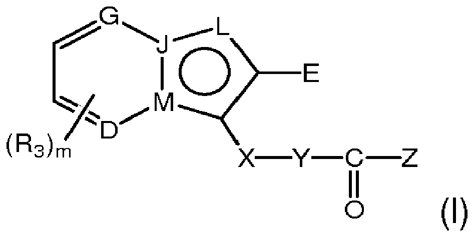 dans lequel,
D, G et L sont indépendamment choisis dans le groupe constitué par : CH, C et N, et J et M sont indépendamment choisis dans le groupe constitué par C et N, à condition que l'un de J et M soit C et que l'autre soit N, dans lequel au moins deux de D, G, M, J et L sont N ;
X est CH₂ ;
Y est absent ;
Z est NR₁R₂ ;
R₁ et R₂ sont indépendamment choisis dans le groupe constitué par : l'hydrogène, le groupe alkyle en C₁-C₁₀, le groupe alcényle en C₂-C₁₀, le groupe alcynyle en C₂-C₁₀, le groupe aryle et le groupe hétéroaryle, chacun étant facultativement substitué par un ou plusieurs des substituants suivants : l'halogène et un groupe alkyle en C₁-C₆ ;
ou R₁ et R₂, conjointement avec l'azote auquel ils sont fixés, forment un anneau hétérocyclique ayant entre 3 et 7 chaînons, facultativement substitués par un ou plusieurs des substituants suivants : l'halogène et le groupe alkyle en C₁-C₆ ;
R₃ est un substituant fluoro ;
E est un groupe aryle ou un groupe hétéroaryle, dans lequel chacun est facultativement substitué par un ou plusieurs substituants fluoro, ou par un ou plusieurs des substituants suivants : le groupe alkyle en C₁-C₆, le groupe alcényle en C₂-C₁₀, le groupe alcynyle en C₂-C₁₀, le groupe alkyle en QC₁-C₁₀, le groupe alcényle en QC₂-C₁₀, le groupe alcynyle en QC₂-C₁₀, Q(CH₂)ₚ-Q-(CH₂)_{q}CH₃ ou Q(CH₂)ₚ-Q-(CH₂)_{q}-Q-(CH₂)ᵣCH₃, chacun d'eux étant facultativement substitué par un ou plusieurs substituants fluoro, et dans lequel p, q et r sont indépendamment des nombres entiers compris entre 1 et 3, et dans lequel Q est choisi dans le groupe constitué par : NH, O et S ;
m est un nombre compris entre 1 et 3 ;
à condition que E ne soit pas -PhF.

2. Composé de formule (1) selon la revendication 1, dans lequel E est un groupe aryle ou un groupe hétéroaryle, qui est substitué par ¹⁸F ou par ¹⁹F.

3. Composé de formule (1) selon la revendication 1 ou 2, dans lequel Q est de l'oxygène.

4. Composé de formule (1) selon l'une quelconque des revendications 1 à 3, dans lequel m vaut 1 ou 2.

5. Composé de formule (1) selon l'une quelconque des revendications 1 à 4, dans lequel p vaut 1 ou 2, q vaut 1 ou 2, r vaut 1 et Q est de l'oxygène.

6. Composé de formule (1) selon l'une quelconque des revendications 1 à 5, dans lequel :
M et L sont N, D et G sont CH et J est C, ou
D, M et L sont N, J est C et G est CH ; ou
D, J et L sont N, M est C et G est CH.

7. Composé de formule (1) selon l'une quelconque des revendications 1 à 6, dans lequel R₁ et R₂ sont indépendamment choisis dans le groupe constitué par : l'hydrogène, le groupe alkyle en C₁-C₆, le groupe phényle, le groupe pyridyle, le groupe pyrimidinyle, le groupe pyridazinyle et dans lequel R₁ et R₂, conjointement avec l'azote auquel ils sont fixés, forment un anneau hétérocyclique ayant entre 4 et 6 chaînons, dans lequel le groupe alkyle en C₁-C₆, le groupe phényle, le groupe pyridyle, le groupe pyrimidinyle, le groupe pyridazinyle ou l'anneau hétérocyclique sont facultativement substitués par un à trois substituants fluoro.

8. Composé selon l'une quelconque des revendications 1 à 7, qui est marqué au ¹⁸F.

9. Composition pharmaceutique comprenant un composé selon l'une quelconque des revendications 1 à 8, conjointement avec au moins un support, diluant, excipient ou adjuvant pharmaceutiquement acceptable.

10. Composé selon la revendication 8, pour une utilisation dans le diagnostic d'une maladie chez un mammifère **caractérisée par** une densité anormale des récepteurs périphériques des benzodiazépines.

11. Composé selon l'une quelconque des revendications 1 à 7, pour une utilisation dans le traitement d'une maladie chez un mammifère **caractérisée par** une densité anormale des récepteurs périphériques des benzodiazépines, ledit composé n'étant pas marqué au ¹⁸F.
